(19) Europäisches Patentamt
European Patent Office
Office européen des brevets

(11) EP 1 273 667 A1

(12) EUROPEAN PATENT APPLICATION

(43) Date of publication:
08.01.2003 Bulletin 2003/02

(21) Application number: 01870154.0

(22) Date of filing: 04.07.2001

(51) Int Cl.[7]: **C12Q 1/68**, C12N 15/12, C07K 14/47, C07K 16/18, A61K 38/17, A61P 35/00, A61K 39/395, C07K 16/00

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Galapagos Genomics N.V.**
**2800 Mechelen (BE)**

(72) Inventors:
- **Merchiers, Pascal Gerard**
  **2460 Tielen (BE)**
- **van Es, Helmuth Hendrikus Gerardus**
  **2133 DJ Hoofddorp (NL)**
- **Tomme, Peter Herwig Maria**
  **9000 Gent (BE)**

(74) Representative: **Prins, Hendrik Willem et al**
**Arnold & Siedsma**
**Advokaten en Octrooigemachtigden**
**P.O. Box 18558**
**2502 EN Den Haag (NL)**

(54) **High throughput identification of modulators of angiogenesis**

(57) The invention relates to the field of molecular genetics and medicine. In particular, the present invention relates to the field of functional genomics, more in particular to a method for the identification of genes that function in the formation of blood vessels. The present invention provides the methods and means for the identification of nucleic acids and the polypeptides encoded by these nucleic acids that have a function related to angiogenesis, which were isolated in a high-throughput screening assay using the in vitro capillary formation assay as a read-out. The identified compounds are suitable drug-targets to treat human diseases.

EP 1 273 667 A1

## Description

### *Field of Invention*

The invention relates to the field of molecular genetics and medicine. In particular, the present invention relates to the field of functional genomics, more in particular to a method for the identification of genes that function in the formation of blood vessels.

### *Background of the invention*

With the recent publication of the draft sequence of the human genome, we are facing the challenge to attribute functions to the estimated 30-40.000 human genes. Of particular interest is the identification of the gene products that function in human disease pathways, as these potential new drug targets may lead to novel therapeutic strategies. However, the study of gene functions in vertebrates is hampered by the multicellular nature and the complexity of the genome. Because of this, only a limited set of genes is currently available for the screening of drug compounds that interfere with their function. Therefore, there is a need for a method that allows for direct measurement of function of a single gene from a collection of genes in a high-throughput setting in appropriate *in vitro* assays. For this, the current knowledge of human disease pathways will need to be implemented in the development of cellular assays, to allow cell-based high-throughput functional screening methods. The present invention provides the methods and means for the identification of nucleic acid compounds, isolated in a high-throughput screening assay, that have a function in angiogenesis.

### *Angiogenesis*

Remodelling of the primary plexus or the final vascular system occurs through different mechanisms of angiogenesis like branching, intussusceptive growth, sprouting and regression of existing capillaries. In order to create a new vessel, different biological actions take place during angiogenesis. In a first step, the existing endothelial vessel wall has to be destabilized and permeabilized resulting in fibrin deposition. This is followed by EC proliferation and EC migration towards the angiogenic stimulus, which requires the break down of the extra cellular matrix (ECM) through metalloproteinases and the plasminogen-plasmin system. Once the EC have invaded the tissue that has to be vascularized, primitive capillaries (fused EC) are formed. The final step comprises the recruitment of pericytes or smooth muscle cells (SMC) to infer structural stability to the newly formed vessel.

In general, these processes are driven by several cell membrane receptors and their corresponding growth factors, of which VEGF A is the most potent EC specific angiogenic inducer known so far. This protein belongs to the family of VEGF proteins (VEGF A; VEGFB; VEGF C; VEGF D; PIGF and VEGF E) (Ferrara, 1999). All these family members bind with different affinities to one or more of the VEGF receptors (VEGFR1 or flt1, VEGFR2 or KDR, VEGFR3 or Flt3) (see Figure 1). Neuropilin has also been shown to function as a co-receptor for the VEGF receptors (Gale and Yancopoulos, 1999). Despite its requisite role in vascular formation, VEGF must work in concert with other factors. Another family of proteins mainly involved in stabilization of the capillaries, are the angiopoietins and the tie2 receptor (Gale and Yancopoulos, 1999). Growth factors which are not EC specific, but which regulate angiogenesis are the fibroblast growth factors (FGF). FGF1, FGF2, FGF4, and others have been shown to be very strong inducers of EC proliferation. In addition, the broad spectrum growth factors hepatocyte growth factor (HGF) and insulin like growth factor 1 (IGF1) together with the interleukins (IL) and tumor necrosis factor alpha (TNF$\alpha$) have also been implicated in angiogenesis. Platelet derived growth factor (PDGF-B) and transforming growth factor beta (TGF$\beta$) act directly on the SMC and pericytes via recruitment and differentiation of the latter two cell types (Carmeliet and Jain, 2000). In recent years, evidence has accumulated that the ephrin ligands and the eph receptors play an important role in the maturation of the vascular system (Yancopoulos et al., 1998). They are involved in EC-SMC interactions, but also influence the establishment of the arterial-venous interface. In addition to the ephrins, some members of the integrin family are also assumed to be involved in angiogenesis (Bischoff, 1997). It is believed that $\alpha_v\beta_3$ a,d $\alpha_v\beta_5$ integrins are involved in the VEGF and bFGF pathway respectively. Intracellularly, the Akt kinase seems to play a central role (Dimmeler and Zeiher, 2000). Akt kinase is located downstream from many angiogenic growth factors and activates e.g. endothelial nitric oxide synthase, which has many positive effects on angiogenesis. Activation of Akt also leads to a strong anti-apoptotic response through e.g. Bcl2.

Since almost all human physiological processes are highly dependent on the presence of oxygen, the delivery of oxygen rich blood to all tissues through the vascular system is essential. Therefore, angiogenesis is known to direct a large number of biological processes (Carmeliet and Collen, 2000).

Vasculogenesis, i.e. the differentiation of EC, and angiogenesis start early during embryogenesis. Once the vascular system is fully developed, EC of blood vessels normally remain quiescent with no vessel formation, except for the female reproductive cycle (Goede et al., 1998). If disease or injury occurs, the formation of new blood vessels can proceed normally, as in natural wound healing, or be insufficient, as in chronic dermal ulcers, or there is deregulation of growth and an abnormal increase in vessel density as in tumorigenesis, psoriasis and inflammation (Carmeliet and Jain, 2000).

Angiogenesis in the adult is driven by inflammation and hypoxia. Upon hypoxia, the hypoxia inducible factor 1 alpha (HIF1$\alpha$) induces the expression of a number of pro-angiogenic factors like VEGF, PDGF, ANG2, NOS, and the like. In ischaemic myocardium and in stroke, angiogenesis restores the lost blood flow. However, it causes plaque rupture in atherosclerosis and blindness in premature newborns and in diabetic patients (retinopathy) (Carmeliet and Jain, 2000; Celletti et al., 2001). In addition, inflammation is most of the times accompanied by angiogenesis and increased permeability. All the inflammatory cells are known to produce angiogenic and anti-angiogenic molecules. Therefore, diseases like psoriasis, ulcer formation, asthma, rheumatoid arthritis, bone and cartilage destruction, hepatitis, glomerulonephritis and many others are associated with angiogenic changes (Carmeliet and Jain, 2000).

The disease with the highest mortality rate associated with pathological angiogenesis, is cancer. Just like organs, tumors need blood vessels to grow beyond a critical size and to metastasize in the body. These blood vessels are established through similar mechanisms as in physiological angiogenesis (sprouting and intussusception from peripheral blood vessels) or through co-option of an existing blood vessel (Carmeliet and Jain, 2000). These processes are regulated by the same growth factors (e.g. VEGF, the angiopoietins, bFGF, and the like), which are released by the extra cellular matrix or produced by the tumor cells and associated stromal cells, EC and blood cells. In addition, a large number of anti-angiogenic molecules (angiostatin, endostatin, canstatin, soluble VEGFR1, and the like) are expressed by these same cells. If there is a balance between the pro-angiogenic and the anti-angiogenic factors, no angiogenesis will occur.

However, if there is an imbalance in favor of the pro-angiogenic molecules the tumor will be vascularized and starts to grow. If the anti-angiogenic molecules are highly concentrated, the tumor will stay dormant and will suppress the growth of other metastasized tumors.

Angiogenesis is also involved in adipogenesis. In order to survive, adipose tissue needs sufficient blood flow, and thus angiogenesis occurs during the accumulation of fat cells (Sierra-Honigmann et al., 1998).

Thus, angiogenesis is essential for a number of pathological conditions, and it is expected that if the angiogenic processes are disrupted, the pathological process is also inhibited.

In recent years the idea of therapeutic angiogenesis to revascularize hypoxic tissues has been established (Yla-Herttuala and Martin, 2000). A tissue becomes hypoxic, when the blood flow has been disturbed through occlusion or rupture. It has been shown that upon aging, the endothelium becomes dysfunctional, resulting in a weaker angiogenic response towards hypoxia. In order to restore the original response, angiogenic growth factors (e.g. VEGF, bFGF) are administered to the patient (as gene therapy or recombinant protein therapy). These factors then induce the formation of new capillaries that have to restore the blood flow and thus relieve the hypoxic status of the tissue.

At the moment, a number of anti-angiogenic strategies are being developed based on known, natural occurring anti-angiogenic protein fragments, like angiostatin and endostatin, anti-angiogenic compounds targeted to e.g. VEGFR2, and antibodies targeted against key receptors like VEGFR2. These therapies are being tested alone or in combination with cytotoxic therapies in a number of cancers (Klohs and Hamby, 1999).

Thus, inhibition of inappropriate angiogenesis or enhancement of angiogenesis in non-healing wounds or revascularizing hypoxic tissues is therefore an extremely important target for drug discovery programs.

However, research in this area leading to new drug development has been hindered by the lack of high throughput screening (HTS) assays.

Current *in vitro* assay systems are cumbersome, time consuming, often based on *in vivo* systems and certainly not apt for automation. In general, the *in vitro* assay systems used, are based on the development of EC capillaries. The development of *in vitro* EC capillaries depends on two factors: the presence of a growth factor and of an extra cellular matrix (ECM) protein. The ECM into which angiogenesis *in vivo* occurs, consist of collagen type I, III, IV and V, laminin, fibronectin and fibrin depositions

*In vitro* angiogenesis has been performed with laminin, fibronectin, collagen, fibrin and matrigel, which is made by extracting the basement membrane matrix of Englebreth-Holm-Swarm tumors from lathrytic mice (Cockerill et al., 1995). However, some of the ECM substrates already contain angiogenic factors, which obscure the role of other factors under investigation.

Two different types of *in vitro* capillary formation exist, which are well known in the art the two-dimensional (2-D) and the three-dimensional (3-D) capillary formation. Both assays can be considered as representing different processes during angiogenesis. In the 2-D assay, the EC are seeded on top of the ECM and depending on the trigger they will subsequently invade the matrix and form capillaries. In the 3-D model, the EC are embedded in the ECM, and form capillaries. Both assays, evolve to the same endpoint, namely capillary formation but different processes are involved. In the 2-D assay, the cells first have to degrade and to invade (migration) the ECM, after which capillaries are formed. In contrary, the 3-D model is more focussed on survival of the EC, degradation of the ECM and the capillary formation.

Already in 1985, Montesano and Orci demonstrated that bovine microvascular EC, plated out on top of a collagen matrix, formed capillaries in response to the phorbol ester PMA (Montesano and Orci, 1985). Since then a great number of studies have been performed with collagen and with MVEC or human umbilical vein endothelial cells (HUVEC). The capillary formation can be triggered in this system by a number of growth factors like VEGF, FGF2, FGF4, IL3, and the like, but also by hypoxia (Deroanne et al., 1997; Phillips et al., 1995). It was shown that when bovine aortic endothelial cells are embedded in collagen (3-D system) they undergo apoptosis, which can be suppressed by supplementing an angiogenic growth factor like VEGF or FGF2 (Kuzuya et al., 1999; Satake et al., 1998). The capillary formation of HUVEC in collagen involves intracellular vacuole formation and coalescence (Davis and Camarillo, 1996). In addition, MVEC in collagen induce the expression of matrix metalloproteinases MT1-MMP and MMP2 (Haas et al., 1998). All these experiments indicate that *in vitro* capillary formation is a good *in vitro* model to study angiogenesis. However, because of its complexity, it was never used in high throughput screening. Despite the long felt need, *in vitro* capillary formation has been performed in 24-, 48- or at most 96-well plates.

To date, efforts to improve HTS have focused on making wells smaller (miniaturization). As one reduces the well size, one can increase the number of wells on each plate to provide more parallel testing. Further, by decreasing the assay volumes, one also decreases the cost of reagents per well. Moreover, because one can run more parallel tests with smaller assay volumes, one can simultaneously test more compounds to find drug candidates. However, miniaturization has inherent costs and complexities. In addition, the use of smaller wells allows the use of primary cells, which are limited in availability.

These costs and complexities relate directly with the three primary components of miniaturizing a screening format. First, one must be able to make the test containers smaller. Secondly, one must be able to accurately dispense all of the necessary assay reagents into more and smaller wells (usually accomplished by liquid handling robots that simultaneously dispense the reagents into many wells). Third, one must be able to "read" the results of the tests in the high density array.

Given the requirements of paralleled independent assays, each component provides challenges and limits to how much miniaturization is feasible or cost effective. For example, a smaller sample size increases the statistical variability from sample to sample because of the inherent inaccuracies in dispensing smaller volumes of reagents and in measuring weaker sample signals.

A particular problem in digital image analysis is the non-homogeneity of the illumination level. The requirements set by the illumination level has implications on the light source, the staining of the cells and/or irregularities of the plate, which all can prevent accurate interpretation of results.

A further problem in using *in vitro* capillary formation in an high throughput screening is dispensing solutions with an high viscosity, such as ECM substrates, which introduces air bubbles that prevent accurate interpretation of results.

### *Summary of the invention*

In some particularly useful, but non-limiting aspects, the present invention provides functional genomics, compounds and medicines. It is therefore an object of the present invention to provide a high-throughput, sensitive assay to screen for nucleic acid products that can modulate angiogenesis. Generally, it is an object of the invention to provide the identity of nucleic acids that are involved in angiogenesis. One specific object of the invention provides nucleic acids whose products are suitable drug-targets to treat human diseases.

Almost all human physiological processes are highly dependent on the presence of oxygen. Consequently, the delivery of oxygen rich blood to all tissues through the vascular system is essential. Therefore, angiogenesis is known to direct a large number of biological processes. In addition, angiogenesis is essential for a number of pathological conditions, and it is expected that if the angiogenic processes are disrupted, the pathological process is also inhibited. Inhibition of inappropriate angiogenesis or enhancement of angiogenesis in non-healing wounds or revascularizing hypoxic tissues is therefore an extremely important target for drug discovery programs.

In order to efficiently isolate candidate nucleic acids that modulate angiogenesis, nucleic acid libraries were screened for those nucleic acids that could modulate angiogenesis in a high-throughput setting.

For this, an arrayed nucleic acid library was generated. Subsequently, the eventual effect on angiogenesis by these nucleic acids or by the products encoded by these nucleic acids was monitored.

The present invention relates to polynucleotides and the encoded polypeptides that were identified in the high-throughput screen for the modulation of angiogenesis. Moreover, the present invention relates to vectors, host cells, antibodies and diagnostic methods for detecting diseases involving angiogenesis, and therapeutic methods for treating such diseases. The invention further relates to methods and means for drug compound screens designed to develop new therapeutic strategies.

## *DETAILED DESCRIPTION OF THE INVENTION*

The invention relates to a method for the identification of genes that function in the formation of blood vessels in a high throughput setting. The present invention makes use of a method for identifying a group of nucleic acids from which the individual members comprise a nucleic acid that modulates angiogenesis. This method comprises (i) the generation of an arrayed nucleic acid library, (ii) introducing said nucleic acid library into cells in an arrayed format, (iii) in the case that said cells are endothelial cells embedded in an extracellular matrix (ECM), determining the modulation in angiogenesis of the cells into which said arrayed library was introduced, or, alternatively, use the "conditioned medium" resulting from the cells into which said arrayed library was introduced, and apply this "conditioned medium" to endothelial cells embedded in ECM, after which the modulation in angiogenesis of these endothelial cells is determined.

In a preferred embodiment, the present invention provides a method for identifying a sample nucleic acid that modulates angiogenesis, said method comprising:

(a) embedding endothelial cells (ECs) in an extracellular matrix,
(b) introducing a sample nucleic acid into said ECs,
(c) assaying the phenotype and in particular the capillary formation of the ECs which were contacted with said sample nucleic acid, and
(d) identifying the sample nucleic acid that caused the capillary formation as determined in step (c).

It will thus be understood that the term "identifying" relates to the process of recognising whether a particular sample nucleic acid is able to modulate angiogenesis. Thus, the term "identifying" does not relate to the nature or origin of the sample nucleic, e.g. whether the sample nucleic acids "identified" in the said method may or may not have been known previously.

The term "modulates" refers to a sample nucleic acid that is able to modulate, change, or interfere with angiogenesis, including inhibition of angiogenesis as well as enhancing angiogenesis. In this respect, it will be understood that either the sample nucleic acid itself or the product encoded by said sample nucleic acid can interfere with the mechanisms involved in angiogenesis.

The present invention relates in particular to two types of methods for identifying a sample nucleic acid that is able to modulate angiogenesis, i.e. the direct *in vitro* capillary formation (ivcf) assay and the indirect ivcf assay.

<u>In the indirect ivcf assay,</u> a sample nucleic acid is introduced into "producer cells". These producer cells into which the sample nucleic acid has been introduced are incubated for an appropriate period of time to enable the expression of said sample nucleic acid. Said sample nucleic acid can encode for a secretable factor (angiogenic factor) that interferes with angiogenesis. Alternatively, said sample nucleic acid can encode for a protein that interferes with a pathway of which the eventual product is a secretable factor that interferes with angiogenesis. For example, the sample nucleic acid can encode for a factor (such as a factor affecting transcription, or mRNA or protein stability) that induces or enhances the secretion of an angiogenic factor. Secretion of these factors into the medium results in "conditioned medium". The conditioned medium is then transferred to endothelial cells (ECs), which are embedded in an extracellular matrix (ECM). Hence, sample nucleic acids can be identified that either encode for a secretable factor (angiogenic factor) that interferes with angiogenesis, or that encode for a factor that modulates angiogenesis indirectly, e.g. via interference with a pathway involved in angiogenesis, for example, such as described in the background section. Thus, the product encoded by the identified sample nucleic acids modulates directly or indirectly angiogenesis, e.g. resulting in a different phenotype, such as the formation of capillaries of the EC embedded in the ECM.

Thus, the present invention particularly refers to a method for identifying a sample nucleic acid that modulates angiogenesis, said method comprising:

(a) introducing a sample nucleic acid into producer cells,
(b) incubating the producer cells into which the sample nucleic acid has been introduced according to step (a) for an appropriate period of time to produce conditioned medium,
(c) contacting said conditioned medium of step (b) with ECs, which are embedded in an extracellular matrix,
(d) assaying the phenotype and in particular the survival and/or capillary formation of the ECs which were contacted with said conditioned medium according to step (c), and,
(e) identifying the sample nucleic acid of step (a) that caused the capillary formation as determined in step (d).

<u>In the direct ivcf assay,</u> a sample nucleic acid is directly introduced into the EC, after which the ability of said sample nucleic acid or the product encoded by this sample nucleic acid to modulate angiogenesis is determined. It will be clear that in the direct ivcf assay, factors can be identified that exert their effect intracellularly of the EC. In addition, factors that have an effect on neighboring endothelial cells can be identified, as well as the factors that have been described above for the indirect assay could be identified.

It will be appreciated by the man skilled in the art that the subsequent use of the direct ivcf assay and the indirect ivcf assay or vice versa can have a surplus value in the further characterization of the identified factor. In addition, it is envisaged that the specific type of producer cells, such as for example smooth muscle cells, HeLa or U2OS cells, is relevant for the eventual product that determines the conditioned medium.

Similarly, it will be acknowledged by the artisan that other cell types, in addition to EC may be embedded in the ECM. This will facilitate the identification of factors that have an effect on direct neighboring cells, such as EC.

The term "modulator" as used in the present invention thus contemplates any compound such as for example, but not limited to cDNAs, peptides, growth factors and cytokines, that demonstrates an activity that interferes with the pathways that are involved in angiogenesis, whether direct or indirect, i.e. via another factor.

Thus the present invention relates preferentially to a method as described herein in which assaying the phenotype is being performed in an *in vitro* capillary formation assay, in particular a direct *in vitro* capillary formation assay or an indirect *in vitro* capillary formation assay.

The sample nucleic acid according to the present invention can be genomic DNA, cDNA, previously cloned DNA, genes, ESTs, synthetic oligonucleotides, randomised sequences, antisense nucleic acids, small interfering RNA (siRNA), genetic suppressor elements, ribozymes, DNA encoding mutant zinc fingers, DNA encoding antibody sequences or any combination thereof. The sample nucleic acid may encode a full-length protein, but it might also encode a partial, not full-length, protein or a protein domain.

Preferably, the sample nucleic acid is part of a set or library of sample nucleic acids that are cloned into a vector, whereby the set or library of sample nucleic acids may comprise between two and $2x10^7$ individual sample nucleic acids, and usually will contain between 100 and $1x10^6$ different sample nucleic acids.

By introducing a sample nucleic acid into a cell is meant that the sample nucleic acid is transfected, infected or transduced into the cell by any method known to those experienced in the art. The nucleic acids can be delivered by any method known in the art, including, but not limited to, calcium-phosphate co-precipitation and direct needle injection. The nucleic acids may be delivered with delivery vehicles such as viral sequences, viral particles, liposome formulations, lipofectin or precipitating agents and the like. Viral vectors that can be used include, but are not limited to, a herpes virus vector, a baculovirus vector, a lentivirus vector, a retrovirus vector, an alphavirus vector, an adeno-associated virus vector or an adenoviral vector or any combination thereof.

In the present invention, it was surprisingly found that recombinant virus comprising a sample nucleic acid, in particular adenovirus, can infect the EC embedded in the extracellular matrix (ECM), and thus introduce the sample nucleic acid into the cell embedded in the ECM.

The cells or cell line to be used as "producer cells" in a method according to the present invention as defined above can be any desired cell that can be cultured *in vitro,* including primary cells. These cells can be obtained from any organism including, but not limited to, mammals such as humans, canine and rodents, reptiles, birds, amphibia, fish, yeast, fungi, bacteria and plants. Preferred producer cells relate but are not limited to, for example, HeLa cells, U2OS and smooth muscle cells.

In this application, the term "endothelial" refers to the phenotype of the cells, i.e. cells that are involved in angiogenesis, as is well known in the art. These endothelial cells can be obtained from any organism including, but not limited to, mammals such as humans, canine and rodents, reptiles, birds, amphibia, and fish. Preferred endothelial cells are primary HUVEC (human umbilical vein endothelial cells) and hMVEC (human microvascular endothelial cells).

The generation of a recombinant adenoviral vector library containing the sample nucleic acids, and the production of active recombinant viruses by introducing the viral vectors - containing the sample nucleic acid- in their respective packaging cell lines such as the PER.C6 or PER.C6/E2A packaging cell lines (US5994128, WO99/64582), are all performed in a high-throughput setting basically as described in WO99/64582 and further outlined in the examples. To identify and assign function to the product(s) encoded by the sample nucleic acids, the endothelial cell or producer cell is transduced in a high-throughput setting with the recombinant adenoviral vectors that express the product(s) of the sample nucleic acids.

Angiogenesis relates to the remodelling of the vascular system through different mechanisms, which include, for example, capillary formation, migration, proliferation, and/or survival of endothelial cells. In order to create a new vessel, different biological actions take place during angiogenesis. In a first step, the existing endothelial vessel wall has to be destabilized and permeabilized resulting in fibrin deposition. This is followed by EC proliferation and EC migration towards the angiogenic stimulus, which requires the break down of the extra cellular matrix (fibrin, collagen and the like) through metalloproteinases and the plasminogen-plasmin system. Once, the EC have invaded the tissue, that has to be vascularized, primitive capillaries (fused EC) are formed. Thus, capillary formation relates to the formation of structurally ordered, cord-like structures, preferably capillaries, by endothelial cells. The final step comprises the recruitment of pericytes or smooth muscle cells to infer structural stability to the newly formed vessel. Consequently, angiogenesis (branching, intussusceptive growth, sprouting and regression of existing capillaries) relates in part to a change in phenotype of EC, most of which can be readily observed, such as for example by capillary formation, including, migration, proliferation, and/or survival of endothelial cells. Hence, the term "assaying the phenotype" relates to assessing the ability of a sample nucleic to interfere with (modulate) one or more of the mechanisms involved in angiogenesis by determining a change of phenotype compared to controls, preferably by using the method of the invention. The controls display a particular phenotype in absence of the modulator. It will thus be understood that also the survival of endothelial cells due to the influence of a sample nucleic acid is contemplated in the present invention, if this survival is the consequence of the modulator.

By assaying the phenotype of the transduced cells is meant that each well is scored for a difference in phenotype compared to a control well, and in particular the presence or absence of capillary formation, migration, survival and/or proliferation compared to a control well. The set up of controls are well known in the art, e.g. in general a control well will differ in only one parameter from the well under investigation. The scoring can be done visually by light microscopy or by fluorescent microscopy after staining of the cells with a fluorescent marker. However, for high throughput screening, automated data capture and image analyses are preferred.

In view of the foregoing it will be clear that the phenotype can be determined by different assays.

Proliferation of cells can be measured by different means, such as, but not limited to, for example, BrdU incorporation, $^3$H incorporation, enzymatic conversion of 4-methylumbelliferryl heptanoate, calcein-AM or MTT and the like, and counting the cells, preferably in an automated fashion. It will be appreciated that all of these methods are well-know to the man skilled in the art.

The present invention thus in particular relates to a method for identifying a sample nucleic acid that modulates angiogenesis and in particular proliferation, said method comprising:

(a) contacting a modulator with endothelial cells (ECs),
(b) applying BrdU, $^3$H, 4-methylumbelliferryl heptanoate, calcein-AM or MTT to the ECs which were contacted with said modulator,
(c) assaying the BrdU or $^3$H incorporation or the enzymatic conversion of 4-methylumbelliferryl heptanoate, calcein-AM or MTT of said ECs, in which the amount of incorporated BrdU or $^3$H, or the amount of conversion is indicative for the proliferation of said EC.

The present invention thus in particular relates to a method for identifying a sample nucleic acid that modulates angiogenesis, said method comprising:

(a) contacting a modulator with an endothelial cell (EC),
(b) assaying cell migration of the EC which was contacted with said modulator,
(c) identifying the modulator that caused the cell migration as determined in step (b).

The present invention thus in particular relates to a method for identifying a sample nucleic acid that modulates angiogenesis, said method comprising:

(a) contacting a modulator with an endothelial cell (EC),
(b) assaying apoptosis of the EC which was contacted with said modulator,
(c) identifying the modulator that caused the survival of the EC as determined in step (b).

Within the scope of the invention, assaying the ability of a modulator to interfere with angiogenesis can thus be accomplished by several different assays. It will thus be understood that a modulator which has been identified in a particular assay can be "validated" in any other assay of the invention that relates to assaying the ability of said modulator to modulate angiogenesis.

Thus, the present invention relates in particular to a method for validating the sample nucleic acid identified by a method according to the present invention, in which said sample nucleic acid is further validated for modulating migration.

The present invention further relates to a method to validate a modulator ("validation method"), that has been identified by a method as described above, said validation method comprising:

(i) contacting the modulator identified in a method as described above with endothelial cells (ECs),
(ii) assaying the phenotype, and in particular capillary formation, migration, proliferation or survival of the ECs, cell migration, or apoptosis of the ECs which were contacted with said modulator, provided that the assaying step differs from the assaying step used in the method as described above to identify the sample nucleic acid, and,
(iii) validating the modulator that caused the phenotype as determined in step (ii), in particular validating capillary formation, proliferation (BrdU incorporation) cell migration, or survival (apoptosis).

According to a further preferred embodiment, the present invention pertains to a method as described herein, being characterised in that the last step of said methods is followed by a validation method, said validation method comprising:

(i) introducing the sample nucleic acid identified in a method as described herein, into endothelial cells (ECs), in particular HUVECs or HMVECs,
(ii) assaying the phenotype, and in particular migration of the ECs into which the sample nucleic acid was introduced, and
(iii) validating the sample nucleic acid that caused the phenotype, and in particular migration as determined in step (ii).

According to a further preferred embodiment, the present invention pertains to a method as described herein, being characterised in that the last step of said methods is followed by a validation method, said validation method comprising:

(a) introducing the sample nucleic acid identified by a method according to the present invention into producer cells,
(b) incubating the producer cells into which the sample nucleic acid has been introduced according to step (a) for an appropriate period of time to produce conditioned medium,
(c) contacting said conditioned medium of step (b) with ECs, in particular HUVECs or hMVECs,
(d) assaying the phenotype and in particular the migration of the ECs which were contacted with said conditioned medium according to step (c), and,
(e) validating the sample nucleic acid of step (a) that caused the migration as determined in step (d).

It will be appreciated by the man skilled in the art that alternative forms of the validation assays might exist, or that the validation assays might be changed according to specific needs. It is believed that these variation to the assays are within the skill of the artisan.
Hence, alternative validation assays as well as variation to the herein described validation assays are pertained in the present invention.
The assays used in the present invention relate particularly to 3-D capillary formation assays, in which the EC are preferably embedded in the ECM (extracellular matrix). Dependent on the presence of modulators, the EC form capillaries, migrate, proliferate or survive. The ECM substrates that can be used for the assays of the present invention are any ECM substrates in which the EC can form capillaries, survive, migrate and/or proliferate. Preferred substrates are substrates in which the identity of factors (if any) that modulate capillary formation, migration, survival and/or proliferation are known. In particular, the substrates laminin, fibronectin, collagen, and especially collagen type I, and fibrin are contemplated in the present invention.
In order to optimise the detection of living cells, the EC are preferably stained for viability, i.e. a stain which enables the detection of living cells. Preferred stains are stains that become fluorescent in living cells, or of which the emission wave length changes in living cells, for example, via conversion of the stain by an enzyme of a living cell. These stains are well known in the art.
Hence, the present invention relates to a method as described herein, further characterised in that said method comprises additionally staining for viability, in particular by calcein-AM, of the ECs, that have been contacted with said modulator or into which said sample nucleic acid has been introduced.
In this respect, it was surprisingly found that staining by calcein facilitated automated data analysis (see below). The present invention particularly relates to a 384-wells setting, where 10 µl of a 5 µM calcein-AM is added per well.
It will be appreciated by the man skilled in the art that a slight variation to the assays of the present invention enables these assays for identifying anti-angiogenic compounds. In particular, the ECs embedded in the ECM can be induced to capillary formation by factors, such as, for example, VEGF, FGF2, FGF4, IL3, and the like, but also by hypoxia. Subsequently, conditioned medium is applied to these ECs, and the phenotype is assayed, in particular the regression of EC capillaries.
Alternatively, a sample nucleic or an antibody or a drug compound together with an angiogenic growth factor, such as, for example, VEGF, FGF2, FGF4, IL3, and the like, but also hypoxia, is applied simultaneously, separately or subsequently to the ECs embedded in the ECM, and the resulting phenotype is assayed for inhibition of capillary formation or regression of existing capillaries respectively.
The present invention thus particularly refers to a method for identifying a sample nucleic acid that modulates angiogenesis, said method comprising:

(a) embedding the endothelial cells (ECs), in particular HUVECs or hMVECs, which are embedded in an extracellular matrix, preferably collagen,

(b) inducing capillary formation of said ECs,
(c) introducing a sample nucleic acid, an antibody or a drug compound with said capillaries formed in step (b),
(d) assaying the phenotype and in particular the regression of said capillaries, which were contacted with said sample nucleic acid, and
(e) identifying the sample nucleic acid, the antibody or the drug compound that caused said regression as determined in step (d).

The present invention thus particularly refers to a method for identifying a sample nucleic acid that modulates angiogenesis, said method comprising:

(a) introducing a sample nucleic acid into producer cells,
(b) incubating the producer cells into which the sample nucleic acid has been introduced of step (a) for an appropriate period of time to produce conditioned medium,
(c) contacting said conditioned medium of step (b) to capillaries formed by ECs, in particular HUVECs or HMVECs, which are embedded in an extracellular matrix, preferably collagen,
(d) assaying the phenotype and in particular the regression of capillaries of the ECs which were contacted with said conditioned medium, and,
(e) identifying the sample nucleic acid of step (a) that caused the regression as determined in step (d).

Similarly, the present invention refers to a method for identifying a sample nucleic acid, an antibody or a drug compound that modulates angiogenesis, said method comprising:

(a) embedding endothelial cells (ECs), in particular HUVECs or hMVECs embedded in an extracellular matrix, preferably collagen,
(b) introducing into or contacting with said ECs, a sample nucleic acid, an antibody or a drug compound simultaneously, separately or subsequently with an angiogenic factor or hypoxia,
(c) assaying the phenotype and in particular the inhibition and/or regression of capillary formation of the ECs according to step (b), and
(d) identifying the sample nucleic acid, the antibody or the drug compound that caused said inhibition as determined in step (c).

Evidently, the present invention thus also refers to a method for identifying a sample nucleic acid that modulates angiogenesis, said method comprising:

(a) introducing a sample nucleic acid into producer cells,
(b) incubating the producer cells into which the sample nucleic acid has been introduced of step (a) for an appropriate period of time to produce conditioned medium,
(c) contacting said conditioned medium of step (b) with ECs embedded in an extracellular matrix, simultaneously, separately or subsequently with an angiogenic factor or hypoxia,
(d) assaying the phenotype and in particular the inhibition and/or regression of capillary formation of the ECs according to step (c), and
(e) identifying the sample nucleic acid that caused said inhibition as determined in step (d).

**High Throughput Screening**

In view of the complexity, the potential number of factors and the intricate relations between these factors that are involved in the mechanisms of angiogenesis, it is preferred to screen for a large number of factors. Therefore, the measurement of the function of the sample nucleic acids, which in the set-up of the present invention is defined as a modulation of angiogenesis of endothelial cells, is preferably performed in a high-throughput and/or miniaturised set-up.

As used herein, the terms "high-throughput screen" or "HTS" refer to an assay involving the exposure of a known (or target) to a group (or library) of unknowns (or test compounds) in an automated fashion, wherein the progression of a reaction associated with the target is assayed for, the results of which correlate to the level of biological activity of a particular test compound. This target-associated reaction often involves binding, such as ligand to receptor. The HTS describes a method where many discrete compounds are tested in parallel so that large numbers of test compounds are screened for biological activity simultaneously or nearly simultaneously, with the capacity for robotic manipulation, and small sample volume.

Currently, the most widely established techniques utilise 96-well microtitre plates. In this format, 96 independent tests are performed simultaneously on a single 8 cm.times.12 cm plastic plate that contains 96 reaction wells. These wells typically require assay volumes that range from 50 to 500 µl addition to the plates, many instruments, materials, pipettors, robotics, plate washers and plate readers are commercially available to fit the 96-well format to a wide range of homogeneous and heterogeneous assays.The present invention particularly relates to a 384-wells or larger setting.

Hence, the present invention relates to a method as described herein, being characterised in that at least one step, such as, for example the assaying step, but more preferentially two or more steps are performed in a miniaturised and/or high-throughput format.

Air bubbles in the ECM can make up a large proportion of the available space in a well. Air bubbles are thus a particular problem in small spaces, such as the wells of 384 or more-well plates, in which the air bubbles occupy a large portion of the available space. Consequently, high-throughput screening for angiogenic factors was considerably hindered by these air bubbles. In this respect, it was in the present invention surprisingly found that incubating the plates containing the EC embedded in collagen for at least 5, but preferentially 10, and even more preferentially 15, 20 or more minutes at 4 °C prevented and/or dissolved any air bubbles present in the polymerising ECM. Incubating the plates containing the EC embedded in collagen for shorter times resulted in an increasing number of false-positives and/or distorted read-outs of the assays.

The present invention therefore relates to a method as described herein, characterised in that the method comprises the step of preventing and/or dissolving air bubbles in the substrate.

<u>Automated data analysis</u>

The assays of the present invention are preferably performed in HTS, including automated image analysis. It was surprisingly found in the present invention that calcein-AM is most compatible with the HTS assay, because the use of calcein-AM allows to record sharp and clear images of all the wells, which could be used for image analysis. The use of calcein-AM overcomes most of the disturbance due to the remnants of dead cells and the fact that the cells are located in a 3-dimensional structure. As a consequence of this 3-dimensional structure, not all cells can be in focus.

The present invention therefore particularly contemplates an automated step of assaying the phenotype of the EC which was contacted with the modulator of the present invention. The automation of image acquiring and analysis of the phenotype can be performed by any method known in the art.

**Image analysis**

The present invention relates in particular to digital image analysis methods for the improvement of image information for manual interpretation and automatic computer analysis and interpretation. The procedure of the invention results in identification of images that present strong or reduced capillary formation and secondly to quantify the strength or reduction of the capillary formation. For automatic computer analysis, image processing is subdivided in several steps.

Image Acquisition and Format:

The first step in automatic computer analysis is the acquisition of the images for analysis and interpretation. For illumination preferably a very fast scanning table using high definition monochromatic CCD camera and an illumination source can be used. The illumination source can be for instance a lamp, a laser and the like. It will be understood by the man skilled in the art, that any comparable camera with comparable illumination can be used. To facilitate the image processing procedures, an initial setting can be carried out, which can include adjustment of the best configuration in illumination, position and focus. The images can be stored in any format, such as, for example a standard Tiff file format with a resolution of at least 200 x 200 pixels per image, but preferably 1024x1024 pixels per image. It will be understood by the man skilled in the art that better resolution results in a more accurate analysis later on in the procedure.

Image Processing:

The second step in automatic computer analysis relates to the image processing. In Image processing, the acquired image as defined above is used to start with. The information of this image is transformed until a single measurement is obtained, which indicates the amount of capillary present on the image. Each step of this procedure are described below.

For computational manipulation of the image, the following mathematical formalism can be used: a continuous image is defined as an function f (x,y) having the intensity values on the respective x,y location. Since the image under examination is in a digital format, the values of x,y assume a discrete format which range from 0-1023 in this particular case. It will be understood that in the case of a different resolution, the range can be adapted accordingly. The matrix expression is represented by:

$$f(x,y) \approx \begin{bmatrix} f(0,0) & f(0,1) & \cdots & f(0,M-1) \\ f(1,0) & f(1,1) & \cdots & f(1,M-1) \\ \vdots & & & \vdots \\ f(N-1,0) & f(N-1,1) & \cdots & f(N-1,M-1) \end{bmatrix}, \qquad (1)$$

N and M assumes the values of 1023. The value for the function f for each value of x and y represents the intensity of the image at the specific position. This intensity value varies from 0 to 255 grey levels; in which 0 represent the absence of light (black) and 255 the maximum value that represents light intensity (white).

<u>1) Correction of Non-uniform illumination.</u>

The heterogeneous illumination of the background is decreased by converting the background (well and plate) and the cell signal into a uniform gray level, in order to identify the cell signal against a homogeneous background. The present invention relates in particular to the application of the following procedure:

1.1 A window block of size m x n can be created each having the minimum value of intensity in the equivalent m x n image window of the original image.

$$b(p,q) = \sum_{p=1}^{m}\sum_{q=1}^{n} \min(f(x,y)), \qquad (2)$$

1.2 Once this new image is created the resulting image can be a subtraction of the original with the window block image.

$$f' = f - b. \qquad (3)$$

An image intensity rescaling is then applied and the final image for threshold is obtained.

<u>2) Threshold.</u>

At this point, and depending on the window settings, the image is still composed of various leves of gray. It is particularly contemplated that the image may be composed of 256 levels of gray. In order to discriminate between positive and no capillary formation, a threshold can be introduced. Threshold is the process that transforms the gray level image into a bi-level image, having the white level representing the cell signal. To obtain this threshold, the histogram plot of the image can be used, i.e. based on the median value criteria of the distribution.

$$t = \min - ((\min - \max) * p) \qquad (4)$$

where min and max represents the gray value with minimum and maximum distribution respectively. In this expression p is a variable parameter, which depends on the value of the median.

3) Skeletonization.

Since the capillary formation is to be defined and not the complete cell signal, a step towards the length measurement of the cell signal is necessary. The procedure consists basically in thinning the connected component structure into single lines. It is prefered procedure that the transformation still preserves the morphological appearance of the cell signal. This transformation can be obtained through a common procedure in image processing called skeletonization, which in turn is obtained by the use mathematical morphology operators.

4) Cluster selection.

The skeletonized image depicts various small and disconnected clusters. A cluster is defined as a set of pixels. The small clusters do not contribute for the identification of capillary formation. Small clusters can be defined for example, as representing 10% of the average cell size. Thus, the small clusters are eliminated from the final image.

5) Cluster count.

The final value for the amount of capillary present in an image can then be taken as the total pixel count on the resulting image.

Data Analysis:

All the relevant samples can then be automatically scanned and analyzed. The total distribution of the capillary formation measurement can be obtained, and the threshold based on the samples of one or more plates can be calculated. The threshold can be set as the average of all samples + (3 x the standard deviation over all samples). Samples scoring higher than this threshold can be considered positive and represent a hit in the primary screening.

The present invention relates in particular to a method as described herein, wherein said assaying the phenotype is performed in an automated setting, comprising the steps of:

(a) image acquisition,
(b) image processing, comprising:

  (I) optionally auto-focussing,
  (II) optionally correcting for non-uniform illumination,
  (III) transformation of the grey level into a bi-level image,
  (IV) skeletonisation or area determination of the formed capillaries by mathematical morphology operators, such as described by Soille (1999)
  (V) elimination of small clusters,
  (VI) cluster count, and,

(c) data analysis.

Similarly, the present invention relates to a validation method as described herein, in which the assaying step is performed in an automated setting.

**Identified modulators, and the applications thereof.**

It will be clear that the method according to the invention results in the identification of modulators or sample nucleic acids which are able to interfere with the mechanisms of angiogenesis. The modulation of angiogenesis can result from either the sample nucleic acid itself or the product encoded by said sample nucleic acid.

It will thus be clear that in a preferred embodiment, the invention provides any nucleic acid identifiable by the methods according to the present invention.

The invention further provides the sequence identities (SEQ ID NOs: 1 to 4) of the group of nucleic acids and corresponding polypeptides that modulate angiogenesis in a primary screen, and possibly in validation assays.

In a preferred embodiment, the present invention provides an isolated nucleic acid comprising a member selected from a group of nucleic acids identifiable as modulators of angiogenesis according to the method as described herein, said group consisting of:

(a) a nucleic acid comprising a DNA sequence as given in SEQ ID NO 1 or 3, or the complement thereof,

(b) a nucleic acid comprising the RNA sequences corresponding to SEQ ID NO 1 or 3, or the complement thereof,
(c) a nucleic acid specifically hybridising to the nucleotide sequence as defined in (a) or (b),
(d) a nucleic acid having a nucleotide sequence at least 65% identical to the sequence defined in (a),
(e) a nucleic acid encoding a protein with an amino acid sequence which is at least 65% identical to the amino acid sequence as given in SEQ ID NO 2 or 4,
(f) a nucleic acid encoding a protein comprising the amino acid sequence as given in any of SEQ ID NO 2 or 4,
(g) a nucleic acid which is degenerated as a result of the genetic code to a nucleotide sequence of a nucleic acid as given in SEQ ID NO 1 or 3, or as defined in (a) to (f),
(h) a nucleic acid which is diverged due to differences in codon usage between organisms to a nucleotide sequence encoding a protein as given in SEQ ID NO 2 or 4, or as defined in (a) to (g),
(i) a nucleic acid which is diverged due to the differences between alleles encoding a protein as given in SEQ ID NO 2 or 4, or as defined in (a) to (h),
(j) a nucleic acid encoding an immunologically active and/or functional fragment of a protein encoded by a DNA sequence as given in SEQ ID NO 1 or 3,
(k) a nucleic acid encoding a gene family member of the nucleic acid as given in SEQ ID NO 1 or 3, and,
(l) a nucleic acid encoding a protein as defined in SEQ ID NO 2 or 4, or a nucleic acid as defined in any one of (a) to (k) characterised in that said sequence is DNA, cDNA, genomic DNA or synthetic DNA.

With "immunologically active" is meant that a molecule or specific fragments thereof such as epitopes or haptens are recognized by, i.e. bind by antibodies.
In a preferred embodiment, the invention provides a nucleic acid molecule of at least 10 nucleotides in length specifically hybridising with any of the nucleic acids of the present invention. In particular, longer nucleic acid molecules are contemplated, i.e. of about 20, 25, 30, 40, 50, 75, 100, 200 or even more nucleotides. It is to be understood that also shorter probes may be useful (having for instance 10, 11, 12, 13 or 14 nucleotides).
Different types of hybridisation techniques and formats are well known in the art. The said nucleic acid molecule may be labelled with, for example, a radioactive isotope or an immunofluorescent compound, thereby allowing the detection of the hybrid. As such, the present invention provides methods for detecting the nucleic acids of the present invention.
In a further embodiment, the invention provides a nucleic acid molecule of at least 15 nucleotides in length as described above, wherein said nucleic acid molecule is liable to act as a primer for specifically amplifying a nucleic acid of the present invention, or a part thereof. It is to be understood that said primers can be shorter, e.g. 10, 11, 12, 13, or 14 nucleotides, or longer, e.g. 16, 17, 18, 19, 20, 25, or 30 nucleotides.
Sets of said primers may be used in any well described amplification technique known in the art such as Polymerase Chain Reaction (PCR), TMA or NASBA techniques, thereby allowing the amplification and subsequent detection of the nucleic acid of the present invention. Preferably, said primers may also be used to specifically amplify the nucleic acids of the present invention. As such, the present invention provides methods for detecting the nucleic acids of the present invention.
The present invention is also directed to variants of the nucleotide sequence of the nucleic acid disclosed in SEQ ID NOs 1 or 3, or the complementary strand thereto.
The present invention is also directed to nucleic acid molecules which comprise, or alternatively consist of, a nucleotide sequence which is at least 65%, 70%, 75%, 80%, 85%, 90%, 95%, 96%, 97%, 97,5%, 98%, 98,5%, 99% or 99,5% identical to the nucleotide sequences as represented in SEQ ID NOs 1 or 3, or the complementary strand thereto, or parts thereof. Said parts are preferably unique parts.
By a nucleic acid having a nucleotide sequence at least, for example, 95% "identity" to a reference nucleotide sequence of the present invention, it is intended that the nucleotide sequence of said nucleic acid is identical to the reference sequence except that the nucleotide sequence may include up to five point mutations per each 100 nucleotides of the reference nucleotide sequence. In other words, to obtain a nucleic acid having a nucleotide sequence of at least 95% identity to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. As a practical matter, whether any particular nucleic acid molecule is at least 65%, 70%, 75%, 80%, 85%, 90%, 95%, 96%, 97%, 97,5%, 98%, 98,5%, 99% or 99,5% identical to a nucleotide sequence of the present invention can be determined using known algorithms. A preferred method for determining the best overall match between a query sequence (a sequence of the present invention) and a subject sequence can be determined using a Blast search (Altschul et al., 1997).
Nucleic acids which specifically hybridise to any of the strands of the nucleic acid molecules of the present invention as specified under SEQ ID NO 1, or 3, under stringent hybridisation conditions or lower stringency conditions are also particularly encompassed by the present invention. "Stringent hybridisation conditions" refers to an overnight incubation at 68°C in a solution comprising 5xSSC (750 mM NaCl, 75 mM trisodium citrate), 50 mM sodium phosphate (pH 7.6), 5x Denhardt's solution, 10% dextran sulfate and 20 µg/ml denatured sheared salmon sperm DNA, followed by washing the filters in 0.1xSSC at about 65°C. Changes in the stringency of hybridisation are primarily accomplished through the manipulation of the SSC dilution in the washing steps (higher concentration SSC in washing buffer results in lower stringency) and the temperature (lower washing temperature results in lower stringency). For example, lower stringency conditions include washes performed at 1xSSC and at 55-60°C. Hybridisation under high and low stringency conditions are principles which are well understood by the person skilled in the art (see, for instance, Sambrook et al. Molecular Cloning: A laboratory manual. Cold Spring Harbor laboratory press 1989).

In addition, some of the proteins identified herein that play a role in angiogenesis, could serve as specific markers for the pathological process. Therefore, they can be used as diagnostic markers or as therapeutic markers that are used to target specific drugs towards the pathological tissue.

Methods which are well known to those skilled in the art may be used to construct expression vectors containing at least a fragment of the nucleic acids of the present invention together with appropriate transcriptional and translational control elements.

These methods include *in vitro* recombinant DNA techniques, synthetic techniques, and *in vivo* genetic recombination. Such techniques are described, for example, in Sambrook et al. Molecular Cloning: A laboratory manual. Cold Spring Harbor laboratory press 1989.

The present invention relates also to vectors comprising the sample nucleic acid of the present invention. The present invention particularly contemplates recombinant expression vectors, said vectors comprising a vector sequence, an appropriate prokaryotic, eukaryotic or viral or synthetic promoter sequence followed by the sample nucleic acid of the present invention. Preferably, the vector used for expressing the sample nucleic acid according to the present invention can be a vector for expression in *E. coli*, a yeast shuttle vector, or a yeast two-hybrid vector, a plant vector, an insect vector, a mammalian expression vector, including but not limited to, a herpes virus vector, a baculovirus vector, a lentivirus vector, a retrovirus vector, an alphavirus vector, an adenoviral vector or any combination thereof.

In a preferred embodiment, the invention provides a vector comprising a nucleic acid sequence of the present invention. As such, said nucleic acid is a member selected from a group of nucleic acids identifiable as modulators of angiogenesis. Preferably, said nucleic acid is a member selected from a group represented by SEQ ID NOs: 1 or 3, or variants, fragments or homologues thereof.

In a preferred embodiment said vector is an expression vector wherein the nucleotide sequence is operably linked to one or more control sequences allowing the expression of said sequence in prokaryotic and/or eukaryotic host cells.

In a preferred embodiment said vector is an adenoviral vector.

In a preferred embodiment said vector is generated from an adenoviral adapter vector which contains the left ITR and part of the E2B region, and in which the E1 region has been exchanged for a mammalian promotor, a polylinker sequence, and a polyadenylation signal.

As will be understood by those skilled in the art, it may be advantageous to produce products encoded by the aforementioned isolated nucleotide sequences possessing non-naturally occurring codons. For example, codons preferred by a particular prokaryotic or eukaryotic host can be selected to increase the rate of protein expression or to produce an RNA transcript having desirable properties, such as a longer half-life, thereby increasing the amount of expressable polypeptides in a cell, which may be desirable for multiple applications.

The nucleotide sequences of the present invention can be engineered using methods generally known in the art in order to alter protein encoding sequences for a variety of reasons, including but not limited to, alterations which modify the cloning, processing, and/or expression of the gene product. DNA shuffling by random fragmentation or PCR reassembly of gene fragments or synthetic oligonucleotides may be used to engineer the nucleotide sequences. For example, site-directed mutagenesis may be used to insert new restriction sites, alter glycosylation patterns, change codon preference, produce splice variants, introduce mutations, and so forth.

Furthermore, natural, modified, or recombinant nucleotide sequences may be ligated to partial or complete nucleic acid sequences of the present invention to encode a fusion protein. For example, to screen peptide libraries for inhibitors of the product of the nucleic acids of the present invention, it may be useful to encode a chimeric protein that can be recognised by a commercially available antibody. A fusion protein may also be engineered to contain a cleavage site located between the protein coding sequence and the heterologous protein sequence, so that the protein may be cleaved and purified away from the heterologous moiety.

In a further embodiment, the invention provides a host cell containing an integrated or episomal copy of any of the nucleotide sequences of the present invention or any functional parts thereof. In a more preferred embodiment, the invention provides a host cell containing a vector comprising a nucleic acid sequence according to the present invention. Said host cell is obtained from any organism including, but not limited to, mammals, such as humans, canines and rodents, amphibia, reptiles, birds, fish, nematodes, yeast, fungi, bacteria, insects and plants. In this regard, the term "functional parts" refers to any part of the nucleotide sequence of the present invention which exhibits substantially a similar, but not necessarily identical, activity as the complete nucleotide sequence, i.e. is able to modulate angiogenesis both *in vitro* and *in vivo.*

In a preferred embodiment, the invention provides an isolated polypeptide encodable by any of the herein mentioned nucleic acids, or a variant or a derivative thereof, or an immunologically active and/or functional fragment thereof.

It is thus understood that the present invention relates to a polypeptide having an amino acid sequence as given in SEQ ID NO 2 or 4, or a variant or a derivative thereof, or an immunologically active and/or functional fragment thereof.

"Variants" of a protein of the invention are those peptides, oligopeptides, polypeptides, proteins and enzymes which contain amino acid substitutions, deletions and/or additions relative to the said protein with respect to which they are a homologue, while maintaining the ability to modulate angiogenesis. In other words, the term "variant" refers to a polypeptide or protein differing from the polypeptide or protein of the present invention, but retaining essential properties thereof, i.e. modulation of angiogenesis. It will be understood that modulation of angiogenesis by these variants relates to inhibition as well as enhancing angiogenesis, irrespective of the functional activity of the protein the variant relates to. The present invention thus particularly contemplates dominant-negatives. In the present invention, the functional activity of a protein relates to the "function", which refers to the ability per se to modulate angiogenesis (a qualitative measure), and to the "activity" which refers to the amount of this ability to modulate angiogenesis per molecule (a quantitative measure). Generally, variants are overall closely similar, and, in many regions, identical to the polypeptide or protein of the present invention. For example, a homologue of said protein will consist of a bio-active amino acid sequence variant of said protein. To produce such homologues, amino acids present in the said protein can be replaced by other amino acids having similar properties, for example hydrophobicity, hydrophilicity, hydrophobic moment, antigenicity, propensity to form or break $\alpha$-helical structures or $\beta$-sheet structures, and so on. Amino acid substitutions are typically of single residues, but may be clustered depending upon functional constraints placed upon the polypeptide; insertions will usually be of the order of about 1-10 amino acid residues and deletions will range from about 1-20 residues. Preferably, amino acid substitutions will comprise conservative amino acid substitutions. It will be understood by the man skilled in the art, that said variants can easily be tested for their ability to modulate angiogenesis in any of the assays described in the present invention.

Insertional amino acid sequence variants of a protein of the invention are those in which one or more amino acid residues are introduced into a predetermined site in said protein. Insertions can comprise aminoterminal and/or carboxy-terminal fusions as well as intra-sequence insertions of single or multiple amino acids. Generally, insertions within the amino acid sequence will be smaller than amino or carboxyl terminal fusions, of the order of about 1 to 10 residues. Deletion variants of a protein of the invention are characterized by the removal of one or more amino acids from the amino acid sequence of said protein.

Amino acid variants of a protein of the invention may readily be made using peptide synthetic techniques well known in the art, such as solid phase peptide synthesis and the like, or by recombinant DNA manipulations. The manipulation of DNA sequences to produce variant proteins which manifest as substitution, insertion or deletion variants are well known in the art.

"Derivatives" of a protein of the invention are those peptides, oligopeptides, polypeptides, proteins and enzymes which comprise at least about 5 contiguous amino acid residues of said polypeptide but which retain the biological activity of said protein. Preferably said derivatives will comprise at least 6, 7, 8, 9, 10, 11, 12, 13, 14, or 15 contiguous amino acid residues of said protein. A "derivative" may further comprise additional naturally-occurring, altered glycosylated, acylated or non-naturally occurring amino acid residues compared to the amino acid sequence of a naturally-occurring form of said polypeptide. Alternatively or in addition, a derivative may comprise one or more non-amino acid substituents compared to the amino acid sequence of a naturally-occurring form of said polypeptide, for example a reporter molecule or other ligand, covalently or non-covalently bound to the amino acid sequence such as, for example, a reporter molecule which is bound thereto to facilitate its detection.

In the context of the current invention are embodied homologues, derivatives and/or immunologically active fragments of any of the newly identified sequences that modulate angiogenesis as defined above.

The term "homologue" relates to the molecule in a non-human species, that corresponds to the molecule of the present invention, i.e. able to modulate angiogenesis as measured in the method of the invention, with or without dose dependency.

In a preferred embodiment, the invention provides a method for producing the polypeptide of the present invention, the method comprising culturing said host cells of the invention as defined above under conditions allowing the expression of the polypeptide and recovering the produced polypeptide from the culture. Alternative methods for producing said polypeptides of the invention are well known in the art, such as, for example, chemical synthesis.

The present invention is also directed to polypeptides, which comprise, or alternatively consist of, an amino acid sequence which is at least 65%, 70%, 75%, 80%, 85%, 90%, 95%, 96%, 97%, 97,5%, 98%, 98,5%, 99% or 99,5% identical to the amino acid sequences of the present invention, wherein said amino acid sequence of the invention, the so-called reference sequence, is at least 30 amino acids in length. However, for reference sequences smaller than 30 amino acids the polypeptide must consist of an amino acid sequence which is at least 65%, 70%, 75%, 80%, 85%, 90%, 95%, 96%, 97%, 97,5%, 98%, 98,5%, 99% or 99,5% identical to the reference sequence.

By a polypeptide having an amino acid sequence of at least, for example, 95% "identity" to a reference amino acid sequence of the present invention, it is intended that the amino acid sequence of the polypeptide is identical to the reference sequence except that the amino acid sequence may include up to five amino acid alterations per each 100 amino acids of the reference polypeptide amino acid sequence. In other words, to obtain a polypeptide having a amino acid sequence at least 95% identical to a reference amino acid sequence, up to 5% of the amino acids in the reference sequence may be deleted or substituted with another amino acid, or a number of amino acids up to 5% of the total amino acids in the reference sequence may be inserted into the reference sequence. As a practical matter, whether any particular polypeptide is at least 65%, 70%, 75%, 80%, 85%, 90%, 95%, 96%, 97%, 97,5%, 98%, 98,5%, 99% or 99,5% identical to a polypeptide sequence of the present invention can be determined using known algorithms. A preferred method for determining the best overall match between a query sequence (a sequence of the present invention) and a subject sequence can be determined using BLASTp (Altschul et al., 1997).

Thus, the present invention relates particularly to a polypeptide having an amino acid sequence as given in SEQ ID NO 2 or 4, or a variant or a derivative thereof, or an immunologically active and/or functional fragment thereof.

**Antibodies**

In a preferred embodiment, the invention provides an antibody specifically recognising the polypeptides of the present invention, or a specific epitope of said polypeptide. The term epitope refers to portions of a polypeptide having antigenic or immunogenic activity in an animal, preferably a mammal, and most preferably in a human. Epitope-bearing polypeptides of the present invention may be used to induce antibodies according to methods well known in the art including, but not limited to, *in vivo* immunisation, *in vitro* immunisation, phage display methods or ribosome display.

The antibody of the present invention relate to any polyclonal or monoclonal antibody binding to an protein of the present invention. The term "monoclonal antibody" used herein refers to an antibody composition having a homogeneous antibody population. The term is not limiting regarding the species or source of the antibody, nor is it intended to be limited by the manner in which it is made. Hence, the term "antibody" contemplates also antibodies derived from camels (Arabian and Bactrian), or the genus lama. Thus, the term "antibody" also refers to antibodies derived from phage display technology or drug screening programs. In addition, the term "antibody" also refers to humanised antibodies in which at least a portion of the framework regions of an immunoglobulin are derived from human immunoglobulin sequences and single chain antibodies as described in U.S. patent No 4,946,778 and to fragments of antibodies such as $F_{ab}$, $F_{'(ab)2}$, $F_v$, and other fragments which retain the antigen binding function and specificity of the parent antibody. The term "antibody" also refers to diabodies, triabodies or multimeric (mono-, bi -, tetra- or polyvalent/ mono-, bi- or polyspecific) antibodies, as well as enzybodies, ie artificial antibodies with enzyme activity. Combinations of antibodies with any other molecule that increases affinity or specificity, are also contemplated within the term "antibody". Antibodies also include modified forms (e.g. mPEGylated or polysialylated form (Fernandes & Gregoriadis, 1997) as well as covalently or non-covalently polymer bound forms. In addition, the term "antibody" also pertains to antibody-mimicking compounds of any nature, such as, for example, derived from lipids, carbohydrates, nucleic acids or analogues e.g. PNA, aptamers (see Jayasena, 1999).

In specific embodiments, antibodies of the present invention cross-react with murine, goat, rat and/or rabbit homologues of human proteins and the corresponding epitopes thereof. Further included in the present invention are antibodies that bind polypeptides encoded by nucleic acids that hybridise to a polynucleotide of the present invention under stringent hybridisation conditions (as described herein). As such, the present invention provides a method for detecting the polypeptides of the present invention, the method comprising the use of the antibodies in immunoassays for qualitatively or quantitatively measuring levels of the polypeptides of the present invention in biological samples.

Thus the invention contemplates also a method for detecting a nucleic acid or a polypeptide as described herein, preferably by an antibody of the present invention.

In particular, the present invention relates to an antibody specifically recognising a polypeptide encodable by a nucleic acid according to the present invention.

Antibodies of the present invention may act as agonists or antagonists of the polypeptides of the present invention. Preferably, antibodies of the present invention bind an antigenic epitope as disclosed herein, or a particular portion of the proteins of the present invention.

Antibodies of the present invention may be used, for example, but not limited to, to purify, detect, target, and/or inhibit the activity of the polypeptides of the present invention, including both *in vitro* en *in vivo* diagnostic and therapeutic methods, as well as in drug screens (see infra).

## Antisense technology

Antisense technology can be used to control gene expression, for example for inhibition of gene expression, i.e. transcription, as described in the art. As such, antisense nucleic acids can be used as antagonist compounds, and may be employed to regulate the effects of the polypeptides of the present invention on the modulation of angiogenesis both *in vitro* and *in vivo.*
Thus, in a further embodiment, the present invention provides a polynucleotide sequence encoding the antisense nucleic acid of the invention. Such antisense nucleic acids can be constructed by recombinant DNA technology methods standard in the art.
In a preferred embodiment, the present invention provides a vector comprising an antisense nucleic acid. In a more preferred embodiment, said vector is an expression vector wherein the antisense polynucleotide sequence is operably linked to one or more control sequences allowing the expression, i.e. transcription, of said sequence in prokaryotic and/or eukaryotic host cells.
Potential antagonists according to the invention also include catalytic RNA, or a ribozyme. Ribozymes cleave mRNA at site-specific recognition sequences and can be used to destroy mRNAs corresponding to the polynucleotides of the present invention. The construction and production of ribozymes is well known in the art. As in the antisense approach, ribozymes of the invention can be used as antagonist compounds, and can be delivered to cells to, for example, inhibit *in vitro* or *in vivo* angiogenesis, e.g.; cell growth, migration, proliferation, capillary formation effects of the polypeptides of the present invention.
The invention further provides the nucleic acids sequences for controlling gene expression with the use of a small interfering RNA (siRNA, formerly known as double stranded RNA or dsRNA) approach. It has been described in the art (WO 99/32169) that providing siRNA to a target cell can result in the down regulation of the translation/ expression of any desired RNA sequence that may be present in said cell. As such, the nucleic acids of the present invention can be used as antagonistic or agonistic compounds, and may be employed to regulate the effects of the polypeptides of the present invention on the modulation of angiogenesis both *in vitro* and *in vivo.*
Thus, the present invention relates to siRNA for use as a medicament, characterised that said siRNA agonises or antagonises angiogenesis by said polynucleotide sequences.
Accordingly, the present invention relates to a cell, in which the polynucleotide sequences comprising the nucleic acids sequences as described herein have been introduced. It will be understood that said cell could be used as a medicament, in that said cell could be introduced in a patient suffering from pathological angiogenesis. Repopulating with said cells will be beneficial to the patient.

## Therapy and Diagnosis

Except for the female reproductive cycle, angiogenesis in the adult occurs mainly during wound healing and pathological conditions.
Pathological angiogenesis is well known in the art, and relates to a broad spectrum of pathological conditions, which are all associated with the (unwanted) formation of capillaries. For example, cancer, and in particular solid tumors, is the disease with the highest mortality rate that is associated with pathological angiogenesis. Tumors which can be vascularized include but are not limited to intracranial tumors, glioblastoma, meningioma, thyroid carcinomas, lung carcinomas, breast carcinomas, gastrointestinal tract tumors, esophageal carcinomas, gastric carcinomas, small bowel carcinomas, hepatocellular carcinomas, urinary tract carcinoma, kidney carcinoma, bladder carcinoma, female reproductive tract tumors, ovarian carcinomas, uterine cervix carcinoma, germ cell tumors, angiosarcoma.
A subset of cancers is characterized by the development of vascular tumors (haemangiomas). Growth factors specific for EC (e.g. VEGF) are directly involved in the development of vascular tumors. Vascular tumors may arise from genetic mutations or viral infections. Kaposi's sarcoma, is induced by the HIV-1 Tat protein which activates the VEGFR2, binds EC integrins and also retrieves bFGF, all molecules shown to be involved in angiogenesis.
Therefore, it is contemplated that anti-angiogenic therapy is beneficial in the treatment of cancer as well as AIDS.
Angiogenesis causes plaque rupture in atherosclerosis and blindness in premature newborns and in diabetic patients (retinopathy). In addition, inflammation is most of the times accompanied by angiogenesis and increased permeability. All the inflammatory cells are known to produce angiogenic and anti-angiogenic molecules. Therefore, diseases like psoriasis, ulcer formation, asthma, rheumatoid arthritis, bone and cartilage destruction, hepatitis, glomerulonephritis and many others are associated with angiogenic changes, i.e. pathological angiogenesis. Angiogenesis is also involved in adipogenesis. In order to survive, adipose tissue needs sufficient blood flow, and thus angiogenesis occurs during the accumulation of fat cells.
Therefore, it will be understood that if the angiogenic processes are disrupted, the pathological process are also inhibited. Hence, the present invention contemplates a number of anti-angiogenic strategies comprising the modulators according to the present invention. These strategies can be used alone or in combination with cytotoxic therapies in cancer. They can also be employed in the other hypoxia and inflammation driven pathological conditions described previously.

In addition, some of the receptors that play a role in angiogenesis, can serve as specific markers for the pathological process. Therefore, they can be used as diagnostic markers or as therapeutic markers that are used to target specific drugs towards the pathological tissue.

Hence the present invention relates to the use of a nucleic acid or a polypeptide or antibody as described herein for the preparation of a medicament for preparation of a medicament for therapeutic angiogenesis or for preventing, treating and/or alleviating diseases involving pathological angiogenesis.

<u>Therapeutic angiogenesis</u> relates to revascularising hypoxic tissues. A tissue becomes hypoxic, when the blood flow has been disturbed through occlusion or rupture. It has been shown that upon aging, the endothelium becomes dysfunctional, resulting in a weaker angiogenic response towards hypoxia. It is contemplated in the present invention, that in order to restore the original response, angiogenic growth factors (e.g. VEGF, bFGF) can be administered to the patient (as gene therapy or recombinant protein therapy). These factors then induce the formation of new capillaries that have to restore the blood flow and thus relieve the hypoxic status of the tissue.

Hence, the present invention relates to the use of a nucleic acid or a polypeptide or antibody as described herein for the preparation of a medicament for therapeutic angiogenesis.

In a preferred embodiment, the present invention relates to a pharmaceutical composition comprising a substantially purified nucleic acid, polypeptide or antibody according to the present invention for therapeutic angiogenesis or for preventing, treating and/or alleviating diseases involving pathological angiogenesis, and possibly in conjunction with a suitable carrier.

Suitable carriers for adding to the nucleic acids, polypeptides or antibodies of the present invention are well known in the art.

In a preferred embodiment, the present invention provides nucleic acids and fragments thereof, that can be used to regulate angiogenesis in a desired tissue or target cell, *in vitro* or *in vivo.*

In a preferred embodiment, the invention provides a method for regulating angiogenesis, the method comprising:

(a) introducing the expression vectors comprising the sample nucleic acids of the present invention in a desired target cell, *in vitro* or *in vivo,*
(b) expressing of said nucleic acids, and,
(c) regulating angiogenesis by the products expressed by said nucleic acids.

In a preferred embodiment, the invention provides polypeptides, including protein fusions, or fragments thereof, for regulating angiogenesis in a desired target cell, *in vitro* or *in vivo.* For example, inhibition of angiogenesis may occur as a direct result of administering polypeptides to mammalian, preferably human, cells. Delivering compositions containing the polypeptide of the invention to target cells, may occur via association via heterologous polypeptides, heterologous nucleic acids, toxins, or prodrugs via hydrophobic, hydrophilic, ionic and/or covalent interactions.

In another embodiment, the present invention provides antibody-based therapies for regulating angiogenesis in a desired target cell, *in vitro* or *in vivo.* Antibody-based therapies involve administering of anti-polypeptide or anti-polynucleotide antibodies to a mammalian, preferably human, cell. Methods for producing anti-polypeptide and anti-polynucleotide antibodies are known in the art. Such antibodies may be provided in pharmaceutically acceptable compositions as known in the art.

In a preferred embodiment, the present invention provides the nucleic acids, polypeptides or antibodies of the present invention for use as a medicament (both for treatment as for diagnosis of diseases). Said treatment according to the present invention refers to preventing, treating and/or alleviating diseases or disorders involving pathological angiogenesis or therapeutic angiogenesis as defined above and below.

Consequently, the present invention relates to the use of a nucleic acid, polypeptide or antibody as described herein for the preparation of a diagnostic kit for detecting pathological angiogenesis.

In an even more preferred embodiment, the present invention provides nucleic acids, polypeptides or antibodies of the present invention, for the preparation of a medicament for preventing, treating or alleviating diseases involving pathological angiogenesis or for therapeutic angiogenesis at the cellular level.

In one preferred embodiment the present invention provides a gene therapy method for treating, alleviating or preventing disorders and diseases involving pathological angiogenesis as well as therapeutic angiogenesis. The gene therapy methods relate to the introduction of nucleic acid sequences into an animal to achieve expression of a polypeptide of the present invention. This method requires a nucleic acid, which codes for a polypeptide of the invention that is operatively linked to a promotor or any other genetic element necessary for the expression of the polypeptide in the target tissue. Such gene therapy and delivery techniques are known in the art, see, for example, EP-A-0 707 071.

In one embodiment, the nucleic acid of the invention is delivered as a naked polynucleotide. The term naked nucleic acid refers to sequences that are free from any delivery vehicle that acts to assist, promote or facilitate entry into a cell, including viral sequences, viral particles, liposome formulations, lipofectin or precipitating agents and the like. The naked nucleic acids can be delivered by any method known in the art, including, but not limited to, direct needle injection at the delivery site, intravenous injection, topical administration, catheter infusion, and so-called "gene guns".

In another embodiment, the nucleic acids of the present invention may be delivered with delivery vehicles such as viral sequences, viral particles, liposome formulations, lipofectin or precipitating agents and the like. Viral vectors that can be used for gene therapy applications include, but are not limited to, a herpes virus vector, a baculovirus vector, a lentivirus vector, a retrovirus vector, an alphavirus vector, an adeno-associated virus vector or an adenoviral vector or any combination thereof. In a preferred embodiment, viral vectors used are replication deficient, for example such as described for adenoviral vectors in WO99/64582.

Delivery of the nucleic acids into a subject may be either direct, in which case the subject is directly exposed to the nucleic acid or nucleic acid-carrying vectors, or indirect, in which case, cells are first transformed with the nucleic acids *in vitro,* and then transplanted into the subject. These two approaches are known, respectively, as *in vivo* or *ex vivo* gene therapy and are well described.

In a more preferred embodiment, the present invention provides a gene therapy method treating, alleviating or preventing disorders and diseases involving pathological angiogenesis as well as therapeutic angiogenesis comprising the use of the vectors according to the present invention.

Cells into which nucleic acids or polypeptides of the present invention can be introduced, for example for therapeutic purposes, encompass any desired available cell type, including but not limited to endothelial cells, fibroblasts, hepatocytes, adipocytes, bone cells, blood cells such as T lymphocytes, B lymphocytes, monocytes, macrophages, neutrophils, eosinophils, megakaryocytes, mast cells, granulocytes and various stem or progenitor cells, in particular hematopoietic stem or progenitor cells.

In a preferred embodiment, the invention provides a method for treating, alleviating or preventing disorders involving pathological angiogenesis as well as a method for therapeutic angiogenesis comprising the use of a molecule, which allows to interfere with the expression of a polynucleotide and/or expression and/or functional activity of a polypeptide of the present invention in a patient in need of such a treatment.

In a preferred embodiment, the invention provides a kit for the diagnosis of pathological angiogenesis or the need for therapeutic angiogenesis comprising a nucleic acid of the invention, a probe or primer according of the invention, a polypeptide of the invention, or an antibody of the invention, possibly in conjunction with suitable buffers, means for detection or detection format parts (such as, for example, solid carriers, e.g. membranes). Suitable formats and technologies for designing diagnostic kits on the basis of the above are well known in the art. Preferred formats include any type of microarray format known in the art.

In a further embodiment, the invention provides a method for diagnosing pathological angiogenesis, the need for therapeutical angiogenesis, and/or a susceptibility to pathological angiogenesis, in a subject comprising the steps of:

(a) determining the presence or absence of a mutation in the nucleic acid of the invention, including mutations in the genomic and regulatory sequences of said nucleic acid, in a biological sample, and,
(b) diagnosing pathological angiogenesis, the need for therapeutical angiogenesis, and/or a susceptibility to pathological angiogenesis, based on the presence or absence of said mutation.

In another embodiment, the invention provides a method for diagnosing pathological angiogenesis, the need for therapeutical angiogenesis, and/or a susceptibility to pathological angiogenesis, in a subject comprising the steps of:

(a) determining the presence or amount of the nucleic acid of the invention or expression or functional activity of the polypeptide of the invention in a biological sample, and,
(b) diagnosing a pathological angiogenesis, the need for therapeutical angiogenesis, and/or a susceptibility to pathological angiogenesis, based on the presence or amount of said nucleic acid or expression of said polypeptide.

In another embodiment, the invention provides a method for diagnosing pathological angiogenesis, the need for therapeutical angiogenesis, and/or a susceptibility to pathological angiogenesis, in a subject comprising the steps of:

(a) determining the presence of a chromosomal translocation of the gene encoding for the nucleic acid of the present invention, and,
(b) diagnosing pathological angiogenesis, the need for therapeutical angiogenesis, based on the presence of a chromosomal translocation of said gene.

The diagnosis as described in the present invention may be preferably achieved by means of detection using probes, primers or antibodies of the invention.

**Drug screens**

The invention provides methods for identifying compounds or agents that can be used to treat disorders characterised by (or associated with) angiogenesis. These methods are also referred to herein as "drug screening assays" or "bioassays" and typically include the step of screening a candidate/test compound or agent for the ability to interact with (e.g., bind to) a protein of the present invention, in particular represented by SEQ ID NO 2, or 4, or any derivative, homologue, immunologically active or functional fragment thereof, to modulate the interaction of the protein of the present invention and a target molecule, and/or to modulate the expression of the nucleic acids of the present invention and/or activity of the proteins of the present invention. Candidate/test compounds or agents which have one or more of these abilities can be used as drugs to treat disorders characterised by pathological angiogenesis, disregulated expression of the nucleic acids of the present invention and/or disregulated functional activity of the proteins of the present invention. Candidate/test compounds such as antibodies, small molecules, e.g., small organic molecules and peptides, and other drug candidates can be obtained, for example, from combinatorial and natural product libraries.
The screening for therapeutic compounds may be any of a variety of drug screening techniques known in the art.
Thus, the present invention relates to the use of the nucleic acids, the polypeptides or antibodies as described herein for drug compound screens directed to identify drugs or antibodies that interfere with pathological angiogenesis.
In one embodiment, the invention provides a drug screening assay for screening candidate/test compounds which interact with (e.g., bind to) the proteins of the present invention, or any variant or a derivative thereof, or an immunologically active and/or functional fragment thereof. Typically, the assays are cell-free assays which include the steps of combining the proteins of the present invention, its catalytic or immunogenic fragments thereof, and a candidate/test compound, e.g., under conditions which allow for interaction of (e.g., binding of) the candidate/test compound to the protein of the present invention or portion thereof to form a complex, and detecting the formation of a complex, in which the ability of the candidate compound to interact with (e.g., bind to) the protein of the present invention or portion thereof is indicated by the presence of the candidate compound in the complex. Formation of complexes between the protein of the present invention and the candidate compound can be quantitated, for example, using standard immunoassays.
The proteins of the present invention, its catalytic or immunogenic fragments or oligopeptides thereof employed in such a test may be free in solution, affixed to a solid support, borne on a cell surface, or located intracellularly.
In another embodiment, the invention provides screening assays to identify candidate/test compounds which modulate (e.g., stimulate or inhibit) the interaction (and most likely the functional activity of the proteins of the present invention as well) between a protein of the present invention and a molecule (target molecule) with which the protein of the present invention normally interacts, or antibodies which specifically recognise the protein of the present invention. Examples of such target molecules include proteins in the same signaling path as the protein of the present invention, e.g., proteins which may function upstream (including both stimulators and inhibitors of activity) or downstream of the signalling pathways of the proteins of the present invention.
Typically, the assays are cell-free assays which include the steps of combining a protein of the present invention, its catalytic or immunogenic fragments thereof, a protein target molecule (e.g., a ligand to a protein of the present invention) or a specific antibody and a candidate/test compound, e.g., under conditions wherein but for the presence of the candidate compound, the protein of the present invention or biologically active portion thereof interacts with (e.g., binds to) the target molecule or the antibody, and detecting the formation of a complex which includes the protein of the present invention and the target molecule or the antibody, or detecting the interaction/reaction of the protein of the present invention and the target molecule or antibody.
Detection of complex formation can include direct quantitation of the complex by, for example, measuring inductive effects of the protein of the present invention. A statistically significant change, such as a decrease, in the interaction of the protein of the present invention and target molecule (e.g., in the formation of a complex between the protein of the present invention and the target molecule) in the presence of a candidate compound (relative to what is detected in the absence of the candidate compound) is indicative of a modulation (e.g., stimulation or inhibition) of the interaction between the protein of the present invention and the target molecule. Modulation of the formation of complexes between the protein of the present invention and the target molecule can be quantitated using, for example, an immunoassay.
Therefore, the present invention contemplates a drug screening assay for identifying compounds that modulate the interaction between binding partners in a complex, in which at least one of said binding partners is the polypeptide according to any of claims 25 or 26, or a variant or a derivative thereof, or an immunologically active and/or functional fragment thereof, and said method comprising:

(a) contacting a test compound with the complex, for a time sufficient to modulate the interaction in the complex; and thereafter
(b) monitoring said complex for changes in interactions, so that if a change in the interaction is detected, a compound that modulates the interaction is identified.

It should be clear that modulators for interaction between binding partners in a complex, when identified by any of the herein described methods, is contemplated in the invention.
To perform the above described drug screening assays, it is feasible to immobilise either the protein of the present invention or its target molecule to facilitate separation of complexes from uncomplexed forms of one or both of the proteins, as well as to accommodate automation of the assay. Interaction (e.g., binding of) of the protein of the present invention to a target molecule, in the presence and absence of a candidate compound, can be accomplished in any vessel suitable for containing the reactants. Examples of such vessels include microtitre plates, test tubes, and microcentrifuge tubes. In one embodiment, a fusion protein can be provided which adds a domain that allows the protein to be bound to a matrix. For example, the protein of the present invention can be "His" tagged, and subsequently adsorbed onto Ni-NTA microtitre plates (Paborsky et al., 1996), or ProtA fusions with the proteins of the present invention can be adsorbed to IgG, which are then combined with the cell lysates (e.g. $(35)^{S}$-labelled) and the candidate compound, and the mixture incubated under conditions conducive to complex formation (e.g., at physiological conditions for salt and pH). Following incubation, the plates are washed to remove any unbound label, and the matrix is immobilised. The amount of radioactivity can be determined directly, or in the supernatant after dissociation of the complexes. Alternatively, the complexes can be dissociated from the matrix, separated by SDS-PAGE, and the level of the protein binding to the protein of the present invention found in the bead fraction quantitated from the gel using standard electrophoretic techniques.
Other techniques for immobilising protein on matrices can also be used in the drug screening assays of the invention. For example, either the protein of the present invention or its target molecule can be immobilised utilising conjugation of biotin and streptavidin. Biotinylated protein molecules of the present invention can be prepared from biotin-NHS (N-hydroxy-succinimide) using techniques well known in the art (e.g., biotinylation kit, Pierce Chemicals, Rockford, III.), and immobilised in the wells of streptavidin-coated 96 well plates (Pierce Chemical). Alternatively, antibodies reactive with the protein of the present invention but which do not interfere with binding of the protein to its target molecule can be derivatized to the wells of the plate, and the protein of the present invention can be trapped in the wells by antibody conjugation. As described above, preparations of a protein binding to a protein of the present invention and a candidate compound are incubated in the wells of the plate presenting the protein of the present invention, and the amount of complex trapped in the well can be quantitated. Methods for detecting such complexes, in addition to those described above for the GST-immobilized complexes, include immunodetection of complexes using antibodies reactive with the target molecule to the proteins of the present invention, or which are reactive with the protein of the present invention and compete with the target molecule; as well as enzyme-linked assays which rely on detecting an enzymatic activity associated with the target molecule.
Another technique for drug screening which provides for high throughput screening of compounds having suitable binding affinity to the protein of the present invention is described in detail in "Determination of Amino Acid Sequence Antigenicity" by Geysen HN, WO Application 84/03564, published on 13/09/84, and incorporated herein by reference. In summary, large numbers of different small peptide test compounds are synthesised on a solid substrate, such as plastic pins or some other surface. The protein test compounds are reacted with fragments of the protein of the present invention and washed. Bound protein of the present invention is then detected by methods well known in the art. Purified protein of the present invention can also be coated directly onto plates for use in the aforementioned drug screening techniques. Alternatively, non-neutralising antibodies can be used to capture the peptide and immobilise it on a solid support.
This invention also contemplates a competitive drug screening assays in which neutralising antibodies capable of binding the proteins of the present invention specifically compete with a test compound for binding the protein of the present invention. In this manner, the antibodies can be used to detect the presence of any protein which shares one or more antigenic determinants with the protein of the present invention. In particular, the present invention pertains to a competitive drug screening assay comprising:

(a) competing the antibodies according to the present invention with a test compound for binding to the polypeptides, or a variant or a derivative thereof, or an immunologically active and/or functional fragment thereof, and,
(b) determining the amount of competition of said antibodies compared to said test compound.

In yet another embodiment, the invention provides a method for identifying a compound (e.g., a drug screening assay) capable of use in therapeutical angiogenesis or in the treatment of a disorder characterised by (or associated with) pathological angiogenesis, disregulated expression of the nucleic acids of the present invention or disregulated functional activity of the proteins of the present invention. Similarly, the invention provides a method for identifying a compound (e.g. a drug screening assay) capable of use in therapeutical angiogenesis or in the treatment of a disorder characterised by (or associated with) pathological angiogenesis, disregulated expression of the nucleic acids of the present invention or disregulated functional activity of the proteins of the present invention. The method typically includes the step of assaying the ability of the compound or agent to modulate the expression of the nucleic acid of the present invention or the functional activity of the protein of the present invention thereby identifying a compound for use in therapeutical angiogenesis or in the treatment of a disorder characterised by (or associated with) pathological angiogenesis, disregulated expression of the nucleic acids of the present invention or disregulated functional activity of the proteins of the present invention.

Preferably, the invention relates to a drug screening assay for identifying a compound capable of use in therapeutical angiogenesis or in the treatment of a disorder characterised by (or associated with) pathological angiogenesis, comprising:

(a) providing a cell comprising the nucleic acid according to the present invention, or any part thereof, or the polypeptide according to the present invention, or a variant or a derivative thereof, or an immunologically active and/or functional fragment thereof,
(b) providing a compound to the cell of step (a), under conditions which allow said compound to interact with said nucleic acid, or any part thereof, or with said polypeptide, or a variant or a derivative thereof, or an immunologically active and/or functional fragment thereof,
(c) assaying the ability of the compound of step (b) to modulate the expression of said nucleic acid, or any part thereof, or the activity or amount of said polypeptide, or a variant or a derivative thereof, or an immunologically active and/or functional fragment thereof, and,
(d) identifying the compound for treating a disorder characterised by disregulated angiogenesis.

Modulators of cell-cell interactions, the activity of the proteins and/or expression of the nucleic acids of the present invention identified according to these drug screening assays can be used to treat, for example, disorders characterised by or associated with disregulated cell-cell interactions.

In a preferred embodiment the invention provides a drug screening assay for screening compounds for therapeutic angiogenesis and/or for preventing, treating and/or alleviating pathological angiogenesis, comprising:

(a) contacting the compounds to be screened with a nucleic acid according to the present invention, or any part thereof, or a polypeptide according to the present invention, or a variant or a derivative thereof, or an immunologically active and/or functional fragment thereof, and,
(b) determining whether said compound effects an activity of said nucleic acid, or said part thereof, or said polypeptide, or a variant or a derivative thereof, or an immunologically active and/or functional fragment thereof.

The present invention also contemplates the use of competitive drug screening assays in which neutralising antibodies capable of binding polypeptides of the present invention specifically compete with a test compound for binding to the polypeptides or fragments thereof. In this manner, the antibodies are used to detect the presence of any peptide that shares one or more antigenic epitopes with a polypeptide of the invention. In particular, a screening assay for identifying antibodies that modulate the expression or functional activity of the polypeptides of the present invention, or a variant or a derivative thereof, or an immunologically active and/or functional fragment thereof, said method comprising:

(a) providing a cell comprising the polypeptide of the present invention, or a variant or a derivative thereof, or an immunologically active and/or functional fragment thereof,
(b) determining the expression and/or activity of said polypeptide, or a variant or a derivative thereof, or an immunologically active and/or functional fragment thereof,
(c) providing an antibody of the present invention, to the cell of (a), under conditions that said antibody can interact with said polypeptide, or a variant or a derivative thereof, or an immunologically active and/or functional fragment thereof,
(d) determining the modulation of expression and/or activity of said polypeptide, or a variant or a derivative thereof, or an immunologically active and/or functional fragment thereof after said antibody has bound said polypeptides, or a variant or a derivative thereof, or an immunologically active and/or functional fragment thereof.

The assays described herein include but are not limited to an Enzyme Linked Immunosorbent Assays (ELISAs) or cell based Enzyme Linked Immunosorbent Assays (CELISAs). Such assays allow screening for nucleic acid fragments, polypeptides, and therapeutic compounds using libraries of said compounds or molecules that influence or regulate the expression or function of the polypeptides subject of this invention. The expression of the polypeptides subject of this invention may be influenced, induced or inhibited by a target or test compound. In particular cases, said target or test compound is the expression product of a gene that is introduced into an acceptor cell. Said gene may be derived, for example, from a gene expression library. Said acceptor cells include, but are not limited to, human cells. Said gene expression libraries include, but are not limited to, adenoviral expression libraries.

In an especially preferred embodiment, the invention provides the compounds or products identified by the assays according to the present invention.

In a preferred embodiment, the invention provides a method for the production of a composition comprising the steps of admixing a compound identifiable by a method of the invention with a pharmaceutically acceptable carrier.

The nucleotide sequences presented in the present invention may be extended utilising a partial nucleotide sequence and employing various methods known in the art to detect the full sequence in case said sequence would only be a part of a coding region as well as upstream sequences such as promoters and regulatory elements. As such, the invention provides means and methods to regulate the expression of said nucleic acids by providing to a subject or host cell molecules that can positively or negatively influence said regulatory elements of said sequences identified in the present invention, e.g. by genetic modifier elements, chimaeric zinc finger-containing proteins or any other method known in the art.

The disclosure of all patents, publications (including published patent publications), and database accession numbers and depository accession numbers referenced in this specification are specifically incorporated herein by reference in their entirety to the same extent as if each such individual patent, publication, and database accession number, and depository accession number were specifically and individually indicated to be incorporated by reference.

It is to be understood that the following figures and examples are meant to illustrate the embodiments of the present invention and are in no way to be construed as limiting the present invention. The invention may be practised other than as particularly described and still be within the scope of the accompanying claims.

***Short description of the figures***

Figure 1: The different receptors and their ligands involved in angiogenesis.

Figure 2: Schematic representation of the construction of the adenoviral placenta library

Figure 3: Schematic representation of pIPspIAdapt6.VEGF165

Figure 4: Schematic representation of pIPspIAdapt6.IL3

Figure 5: Schematic representation of the HTS *in vitro* capillary assay

A: indirect ivcf
B: direct ivcf

Figure 6: Different steps during image analysis of *in vitro* capillary formation assay

Figure 7: *In vitro* Capillary formation induced by recombinant human VEGF165 protein and by conditioned medium of HeLa cells infected with Ad5.dE1.dE2A.VEGF165.

Figure 8: *In vitro* capillary formation induced by increasing amount of Ad5.dE1.dE2A.VEGF165 and Ad5.dE1.dE2A.eGFP viruses.

A: Induction of capillary formation by Ad5.dE1.dE2A.VEGF165 compared to Ad5.dE1.dE2A.eGFP.
B: Corresponding VEGF165 concentration at the same MOI's.

Figure 9: Determination hit rate VEGF virus.

A: set up of VEGF library plate.
B: Scatterplot of results VEGF library plate in *in vitro* capillary formation assay.

Figure 10: Induction of *in vitro* capillary formation using Ad5.dE1.dE2A.IL3 virus.

A: Induction of capillary formation by different MOI's of Ad5.dE1.dE2A.IL3 compared to Ad5.dE1.dE2A.eGFP.
B: Scatterplot showing all the IL3 datapoints and deduction of hit rate IL3 virus.

Figure 11: Induction of capillaries using Ad5.dE1.dE2A.VEGF165 in the direct *in vitro* capillary formation assay.

Figure 12: Control plate.

A: set up of the control plates containing 50µl of the indicated viruses and MOI's.
B: Evaluation of a control plate in the *in vitro* capillary assay.

Figure 13: Examples of results from plates obtained during the screening of 11000 viruses.

A: Scatterplots from 1 batch of library plates screened in the in vitro capillary assay.
B: Table showing the different thresholds calculated for the different batches of the screening.
C: Distribution of samples of plate 233174.
D: Results obtained for control plate during screening.
E: Overview of hits obtained during screening of 11000 viruses. Results are shown as fold induction of capillary formation.

Figure 14: Rescreening hits obtained during screening 11000 viruses (n =7)

Figure 15: Evaluation hits obtained from screening 11000 viruses in the direct in vitro capillary assay. (n=4)

Figure 16: Evaluation hits obtained from screening 11000 viruses in an EC proliferation assay. (n=2)

Figure 17: Effect of anti-angiogenic compounds on capillary formation

Figure 18: Nucleotide and deduced amino acid sequences of hits H3-34 (18.1) and H3-36 (18.2).

## *EXAMPLES*

### *Example 1: Library construction*

An arrayed adenoviral human placenta cDNA library was constructed and screened using an *in vitro* capillary formation assay. Under 'arrayed adenoviral cDNA library', we understand a collection of adenoviruses (in the present example, the adenoviruses are contained in 96 well plates) mediating the expression of various (human) cDNAs, in which every well contains a single virus type. Further details about the concept of arrayed adenoviral libraries can be found in WO 99/64582 (Arrayed adenoviral libraries for performing functional Genomics). The generation of a placental adenoviral cDNA library has been described (European patent application 01870038-5).
A schematic representation of the library construction is shown in Figure 2.

### *Example 2: HTS applicable in vitro capillary formation assay*

-Introduction:

A high throughput screening applicable *in vitro* capillary formation assay is developed to identify novel or known cDNAs involved in angiogenesis. In the present example, the *in vitro* capillary formation assay uses primary human umbilical vein endothelial cells (HUVEC) and bovine collagen type I extracellular matrix. Upon embedding of the HUVEC in the collagen an apoptotic signal is triggered, which can be overcome via addition of an angiogenic signal (e.g. VEGF). The supplied growth factor and the collagen then induce capillary formation.
To supply the angiogenic protein, we either infect the HUVEC directly in the collagen or we make use of producer cells, which are infected with the adenoviruses. After 48h the conditioned medium of these producer cells is transferred to the HUVEC embedded in the collagen. This is further incubated for 48h after which imaging analysis is performed to quantify the number of capillaries/ well (Figure 5).

- Preparation of the collagen:

Bovine collagen type I isolated from calf skin is resuspended in 0.1% acetic acid at a concentration of 3.3 mg/ml. This collagen is transferred to a dialysis membrane (cut off MW 12000 Da) and dialyzed for 30h at 4°C against a 0.1 x Modified Eagle's medium (MEM) solution containing 100 U/ml Penicilin and 100 μg/ml Streptomycin (P/S). The dialysis solution is refreshed after 6h and 18h. The collagen is stored at 4°C and is used during one month from the moment of dialysis. This procedure is based upon Montesani and Orci, 1985.

- HUVEC cell culture

HUVEC (passage 2: P2) were purchased as cells in culture from Clonetics and are cultured in Clonetics EBM2 supplemented medium, containing human epidermal growth factor, human fibroblast growth factor, vascular endothelium growth factor, ascorbic acid, hydrocortisone, human recombinant insulin like growth factor, heparin and FBS. The medium is refreshed every two days. The HUVEC arrived as a cell culture (P2) in a T75. The cells were expanded to a T185 upon 90% confluency (P3). Upon confluency, the cells are expanded to 3 x T175 (P4). At 90% confluency the cells are frozen and stored in liquid nitrogen. For the assay, HUVEC are thawed on Day 0 and cultured in a T75 (P4). On day 4, the cells are expanded to a T175 (P5). On day 7, the HUVEC are transferred to 3 x T175 (P6). Since HUVEC are cultured in EBM2 supplemented medium, which contains a number of growth factors, they display the activated phenotype. Therefore, the culture medium is changed on day 10 to M199/ RPMI1640 10% human serum 10% fetal bovine serum to make the cells quiescent. On day 12 the cells are used in the assay.

- The adenoviruses:

The adenoviruses used to set up the assay are the Ad5.dE1.dE2A.Adapt.VEGF165, Ad5dE1.E2A.plPspl.Adapt.VEGF165 and the Ad5dE1.dE2A.plPspl.Adapt.IL3. pAdaptVEGF165, Ad5.Adapt.VEGF165 and pCLIP.IL3 were obtained from CruCell, the Netherlands. The cDNA for VEGF165 was digested from pAdaptVEGF165 as a Hindll Xba I fragment and was cloned in the corresponding sites in plPspAdapt6 (Figure 3). Due to the introduction of a BamHI site at 3' of the VEGF coding sequence, the C-terminal residues are GS instead of RR. The cDNA for IL3 was cloned as a HindIII BamHI fragment in plPspAdapt6 (Figure 4). dE1/dE2A adenoviruses were generated from these adapter plasmids by co-transfection of the helper plasmid pWEAd5AflII-rITR.dE2A in PER.C6/E2A packaging cells, as described (WO99/64582).

- The assay:

The assay:

*Experimental background of the assay*

Initial *in vitro* capillary experiments were performed at the 24 well plate level with recombinant human VEGF165 protein. After evaluation of several parameters like the amount of collagen needed, the HUVEC cell density in the collagen matrix and the incubation time during which the capillary formation proceeds, we succeeded after non-routine trial and error and the application of inventive skill to adapt the assay to the 384 well plate level. In addition, we implemented the use of adenoviruses for the delivery of the angiogenic growth factors. At this moment two alternatives were analysed. In the first type of assay, the HUVEC are directly infected after embedding in the collagen matrix. In the other approach, a second cell line ('the producer cells') is infected with the adenovirus and after a certain time period, the resulting conditioned medium is transferred to HUVEC embedded in collagen. If the producer cells are infected with an adenovirus containing an angiogenic growth factor or a factor that induces the secretion of an angiogenic factor, the latter one would induce capillary formation upon transfer to the HUVEC.

Several producer cells, like A549, HeLa, PerC6 and U2OS were evaluated (see Table 1). From these, the HeLa and the U2OS cells yielded the best results. In addition, several media (OPTIMEM, Serum Free Medium (SFM), DMEM 2% FBS) or combinations of media (M199/RPMI1640 0.15% human serum 0.15% fetal bovine serum) were tested.

*Image analysis*

Introduction:

Because of the high throughput character of the assay (e.g. 9600 wells = images/day), automated image analysis of the assay is a prerequisite. In order to perform this image analysis, a picture of each well has to be recorded (9600 pictures/ day). Since a recorded image with bright light microscopy is troubled with the remnants of dead cells and the fact that the cells are located in a 3D structure, staining of the surviving cells is essential for the automated image analysis. Different staining methods have been described in the literature or have been tested by us (see Table 2). Surprisingly, the dye the most compatible with the HTS assay was calcein-AM. The use of Calcein-AM allowed to record sharp and clear images of all the wells, which could be used for image analysis.

Image Acquisition and Format:

The acquisition of the images for analysis and interpretation is a very important step towards a successful automatic system. In our case, we made use of a very fast scanning table using a high definition monochromatic CCD camera with a laser illumination source. As an initial setting we tried to adjust the best configuration in illumination, position and focus so to facilitate the image processing procedures. In this particular example, the images were stored in standard Tiff file format with a resolution of 1024x1024 pixels per image.

Image Processing:

In this procedure we started with the acquired image and proceeded with the transformation of the information on this image until we obtained a single measurement, which indicated the amount of capillary present on the image. Each step of this procedure is described below.

For computational manipulation of the image we have used a mathematical formalism; i.e. a continuous image is defined as an function *f (x,y)* having the intensity values on the respective x,y location. Since we are dealing with a digital image the values of x,y assume a discrete format which range from 0-1023 in our particular case. The matrix expression is represented by:

$$f(x,y) \approx \begin{bmatrix} f(0,0) & f(0,1) & \cdots & f(0,M-1) \\ f(1,0) & f(1,1) & \cdots & f(1,M-1) \\ \vdots & & & \vdots \\ f(N-1,0) & f(N-1,1) & \cdots & f(N-1,M-1) \end{bmatrix}, \qquad (1)$$

N and M assume the values of 1023. The value for the function *f* for each value of x and y represents the intensity of the image at the specific position. This intensity value varies from 0 to 255 grey levels; in which 0 represent the absence of light (black) and 255 the maximum value that represents light intensity (white). A typical image obtained in this experiment is show in the Figure 6.

<u>1) Correction of Non-uniform illumination.</u>

The main objective now was to identify the cell signal (strong white signal) against the background. On several images this background image presented a variation on intensity not only comparing two different images but also within the same image. Another problem is that we observed a field outside the well that has a dark representation. The objective with this procedure then is to bring on the background (well and plate) and the cell signal into a different level in grey. The following procedure was applied.

1.1 A window block of size m x n was created each having the minimum value of intensity in the equivalent m x n image window of the original image.

$$b(p,q) = \sum_{p=1}^{m} \sum_{q=1}^{n} \min(f(x,y)), \qquad (2)$$

1.2 Once this new image was created the resulting image was a subtraction of the original with the window block image.

$$f' = f - b. \quad (3)$$

An image intensity rescaling was then applied and the final image for threshold was obtained, as show in Figure 6.

2) Threshold.

At this point, the image was still composed by 256 levels of gray. Threshold is the process that we transform the gray level image into a bi-level image, having the white level representing the cell signal. To obtain this threshold we have made use of the histogram plot of the image. The threshold used was based on the median value criteria of the distribution, i.e.

$$t = \min - ((\min - \max) * p) \quad (4)$$

where min and max represents the gray value with minimum and maximum distribution respectively. In this expression p is a variable parameter, which depends on the value of the median. The resulting image is shown in Figure 6.

3) Skeletonization.

Since we wanted to define capillary formation and not complete cell signal a step towards the length measurement of the cell signal was necessary. The procedure consisted basically in thinning the connected component structure into single lines. However, the procedure had to be done in a way that the transformation still preserved the morphological appearance of the cell signal. This transformation was obtained through a common procedure in image processing called skeletonization, which in turn was obtained by the use mathematical morphology operators. The resulting image, applied in the threshold image, is shown in Figure 6.

4) Cluster selection.

As can be seen in the skeletonized image, various small and disconnected clusters can be found after this procedure. These small clusters do not contribute for the identification of capillary formation, even though they all represent cell signals. Thus, we eliminated those clusters from the final image.

5) Cluster count.

The final value for the amount of capillary present in an image is then taken as the total pixel count on the resulting image. In this example we obtained the value of 28482. Another interesting value that can be taken into consideration is the number or connected components, in this case 63. Note that if we have a larger value for the total pixel count and a small value for the number of connected components, this means that the image clusters are highly connected thus representing an image with a high capillary formation structure.

Data Analysis:

In our experiments we, used 384 well plates, which were automatically scanned and analyzed. We obtained the total distribution of the capillary formation measurement and calculated the threshold based on the samples of one plate. The threshold was set as the average of all 384 samples + (3 x the standard deviation over all 384 samples). Samples scoring higher than this threshold are considered positive and represent a hit in the primary screening.

*The indirect in vitro capillary formation assay*

In the following experiment the use of recombinant human VEGF165 is compared to the use of an adenovirus containing VEGF165 cDNA. Dialysis of collagen and the HUVEC culture was performed as described above. The HeLa producer cells were cultured in DMEM 10% FBS. 5000 HeLa cells/ well (384 well plate) were plated in 60 µl of endothelial specific serum free medium (SFM) supplemented with penicillin/streptomycin (P/S) (n=3 for each condition). 4h later, the cells were infected with 0.1 µl Ad5dE1.dE2A.Adapt.VEGF165 (approximately 1E10 viral particles (vp)/ml) or with 1µl Ad5.dE1.dE2A.plPspAdapt.eGFP (approximately 1E9vp/ml). These viruses were added to the cells in a final volume of 40µl SFM. 24h later, the proliferation medium (EBM2 supplemented) of the HUVEC P6 was changed to M199-RPMI1640 10% FBS 10% HS to induce quiescence. Quiescence of HUVEC is evaluated on the basis of morphology (elongated proliferating cells compared to a quiescent monolayer showing a cobblestone pattern). 48h after infection of the producer cells, quiescent HUVEC P6 are trypsinized, pelleted, resuspended in M199 and counted. A dilution of $5^{E}6$ HUVEC/ ml in M199 is prepared. The collagen matrix is prepared on ice by mixing 0.7 volumes dialyzed collagen, 0.2 volumes of $NaHCO_3$ and 0.1 volume of 10 x MEM. The $5^{E}6$ HUVEC/ ml dilution is added to collagen matrix in a volume corresponding to 10% of the total volume. 10 µl of the HUVEC collagen mixture is seeded in a 384 well plate with the aid of a multidrop, resulting in 5E3 HUVEC/ well. Following the polymerization of the collagen during 20 minutes at 37°C, 40µl of the conditioned medium from the HeLa producer cells or M199-RPMI1640 0.15%FBS 0.15% HS containing 50 ng/ml rhVEGF165 or control is transferred to the HUVEC embedded in the collagen. Capillary formation is allowed to proceed for 48h at 37°C 10% $CO_2$, after which the cells are stained by addition of 10µl 25µM calcein-AM (final concentration 5µM). Calcein-AM (Molecular Probes) is a probe, which is converted by an esterase to a fluorescent dye (Exc: 485; Em: 530), leading to specific fluorescence staining of living cells. After 30 minutes incubation at 37°C 10% $CO_2$, images are recorded and analysed for capillary length. The data shows that the background signal for the conditioned medium is higher then for the recombinant protein (Figure 7). However, the absolute signal of VEGF is also higher, resulting in a clear induction of capillary formation (rhVEGF165 yields a 14-fold induction compared to background, Ad5.dE1.dE2A.VEGF165 yields a 7-fold induction compared to Ad5.dE1.dE2A.eGFP).

In the next experiment, a dose response curve was set up with Ad5.dE1.dE2A.VEGF165 for the *in vitro* capillary formation assay, and these data were linked to the concentration of the produced VEGF165 in the same experiment. This experiment is performed in a completely automated fashion, as for the screening. A multidrop dispenser was used to seed HeLa cells in a 384 well plate in 60µl SFM medium at a cell density of 5000 cells/well. 4h later, the cells are infected with 1µl of the appropriate virus dilution to reach the different multiplicities of infection (MOI's) for Ad5.dE1.dE2A.VEGF165 and Ad5.dE1.dE2A.eGFP (MOI 25; 12; 6; 3; 1; no infection) by means of a 96 channel Hydra dispenser (Robbins). Infection efficiency, scored after 24h as GFP fluorescence in the wells infected with GFP virus, was OK. 48h later, the assay plate was prepared using the multidrop to seed quiescent HUVEC P6 mixed with collagen (5000 cells/10 µl collagen/well). When using the multidrop to dispense the collagen, airbubbles are frequently generated after polymerisation at 37°C 10% $CO_2$. Since these airbubbles introduced a considerable number of false positives after image analysis, the protocol was adapted. The collagen is seeded in plates which are preincubated at 4°C. After the dispensing, a further incubation at 4°C is performed for 20', which is followed by incubation at room temperature for 5 minutes to allow complete polymerisation. Surprisingly, it was observed that by doing so, the number of airbubbles was reduced considerably. In addition, this change of protocol did not influence the *in vitro* capillary formation. Samples (n=4) were collected from the producer plate to perform the VEGF165 ELISA (R&D systems). The VEGF165 ELISA was performed according to the manufacturer's instructions. 40µl of the conditioned medium of the remaining wells was transferred to the assay plate with the Hydra robot. The assay plate is incubated at 37°C 10% $CO_2$. 2 days later, the capillaries and other surviving cells were stained with calcein-AM via addition of 10µl 5µM calcein-AM (final concentration 1µM) with the multidrop. The cells are incubated for another 3h at 37°C 10% $CO_2$, after which images are taken from all the wells. Image analysis was performed with the algorithm as described herein (difference with screening: cluster selection was set more stringent: >200 pixels). There is a clear correlation between the concentration data of VEGF165 and the capillary inducing capacity of the different MOI's. Infection of HeLa cells with Ad5.dE1.dE2A. VEGF165 at a MOI of 25 yields +/- 40 ng/ml VEGF165 in the supernatant which results in a +/- 8 fold induction of capillary formation compared to infection with the Ad5.dE1.dE2A.eGFP virus (Figure 8.A). Increasing the MOI, increases the amount of VEGF165 produced by the HeLa cells and the capillaries induced by the HeLa conditioned medium (Figures 8A and 8B). It was observed that at MOI's higher then 25, a plateau was reached concerning the capillary inducing capacity of the HeLa conditioned medium, something which was also observed with recombinant VEGF165 at concentrations higher then 50 ng/ml (data not shown). A similar dose response curve was obtained when using U2OS cells as producer cells (data not shown).

To determine what would be the chance to pick up VEGF when present in the arrayed adenoviral cDNA library, a 96 well plate containing pIPspAdapt6VEGF165 in the columns 2-11, pIPspAdapt6empty and pIPspAdapt6eGFP plasmids in the first column (see set up plate Figure 9A) was used to prepare virus according to the library production protocol (= VEGF library plate). Because virus production does not yield 100% efficiency, not all the wells containing plasmid result in CPE positive wells. This transfection plate was screened in a fully automated way similar to the real screening. In short, the multidrop dispenser was used to seed HeLa cells in a 384 well plate at a cell density of 5000 cells/well using 60 µl SFM medium. The cells were infected with 1µl of crude lysate virus using the 96-channel Hydra dispenser (A1 96 well plate is pipeted into A1; A2; B1; B2 of the 384 well plate). After 48h, the assay plate was prepared as described previously and 40µl of the HeLa conditioned medium was transferred to the assay plate by means of the 96-channel Hydra dispenser. The assay plate is incubated at 37°C 10% CO2 during 48h, after which the viable cells are stained by addition of 10µl 5µM calcein-AM (Multidrop). Images of all the wells are recorded and the Galapagos algorithm was used to score the capillary formation. A cut off value is determined by adding three times the standard deviation of the background (Ad5.dE1.dE2A.empty and Ad5.dE1.dE2A.eGFP) to the average of the same background.

When using this cut off value, it is clear that 99.4% of all the CPE positive wells containing VEGF virus are positive, indicating that VEGF or a similar potent growth factor would be identified with a very high probability (Figure 9.B). This experiment also shows that even virus concentrations which do not result in CPE, can score positive in the capillary formation assay, although there is much more variation (values for one virus are located above and under cut off value). Therefore, the chance of identifying them is smaller then for CPE positive wells.

To evaluate whether weaker inducers of angiogenesis are also functional in our assay, the Ad5.dE1.dE2A. IL3 virus was tested in the *in vitro* capillary formation assay. In short, HeLa cells seeded as described above, were infected with Ad5.dE1.dE2A.IL3 or Ad5.dE1.dE2A.eGFP virus at a MOI of 200 and 50 (n= 8 for each condition). 48h later, the assay plate was prepared and the conditioned medium of the HeLa cells transferred to the assay plate which was further incubated at 37°C 10% CO2 for another 48h. Then the capillaries were stained with calcein-AM and images of the wells were recorded. The algorithm as described herein was used to calculate the amount of capillaries present in each well. Looking at the averages of the different MOI's, a dose response also becomes clear, although with a higher standard deviation (Figure 10A). When a cut off value was determined (average + (3* stdev) for eGFP virus), it is observed that a high number of IL3 viruses scores positive, although the number of false negatives is higher for IL3 than for VEGF165 (Figure 10B). The 75% success rate for IL3 is lower than for VEGF165, which is probably due to the fact that IL3 is a weaker angiogenesis inducer than VEGF. A success rate of 75% for a weak inducer is considered satisfactory.

*The direct in vitro capillary assay*

In contrast to the indirect *in vitro* capillary formation assay, which is thought to identify secreted factors or genes inducing the expression of the latter ones in the producer cells, the direct *in vitro* capillary formation assay can also result in the identification of EC intracellular molecules located downstream from the growth factor receptors on the EC.

Quiescent HUVEC P6 were trypsinized, pelleted and counted, after which a dilution of $5^E6$ cells/ml is prepared. 0.7 volumes of the dialyzed collagen was supplemented with 0.2 volumes of $NaHCO_3$ and 0.1 volume of 10 x MEM. The HUVEC were added to the collagen matrix in a volume corresponding to 10% of volume of the collagen matrix. 10 µl of the HUVEC and collagen mix was seeded in the wells of a 384 well plate with the multidrop. The plate was incubated at 4°C for 20 minutes to avoid air bubbles during polymerization. Then 40 µl of SFM medium containing 0.1 µl Ad5.dE1.dE2A.VEGF165 or 1µl Ad5.dE1.dE2A.eGFP virus was transferred on top of the collagen using the 96-channel Hydra dispenser. The assay plate was incubated at 37°C 10% $CO_2$ for 48h. Images of the wells were recorded after staining with 10 µl 5µM calcein-AM during 3h.

The experiment showed that direct infection of HUVEC with Ad5.dE1.dE2A.VEGF165 yields a 3-fold induction of *in vitro* capillary formation (Figure 11).

***Example 3: Screening of 11000 viruses in the in vitro capillary* assay *for capillary inducing cDNA's***

The control plates:

Control plates to check the screening procedure were prepared as follows: 4 wells in a 96 well plate were filled with 50µl dilutions of Ad5.dE1.dE2A.eGFP or Ad5.dE1.dE2A.VEGF165 virus to yield MOI's of 500, 200 and 50 (Figure 12.A). Virus stock solutions used to prepare the dilutions were obtained after propagation of the respective viruses in T175 flasks containing $5^E6$ PerC6.E2A cells. Propagation was allowed to proceed for 7 days untill full CPE was reached. Plates were stored at -80°C. Before the screening, one control plate was tested to evaluate their functionality.

HeLa cells were seeded in SFM medium at a cell density of 5000 cells/well in a 384 well plate. 4h later the 96-channel Hydra dispenser was used to infect the cells with 1µl of the viruses from a control plate (A1 from the 96 well plate is used to infect A1; A2; B1 and B2 in 384 well plate). The plates are incubated at 37°C 10% $CO_2$. Two days later the assay plate was prepared: quiescent HUVEC P6 were trypsinized, pelleted and counted. A $5^E6$ cells/ml dilution in M199 was prepared and used to add to the collagen matrix, which was supplemented with 0.2 volumes $NaHCO_3$ and 0.1 volume of 10 xMEM. Finally, 10µl of this mix of collagen and HUVEC's was seeded in a well of a 384 well plate, resulting in 5000 HUVEC/well. 40µl of conditioned medium from the HeLa cells infected with the viruses from the control plate was transferred to the collagen containing the HUVEC. 48h later, the capillaries were visualized by adding 10µl 5µM calcein-AM/well. 3h later the images of all wells were recorded and image analysis with the galapagos algorithm was performed. The results are summarized in Figure 12.B. When the cut off value is calculated based on the average and standard deviation of all GFP infected wells, 98% of all VEGF infected wells score positive. It clearly shows that all VEGF containing wells are scored positive compared to the eGFP containing wells, indicating that the control plates can serve as a positive control during the screening.

Procedure used during the screening

The assay was screened in such a way, that each virus (cDNA) was tested only once (one virus/ one well in the assay plate).

HeLa cells were cultured in DMEM 10% FBS. On day 1, the multidrop dispenser was used to seed HeLa cells in 60 µl SFM + P/S medium at a cell density of 5000 cells/well in a 384 well plate. 30 producer plates were prepared. The selected library propagation plates and 1 control plate were thawed at room temperature, after which they were stored at 4°C during the infection day. The viruses from the control plate were transferred manually to a 384 well plate. 4h after seeding the HeLa cells, cell attachment was checked and if the majority of all the cells were attached, the infection was started. To infect the producer plates, the 96-channel Hydra dispenser pipetted up to 5µl of virus from the propagation plate and dispensed 1µl of the virus in the producer plate. Extensive washing procedures between different infections were applied (3 x 25 µl bleach to decontaminate the needles and 3 x 55 µl water to remove the bleach from the needles). Each 4 library plates, viruses from the control plate were transferred to a producer plate. So, on a total of 20 library plates, 5 control plates were processed. After plate 10 and 20, two dummy plates were put in the source and destination position and the infection procedure was run. If there would be a contamination problem from one plate to another plate, then this would have been picked up by evaluation of the dummy plates for GFP. After the infection, the producer plates were incubated at 37°C 10% $CO_2$. At the end of the day the library propagation plates were returned to -80°C.

On day 2, the infection efficiency was determined by evaluating the GFP fluorescence in the producer plates infected with the control plate.

On day 3, the assay plate was prepared. HUVEC were cultured as described previously. Because of the lower speed of the Hydra, the assay plates were prepared in batches of five plates, introducing variability between different batches. The COSTAR assay plates were preincubated at 4°C. 3 x T175 flasks were trypsinized, pooled and pelleted. Cells were resuspended in M199 medium and counted with a Burker chamber. A cell dilution of $5^E6$ cells/ml was prepared in M199 medium. 12.6 ml of dialyzed collagen was mixed with 1.8 ml of 10 x MEM on ice. In addition, 3.6 ml of $NaHCO_3$ and 1.8 ml of the HUVEC dilution was added. The mixture was quickly vortexed and 10 µl/well was seeded in the assay plates using the multidrop dispenser. The assay plates were then incubated at 4°C for 20 minutes. Then 40 µl of the conditioned medium of the producer plates (4 library plates and 1 control plate) was transferred by means of the 96-channel Hydra dispenser. Between two transfers, the Hydra was washed extensively (3 x 60 µl bleach and 3 x 100 µl water).

On day 5, 10 µl of 5µM calcein-AM was added to all the wells with the aid of the multidrop. The plates were further incubated during 3h at 37°C 10% $CO_2$. Then images of all the wells were recorded (+/- 8 gigabyte in total) and analysed with the algorithm as described herein. During the screening, the algorithm used a cluster selection of 25 pixels, because in this way also surviving cells, which do not form capillaries, were counted. Then the data were imported in an excel file, where for each plate the average and the standard deviation over the whole plate was calculated. Based on these numbers the cut off value was determined, based on: average + (3* standard deviation). All wells, which had a value higher than the cut off, were scored positive and were being considered as a hit.

Results:

In total 40 Pheno Select library plates (11380 CPE positive wells) were evaluated in two separate runs.

The results of one batch of plates from the first run are shown as scatter plots (Figure 13.A). In addition, a distribution curve of plate 233174 is shown (Figure 13.C). It is clear that the data follow a Gaussian distribution, and that hits are represented as outliers. The inter batch variations are illustrated by the different thresholds shown in Figure 13.B. Since there can be a big difference for the threshold from batch to batch, it is safer to calculate the threshold per plate or per batch. To test the quality of the produced batch, a control plate was included in each batch (e.g. Figure 13.D). The hit rate of VEGF viruses on the control plates was 97% for both runs, which is corresponding the expectations.

Based on the cut off value determined per plate, respectively 30 and 32 hits were identified (excluding the IL3 positive controls; see later) (Figure 13.E), resulting in a hit rate of 0.56% and 058% for the two runs, respectively. Visual inspection of all the identified hits, eliminated 3 hits because of imaging mistakes. In total, 58 hits were retained from the primary screening.

Within the 40 PhenoSelect library plates that were screened, 23 plates contained control viruses in the first two columns. These Ad5.dE1.dE2A control viruses contain the cDNA encoding luciferase, GFP, lacZ, IL3, PLAP, ceNOS and empty virus. Thus, each of these plates contained 4 or less IL3 viruses (depending on the CPE scoring) on the positions M1/ M2/ N1/ N2, which served as an internal control during the screening of the *in vitro* capillary formation assay. The IL3 viruses showed a hit rate of 81% for both runs, which is close to the predicted value of 75% (see Example 2), indicating that the screening performed appropiately.

***Example 4: Rescreening of the hits originating from the primary screening***

The 58 adenoviruses selected from the primary screen were repropagated. PerC6.E2A cells were seeded at a cell density of 22500 cells/well in a 96 well plate in DMEM 10% FBS. The original library plates used in the screen were thawed at room temperature and stored at 4°C, untill 1µl of virus was picked manually from the selected wells and transferred to the PerC6.E2A cells. The 96 well plate was incubated at 34°C 10% $CO_2$ during 7 days. 3 viruses out of 58 did not repropagate and were not analyzed further. At the end of the propagation, the plate was stored at -80°C. After thawing at 37°C, the propagated viruses were aliquoted in a volume of 20µl in 384 well plates resulting in 6 aliquotation plates that were stored at -80°C.

To rescreen the hits from the primary screen, the propagated viruses were evaluated in three independent (n=2 or 3) *in vitro* capillary formation assays. A series of 24 Ad5.dE1.dE2A.VEGF and Ad5.dE1.dE2A.eGFP control viruses were manually applied on the aliquotation plate. Two of these experiments were performed in an identical way as described for the screening. The other one was performed according the same procedure, except for the 96-channel Hydra dispenser. All steps otherwise performed using the Hydra, were performed manually. In total 7 datapoints for each hit were obtained. These datapoints were analyzed based upon a cut off value determined on the average + (3 * standard deviation) of the Ad5.dE1.dE2A.eGFP viruses. Only the hits, which scored positive more than 4 out of 7 times were retained. Based on this criterion, three hits were retained: H3.7, H3.34 and H3.36. All datapoints for these three hits are shown in Figure 14.

***Example 5: Validation of hits***

All 58 hits from the primary screen were evaluated in the direct *in vitro* capillary formation assay.

HUVEC were cultured as described previously. The COSTAR assay plates are preincubated at 4°C. Quiescent HUVEC P6 were trypsinized, pooled and pelleted. Cells were resuspended in M199 medium and counted with a Burker chamber. A cell dilution of $5^E6$ cells/ml was prepared in M199 medium. 0.7 volumes of the dialyzed collagen was supplemented with 0.2 volumes of $NaHCO_3$ and 0.1 volume of 10 x MEM. The HUVEC were added to the collagen matrix in a volume corresponding to 10% of volume of the collagen matrix. 10 µl of the HUVEC and collagen mix was seeded in the wells of a 384 well plate with the multidrop. The plate was incubated at 4°C for 20 minutes to avoid air bubbles during polymerization. In the meanwhile, the multidrop was used to add 60µl of SFM +P/S in each well of a 384 well plate. 1µl of virus, from an aliquotation plate of the repropagated hits supplemented with VEGF and eGFP control viruses, was added to the wells with the Hydra (same program as to infect the producer cells). This virus containing SFM medium was then transferred from the latter 384 plate to the 384 well plate containing the HUVEC embedded in the collagen. This was performed using the 96 channel Hydra dispenser in an identical way as for the transfer of conditioned medium from a producer plate to an assay plate. The plates were incubated at 37°C 10% $CO_2$ for 48h.

On day 3, 10 µl of 5µM calcein-AM was added to all the wells with the aid of the multidrop. The plates were further incubated during 3h at 37°C 10% $CO_2$. Then images of all the wells were recorded and analysed with the algorithm as described herein, using a cluster selection of 25 pixels. Then the data were imported in an excel file, where for each plate the average and the standard deviation over the whole plate was calculated. Based on these numbers the cut off value is determined: average + (3* standard deviation). All wells, which have a value higher than the cut off, were scored positive.

4 data points for all of the hits were obtained in three independent experiments. In contrast to the positive control (Ad5.dE1.dE2A.VEGF165), none of the hits scored positive, except for H3.10, which scored 2 out of 4 times positive (Figure 15). H3.10 scored in the indirect *in vitro* capillary assay only 1 time. H3.7, H3.34 and H3.36 did not score positive, underscoring the different character of the approach.

In another series of experiments, the effect of the hits on the proliferation of HUVEC was assessed. Proliferation of HUVEC was induced through the use of conditioned medium from adenovirus infected U2OS cells. Proliferation was measured via BrdU incorporation and subsequent chemilumniscent detection of the antibody directed against incorporated BrdU (Roche).

U2OS cells were cultured in DMEM 10% FBS at 37°C 10% $CO_2$. On day 1, U2OS cells were seeded in 40µl SFM + P/S at a cell density of 5000 cells/ well in a 384 well plate. 4h later the cells were infected with viruses from an aliquotation plate of the repropagated hits supplemented with VEGF and eGFP control viruses. The Hydra robot and the corresponding infection program used to infect the HeLa producer cells for the *in vitro* capillary assay was applied. On day 2 the infection efficiency was determined by evaluating the GFP fluorescence in the wells infected with the Ad5.dE1.dE2A.eGFP viruses.

HUVEC were cultured as described in previously. On day 3, quiescent HUVEC P6 were seeded with the aid of a multidrop in a 384 well plate in 20 µl M199 5% FBS at a cell density of 1500 cells/ well. The cells were supplemented with 10 µl of conditioned medium from the U2OS cells, which was transferred with the Hydra robot. Extensive washing of the needles between the different transfers was applied (3 x 80 µl bleach and 3 x 100µl water). On day 5, 6µl BrdU was added to each well and incubated for another 4h at 37°C 10% $CO_2$. After aspirating off the BrdU, 20 µl FixDenat solution was applied at room temperature during 30 minutes to fix the cells and denature the DNA. This solution was aspirated and 10 µl of the anti-BrdU-POD Fab fragment was added to the well and the wells were incubated for 1h30 minutes at room temperature. Extensive washing (360 µl) with PBS was performed to remove unbound antibody, after which 10 µl of the luminol substrate was added to each well. The samples were shaken for 3 minutes at room temperature and were subsequently measured in the luminometer (300 ms; PMT 893; LumiAscent, LabSystems). The measured chemiluminescence correlated to the amount of antibody present in the well, which was indicative for the number of proliferative cells. Again, a cut off value was determined based on the average +(3 x standard deviation) of the GFP control viruses. When using this criterion, only 2 hits scored positive: H3.7 and H3.34 induced proliferation 4-5 and 3-4 fold, respectively (see Figure 16). H3.10 and H3.36 had only a minor or no effect.

***Example 6: the in vitro capillary*** **assay** ***for anti-angiogenic proteins or peptides***

A multidrop dispenser was used to seed HeLa cells in a 384 well plate in 60µl SFM medium at a cell density of 5000 cells/ well. 4h later, the cells were infected with 1µl of Ad5.dE1.dE2A.VEGF165 or Ad5.dE1.dE2A.eGFP by means of a 96 channel Hydra dispenser (Robbins). In addition, the cells were infected with 1 µl empty virus or 1µl of Ad5.dE1.dE2A.angiostatin.. 48h later, the assay plate was prepared using the multidrop to seed quiescent HUVEC P6 mixed with collagen (5000 cells/10 µl collagen/ well. Plates were incubated at 4°C and room temperature during 20 ' and 5' respectively. 40µl of the conditioned medium of the producer cells, treated as described above, was transferred to the assay plate with the Hydra robot. The assay plate was incubated at 37°C 10% $CO_2$. 2 days later, the capillaries and other surviving cells were stained with calcein-AM via addition of 10µl 5µM calcein-AM (final concentration 1µM) with the multidrop. The cells were incubated for another 3h at 37°C 10% $CO_2$, after which images were taken from all the wells. Image analysis was performed with the algorithm as described herein. It is clear that conditioned medium from producer cells co-infected with VEGF165 virus and empty virus induces capillary formation. However, when the VEGF165 virus is co-infected with the angiostatin virus, the capillary formation was strongly reduced (Figure 17), showing the negative effect of angiostatin on angiogenesis. These results indicate that this assay can be used to screen for anti-angiogenic molecules.

***Example 7: Polynucleotides and polypeptides of the invention***

<u>7.1 Features of Hit H3-34</u>

Hit H3-34 has been detected as an inducer of capillaries in the EC capillary formation assay.
This cDNA sequence (Figure 18.1, SEQ ID NO: 1) is nearly identical to *Homo sapiens* interferon regulatory factor I (IRF1), as described in Genbank record with accession number NM_002198. The H3-34 cDNA does not contain 176 bps at the 5' end, but is 419 bps longer at the 3' end as compared to the sequence described in NM_002198.
Based on the genomic sequence (chromosome 5 fragment given in Genbank record NT_026285), these extra bases at the 3' end are an extension of the 3' untranslated region of this gene.
Based on the order number and similarity with published sequences, open reading frame (ORF) number 7 most likely will be the coding sequence of this cDNA.. ORF 7 matches perfectly with the protein sequence of Genbank record NP_002189. ORF 7 on the direct strand extends from base 22 to base 999. The translational product of ORF 7 is depicted in Figure 18.1; SEQ ID NO: 2.
IRF1 encodes interferon regulatory factor 1, a member of the interferon regulatory transcription factor (IRF) family. IRF1 serves as an activator of interferons alpha and beta transcription, and in mouse it has been shown to be required for double-stranded RNA induction of these genes. IRF1 also functions as a transcription activator of genes induced by interferons alpha, beta, and gamma. Further, IRF1 has been shown to play roles in regulating apoptosis and tumor-suppression.
Therefore, the finding, as disclosed in the present invention, that IRF1 and proteins related thereto, induce capillary formation, provides new and unexpected insights in the function(s) of IRF1 and related proteins.

<u>7.2 Features of Hit H3-36</u>

Hit H3-36 has been detected as an inducer of capillaries in the EC capillary formation assay.
This cDNA sequence (Figure 18.2, SEQ ID NO: 3) maps perfectly on human chromosome 17, identified by GenBank accession number NT_024901.
Based on the order number open reading frame (ORF) number 2 most likely will be the coding sequence of this cDNA.. ORF 2 on the direct strand extends from base 142 to base 240. The translational product of ORF 2 is depicted in Figure 18.2; SEQ ID NO: 4.

No functional annotations have been detected for this cDNA by database searchings.

Therefore, the finding, as disclosed in the present invention, that the sequence of Hit 3-36 induces capillary formation, provides new and unexpected insights in the function(s) of this sequence.

**Tables:**

Table 1:

| Comparison of different dyes to stain EC capillaries *in vitro* and their assay/HTS image analysis compatibility: | | |
|---|---|---|
| *dye* | *Assay/HTS image analysis compatibility* | *reason* |
| Toluidine blue | Not ok | a) Fixation with glutaraldehyde is needed (extra step)<br>b) Heterogenous cell staining |
| $DiI_{C12}$ dialkylcarbocyanins | Not ok | a) Heterogenous cell staining<br>b) Staining of dead cells |
| CMFDA 5-chloromethylfluorescein diacetate | Not ok | a) Staining of dead cells<br>b) Weak signal |
| CMAC 7-amino-4-chloromethylcoumarin | Not ok | a) Staining of dead cells<br>b) Weak signal |
| Calcein-AM | ok | a) One step addition at end of assay<br>b) Stains only the living cells<br>c) Strong signal |

Table 2:

| Different cell types evaluated as producer cells in the indirect assay: | |
|---|---|
| *Cell type* | *Assay compatibility* |
| PerC6 | No |
| A549 | Yes |
| HeLa | Yes |
| U2OS | Yes |

**REFERENCES**

Altschul, S.F., Madden, T.L., Schaffer, A.A., Zhang, J., Zhang, Z., Miller, W. and Lipman, D.J. (1997) Gapped BLAST and PSI-BLAST: a new generation of protein database search programs. Nucleic Acids Research 25: 3389-3402.

Bischoff, J. (1997). Cell adhesion and angiogenesis. J Clin Invest *100*, S37-39.

Carmeliet, P., and Collen, D. (2000). Transgenic mouse models in angiogenesis and cardiovascular disease. J Pathol *190,* 387-405.

Carmeliet, P., and Jain, R. K. (2000). Angiogenesis in cancer and other diseases. Nature *407,* 249-257.

Celletti, F. L., Waugh, J. M., Amabile, P. G., Brendolan, A., Hilfiker, P. R., and Dake, M. D. (2001). Vascular endothelial growth factor enhances atherosclerotic plaque progression. Nat Med *7*, 425-429.

Cockerill, G. W., Gamble, J. R., and Vadas, M. A. (1995). Angiogenesis: models and modulators. Int Rev Cytol *159*, 113-160.

Davis, G. E., and Camarillo, C. W. (1996). An alpha 2 beta 1 integrin-dependent pinocytic mechanism involving intracellular vacuole formation and coalescence regulates capillary lumen and tube formation in three-dimensional collagen matrix. Exp Cell Res *224*, 39-51.
Deroanne, C. F., Hajitou, A., Calberg-Bacq, C. M., Nusgens, B. V., and Lapiere, C. M. (1997). Angiogenesis by fibroblast growth factor 4 is mediated through an autocrine up-regulation of vascular endothelial growth factor expression. Cancer Res *57*, 5590-5597.
Dimmeler, S., and Zeiher, A. M. (2000). Akt takes center stage in angiogenesis signaling. Circ Res *86*, 4-5.
Fernandes, A. & Gregoriadis,G., Polysialylated asparaginase: preparation, activity and pharmacokinetics. Biochem. Biophys. Acta, *1341*, 26-34, 1997
Ferrara, N. (1999). Molecular and biological properties of vascular endothelial growth factor. J Mol Med *77*, 527-543.
Gale, N. W., and Yancopoulos, G. D. (1999). Growth factors acting via endothelial cell-specific receptor tyrosine kinases: VEGFs, angiopoietins, and ephrins in vascular development. Genes Dev *13*, 1055-1066.
Goede, V., Schmidt, T., Kimmina, S., Kozian, D., and Augustin, H. G. (1998). Analysis of blood vessel maturation processes during cyclic ovarian angiogenesis. Lab Invest *78*, 1385-1394.
Haas, T. L., Davis, S. J., and Madri, J. A. (1998). Three-dimensional type I collagen lattices induce coordinate expression of matrix metalloproteinases MT1-MMP and MMP-2 in microvascular endothelial cells. J Biol Chem *273*, 3604-3610.
Jayasena, S.D. (1999) Aptamers: an emerging class of molecules that rival antibodies in diagnostics. Clin. Chem. 45: 1628-1650.
Klohs, W. D., and Hamby, J. M. (1999). Antiangiogenic agents. Curr Opin Biotechnol *10*, 544-549.
Kuzuya, M., Satake, S., Ramos, M. A., Kanda, S., Koike, T., Yoshino, K., Ikeda, S., and Iguchi, A. (1999). Induction of apoptotic cell death in vascular endothelial cells cultured in three-dimensional collagen lattice. Exp Cell Res *248,* 498-508.
Montesano, R., and Orci, L. (1985). Tumor-promoting phorbol esters induce angiogenesis in vitro. Cell *42,* 469-477.
Paborsky, et al. (1996) Anal. Biochem. 234: 60-65.
Phillips, P. G., Birnby, L. M., and Narendran, A. (1995). Hypoxia induces capillary network formation in cultured bovine pulmonary microvessel endothelial cells. Am J Physiol *268*, L789-800.
Sambrook et al. (1989) Molecular Cloning: A laboratory manual. Cold Spring Harbor laboratory press.
Satake, S., Kuzuya, M., Ramos, M. A., Kanda, S., and Iguchi, A. (1998). Angiogenic stimuli are essential for survival of vascular endothelial cells in three-dimensional collagen lattice. Biochem Biophys Res Commun *244*, 642-646.
Sierra-Honigmann, M. R., Nath, A. K., Murakami, C., Garcia-Cardena, G., Papapetropoulos, A., Sessa, W. C., Madge, L. A., Schechner, J. S., Schwabb, M. B., Polverini, P. J., and Flores-Riveros, J. R. (1998). Biological action of leptin as an angiogenic factor. Science *281*, 1683-1686.
Soille, P. (1999) "Morphological image analysis" , Springer Verlag Berlin Heidelberg.
Yancopoulos, G. D., Klagsbrun, M., and Folkman, J. (1998). Vasculogenesis, angiogenesis, and growth factors: ephrins enter the fray at the border. Cell *93*, 661-664.
Yla-Herttuala, S., and Martin, J. F. (2000). Cardiovascular gene therapy. Lancet *355*, 213-222.

## Claims

1. A method for identifying a sample nucleic acid that modulates angiogenesis, said method comprising:

   (a) embedding endothelial cells (ECs), in particular HUVECs or hMVECs in an extracellular matrix, preferably collagen,
   (b) introducing a sample nucleic acid into said ECs,
   (c) assaying the phenotype and in particular the capillary formation, proliferation and/or survival of the ECs which were contacted with said sample nucleic acid, and
   (d) identifying the sample nucleic acid that caused the capillary formation, proliferation and/or survival as determined in step (c).

2. A method for identifying a sample nucleic acid that modulates angiogenesis, said method comprising:

   (a) introducing a sample nucleic acid into producer cells,
   (b) incubating the producer cells into which the sample nucleic acid has been introduced according to step (a) for an appropriate period of time to produce conditioned medium,
   (c) contacting said conditioned medium of step (b) with ECs, in particular HUVECs or HMVECs, which are embedded in an extracellular matrix, preferably collagen,
   (d) assaying the phenotype and in particular the capillary formation, proliferation and/or survival of the ECs which were contacted with said conditioned medium according to step (c), and,
   (e) identifying the sample nucleic acid of step (a) that caused the capillary formation, proliferation and/or survival as determined in step (d).

3. A method for validating the sample nucleic acid identified by a method according to any of claims 1 or 2, in which said sample nucleic acid is further validated for modulating migration.

4. The method according to any of claims 1 to 3, being **characterised in that** at least the assaying step is performed in a high-throughput format.

5. The method according to any of claims 1 to 4, **characterised in that** said method comprises the step of preventing and/or dissolving air bubbles in the extracellular matrix.

6. The method according to any of claims 1 to 5 wherein said EC are additionally stained for viability, in particular by calcein-AM.

7. The method according to any of claims 1 to 6, wherein said assaying the phenotype is performed in an automated setting, comprising the steps of:

   (a) image acquisition,
   (b) image processing, comprising:

      (I) optionally auto-focussing,
      (II) optionally correcting for non-uniform illumination,
      (III) transformation of the gray level into a bi-level image,
      (IV) skeletonisation or area determination of the formed capillaries by mathematical morphology operators,
      (V) elimination of small clusters,
      (VI) cluster count, and,

   (c) data analysis.

8. An isolated nucleic acid comprising a member selected from a group of nucleic acids identifiable as modulators of angiogenesis according to the method of any of claims 1 to 7, said group consisting of:

   (a) a nucleic acid comprising a DNA sequence as given in SEQ ID NO 1 or 3, or the complement thereof,
   (b) a nucleic acid comprising the RNA sequences corresponding to SEQ ID NO 1 or 3, or the complement thereof,
   (c) a nucleic acid specifically hybridising to the nucleotide sequence as defined in (a) or (b),
   (d) a nucleic acid having a nucleotide sequence at least 65% identical to the sequence defined in (a),
   (e) a nucleic acid encoding a protein with an amino acid sequence which is at least 65% identical to the amino acid sequence as given in SEQ ID NO 2 or 4,
   (f) a nucleic acid encoding a protein comprising the amino acid sequence as given in any of SEQ ID NO 2 or 4,
   (g) a nucleic acid which is degenerated as a result of the genetic code to a nucleotide sequence of a nucleic acid as given in SEQ ID NO 1 or 3, or as defined in (a) to (f),
   (h) a nucleic acid which is diverged due to differences in codon usage between organisms to a nucleotide sequence encoding a protein as given in SEQ ID NO 2 or 4, or as defined in (a) to (g),
   (i) a nucleic acid which is diverged due to the differences between alleles encoding a protein as given in SEQ ID NO 2 or 4, or as defined in (a) to (h),
   (j) a nucleic acid encoding an immunologically active and/or functional fragment of a protein encoded by a DNA sequence as given in SEQ ID NO 1 or 3,
   (k) a nucleic acid encoding a gene family member of the nucleic acid as given in SEQ ID NO 1 or 3, and,
   (l) a nucleic acid encoding a protein as defined in SEQ ID NO 2 or 4, or a nucleic acid as defined in any one of (a) to (k) **characterised in that** said sequence is DNA, cDNA, genomic DNA or synthetic DNA.

9. An antibody specifically recognising a polypeptide encodable by a nucleic acid according to claim 8.

10. Use of a nucleic acid, a polypeptide or antibody according to claim 8 or 9 for the preparation of a medicament for therapeutic angiogenesis or for preventing, treating and/or alleviating diseases involving pathological angiogenesis.

Figure 1: different receptors and their ligands involved in angiogenesis

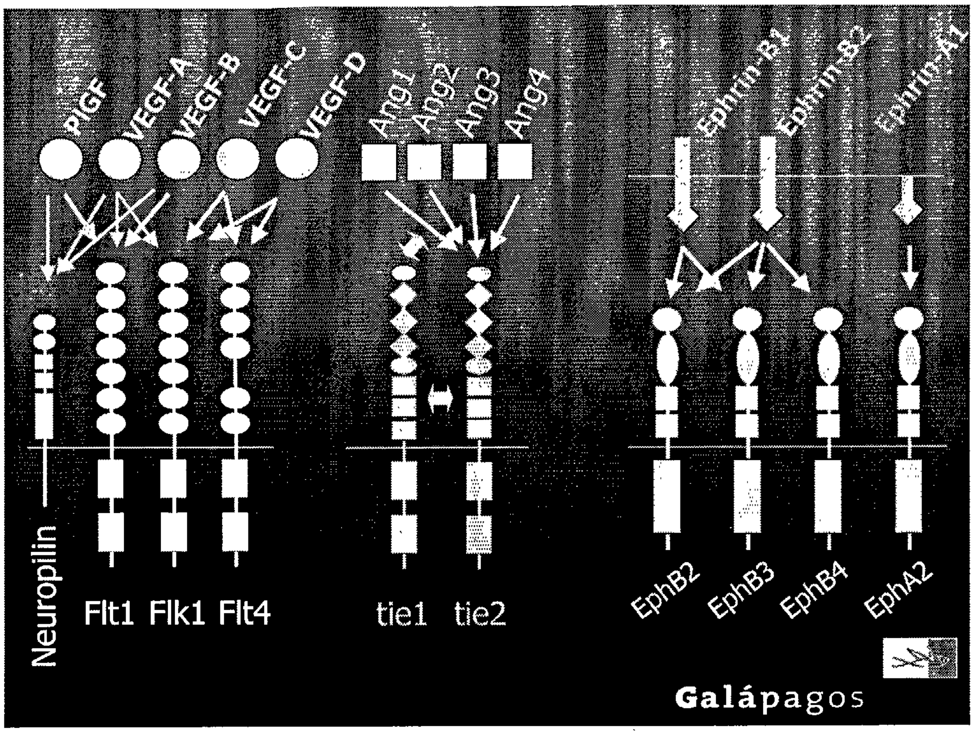

Figure 2: library construction:

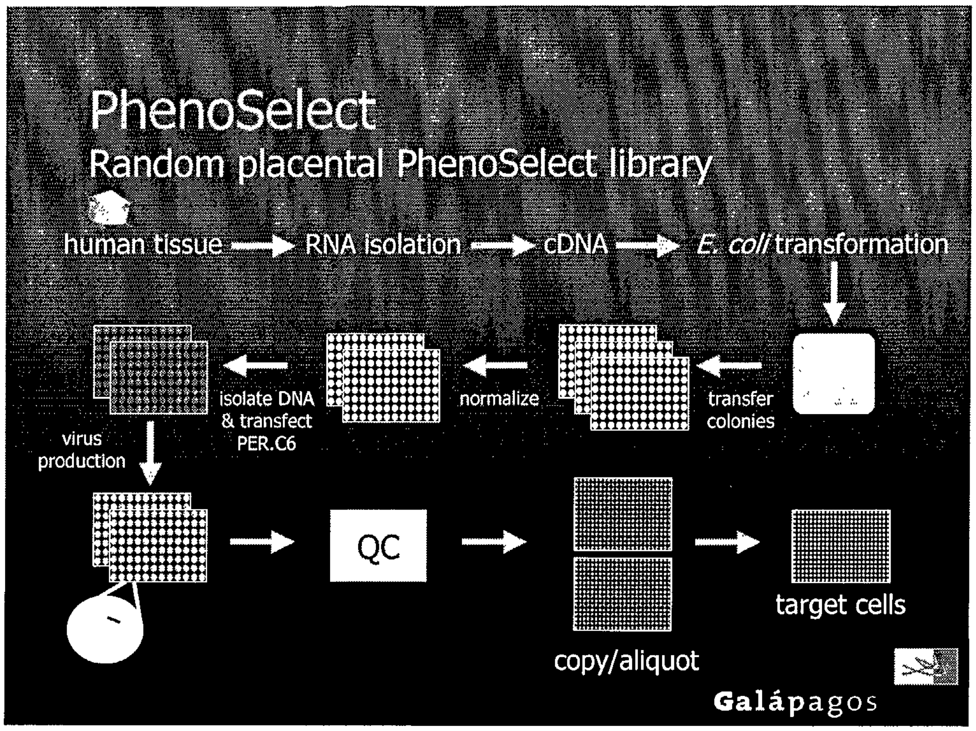

Figure 3: Schematic representation of pIPspIAdapt6.VEGF165:

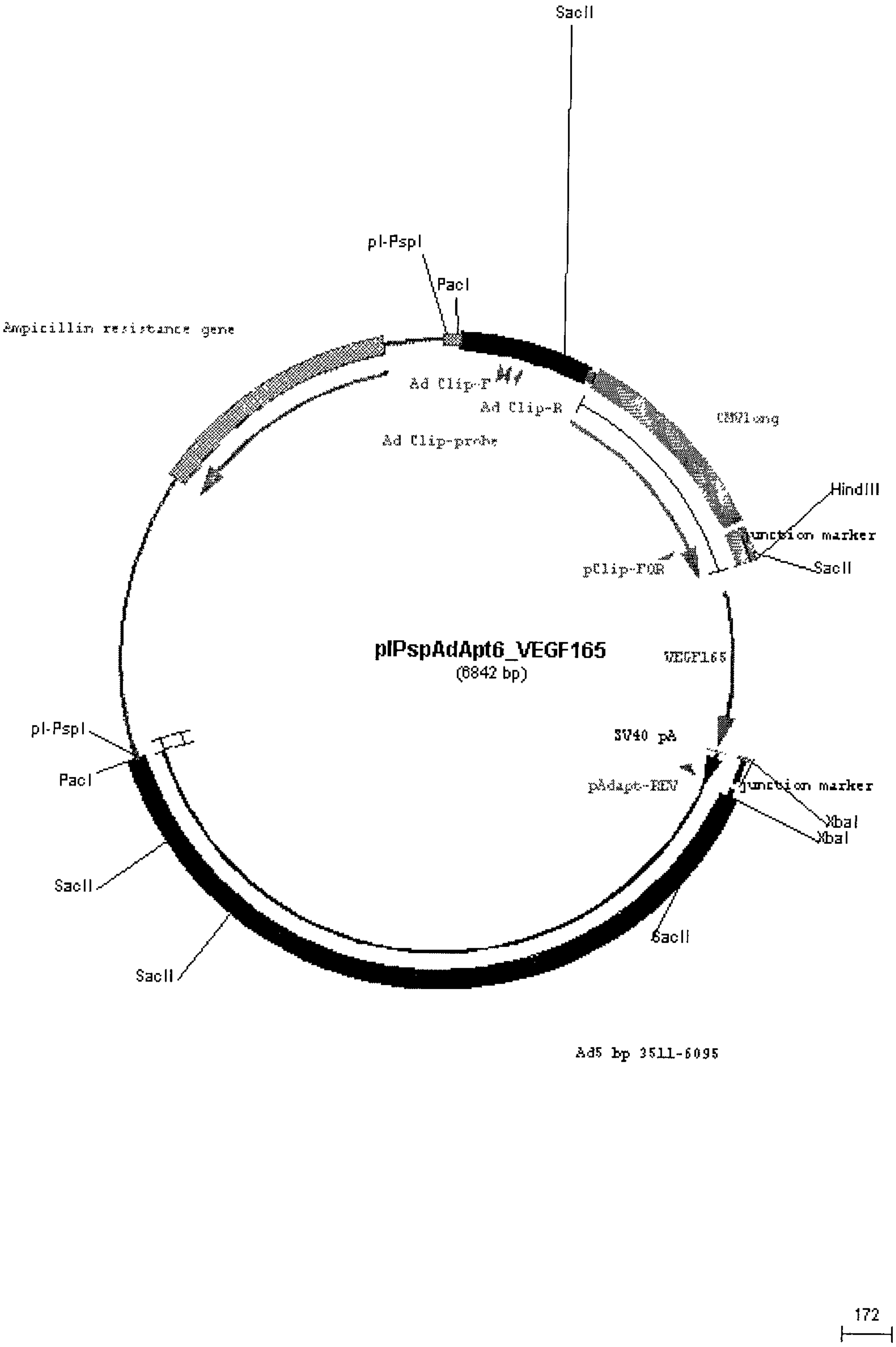

Figure 4: Schematic representation pIPspAdapt6.IL3

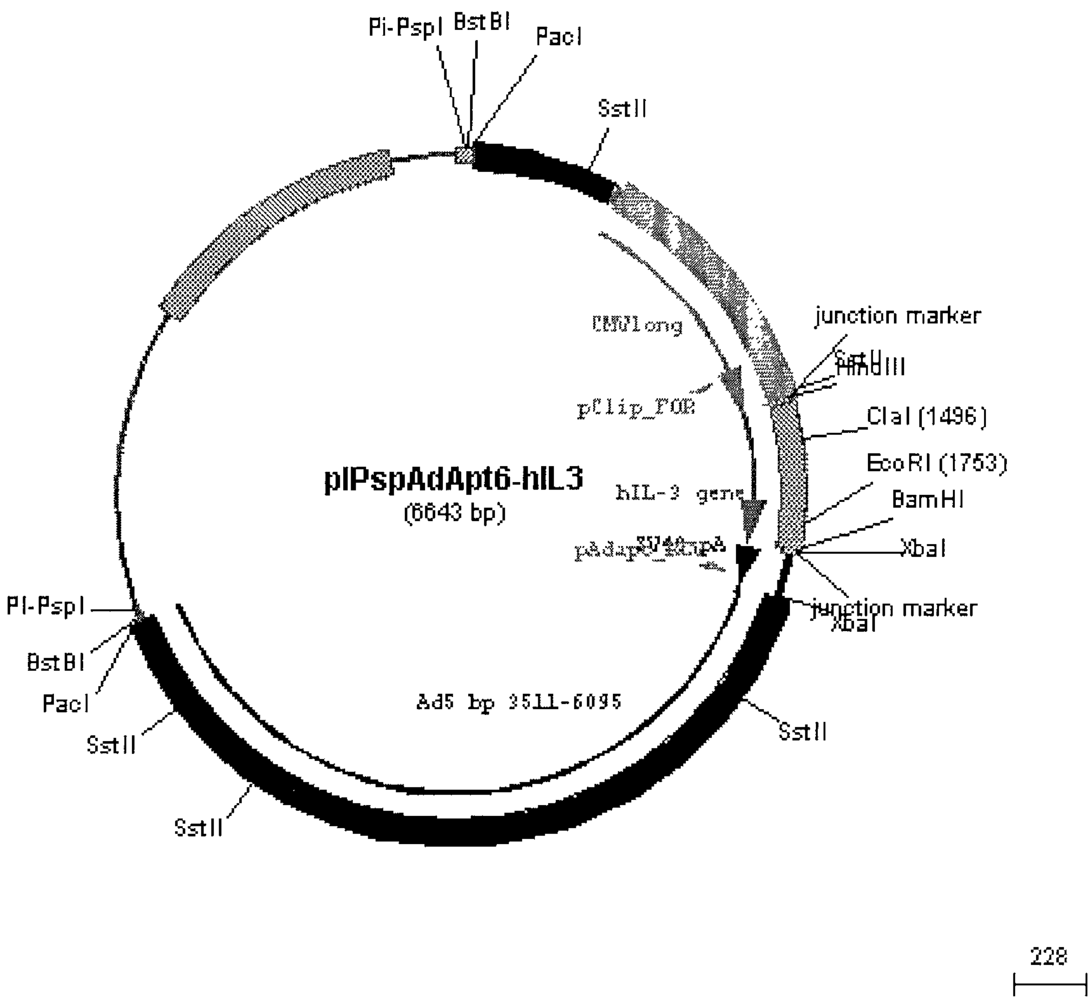

**Figure 5:** Schematic representation of the HTS in vitro capillary assay

A: indirect ivcf

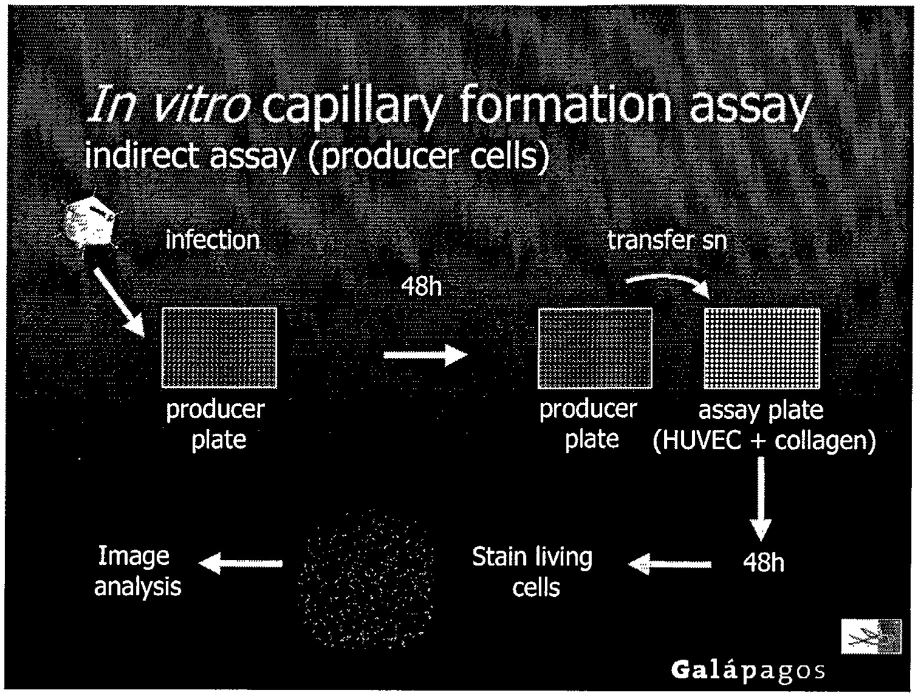


B: direct ivcf

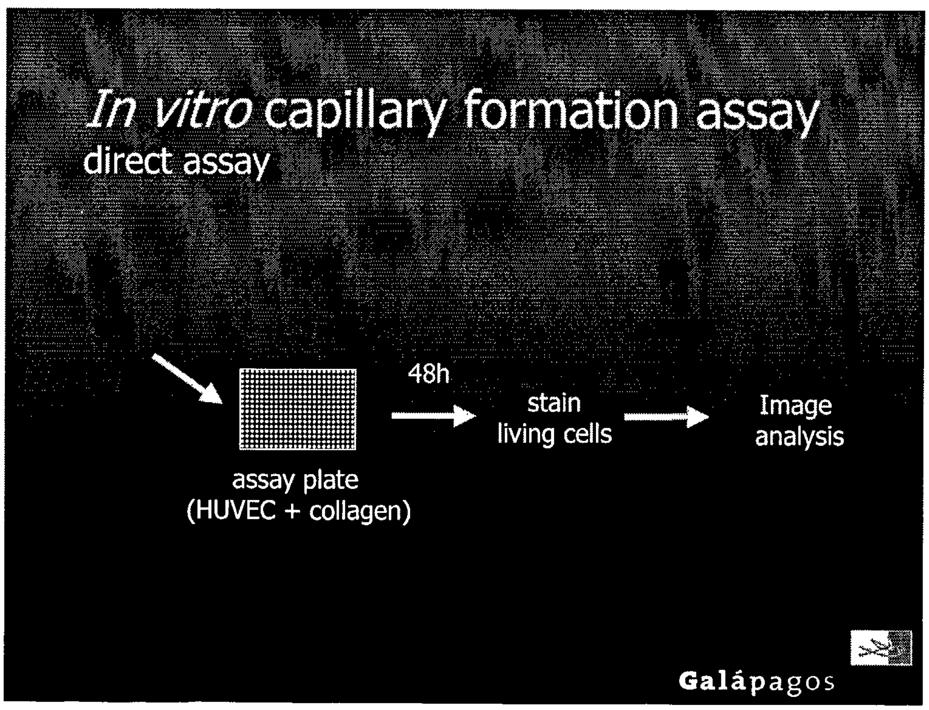

Figure 6: Different steps during image analysis of *in vitro* capillary formation assay

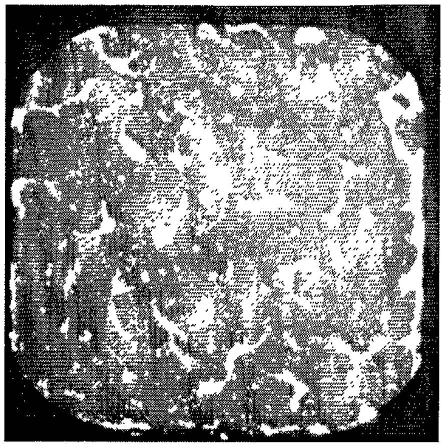

Typical Image

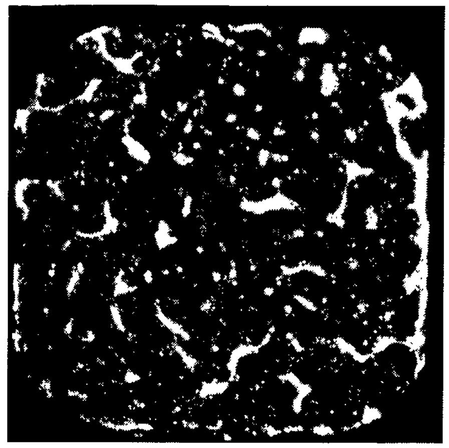

Correction in the Illumination

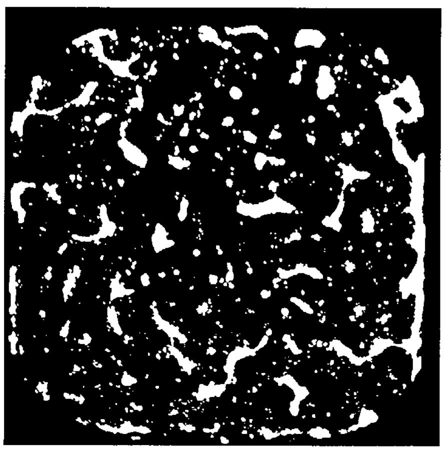

Threshold Image

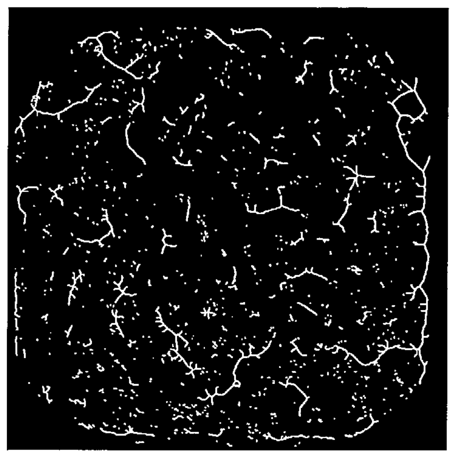

Skeletonized Image

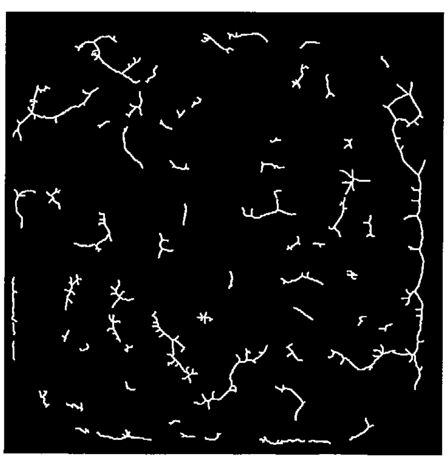

Cluster selected Image

Figure 7:

*In vitro* Capillary formation induced by recombinant human VEGF165 protein and by conditioned medium of HeLa cells infected with Ad5.dE1.dE2A.VEGF165.

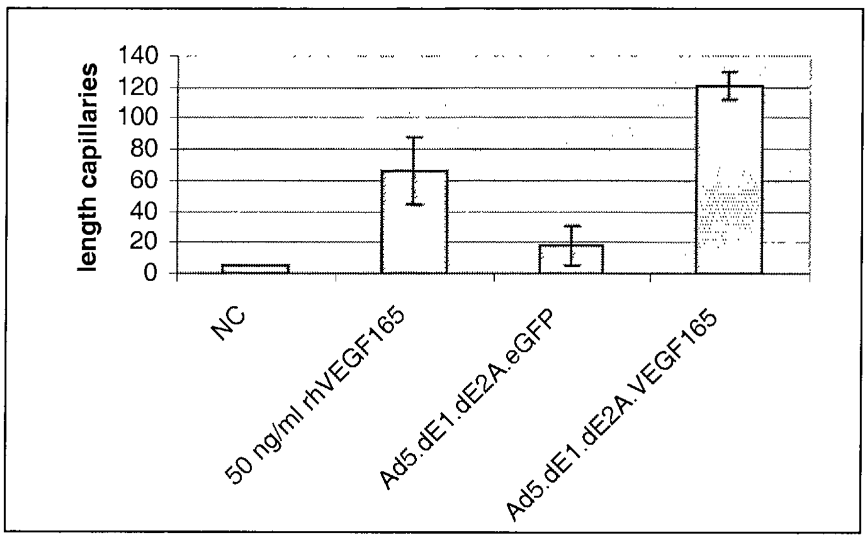

Figure 8: *In vitro* capillary formation induced by increasing amount of Ad5.dE1.dE2A.VEGF165 and Ad5.dE1.dE2A.eGFP viruses.

A: Induction of capillary formation by Ad5.dE1.dE2A.VEGF165 compared to Ad5.dE1.dE2A.eGFP.

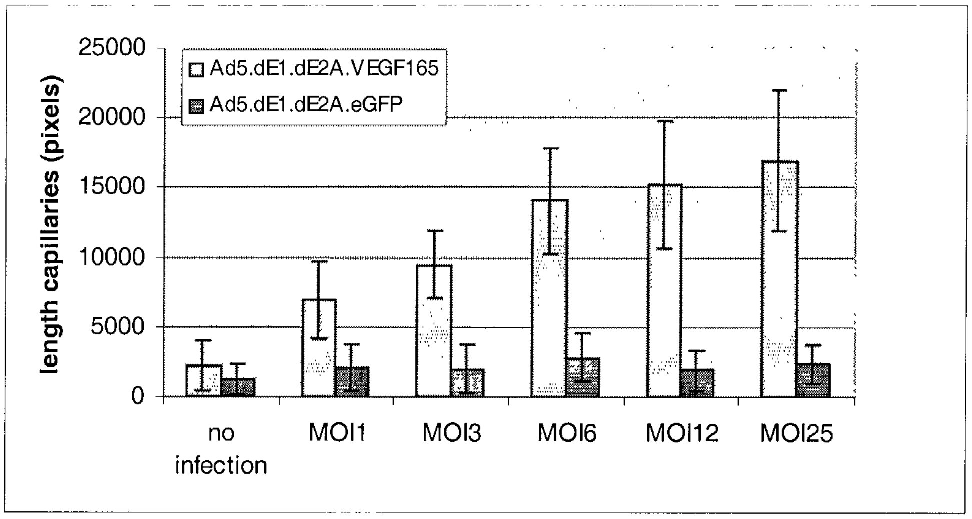


B: Corresponding VEGF165 concentration at the same MOI's. VEGF165 concentration for Ad5.dE1.dE2A.eGFP viruses corresponds to the no infection.

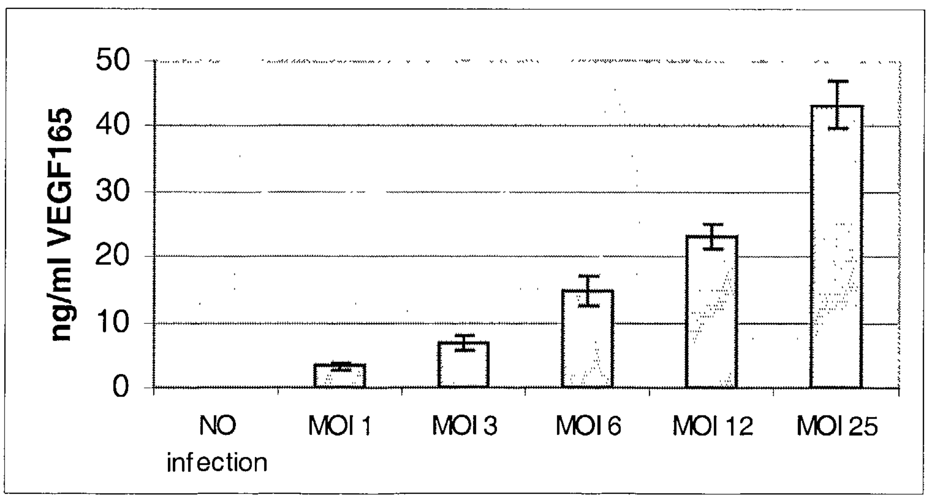

Figure 9:

Determination hit rate VEGF virus.

A: set up of VEGF library plate.

A:

| | 1 | 2 | 3 | 4 | 5 | 6 | 7 | 8 | 9 | 10 | 11 | 12 |
|---|---|---|---|---|---|---|---|---|---|---|---|---|
| A | | | | | | | | | | | | |
| B | | | | | | | | | | | | |
| C | | | | | | | | | | | | |
| D | | | | | | | | | | | | |
| E | | | | | | | | | | | | |
| F | | | | | | | | | | | | |
| G | | | | | | | | | | | | |
| H | | | | | | | | | | | | |

Ad5.dE1.dE2A.empty

Ad5.dE1.dE2A.eGFP

Ad5.dE1.dE2A.VEGF165

B: Scatterplot of results VEGF library plate in in vitro capillary formation assay.

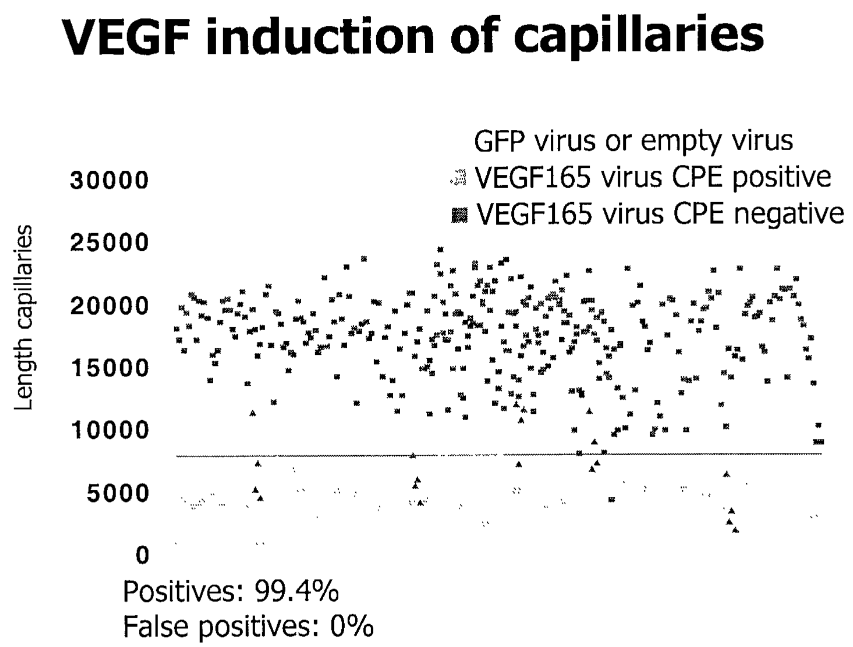

Figure 10:

Induction of *in vitro* capillary formation using Ad5.dE1.dE2A.IL3 virus.

A: Induction of capillary formation by different MOI's of Ad5.dE1.dE2A.IL3 compared to Ad5.dE1.dE2A.eGFP.

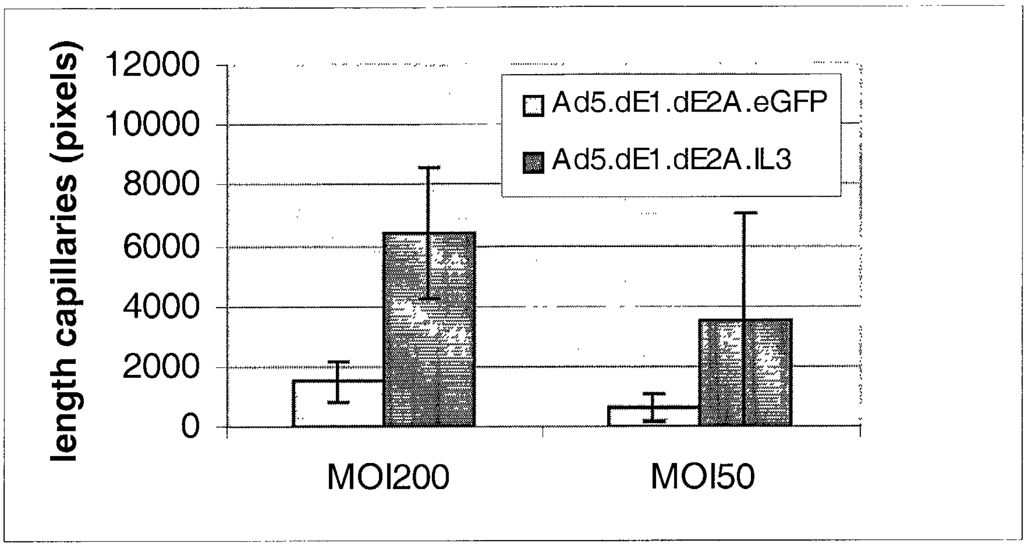

B: Scatterplot showing all the IL3 datapoints and deduction of hit rate IL3 virus.

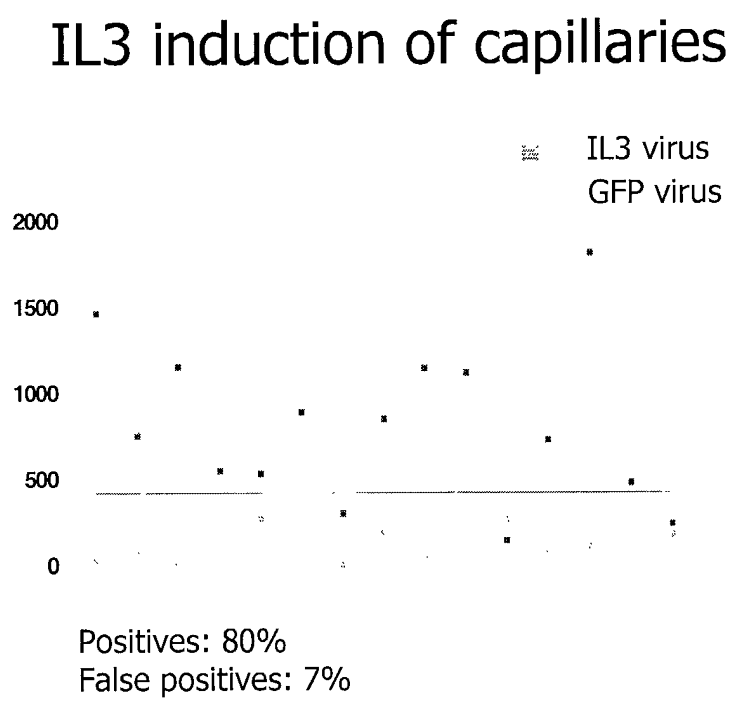

Figure 11:

Induction of capillaries using Ad5.dE1.dE2A.VEGF165 in the direct in vitro capillary formation assay.

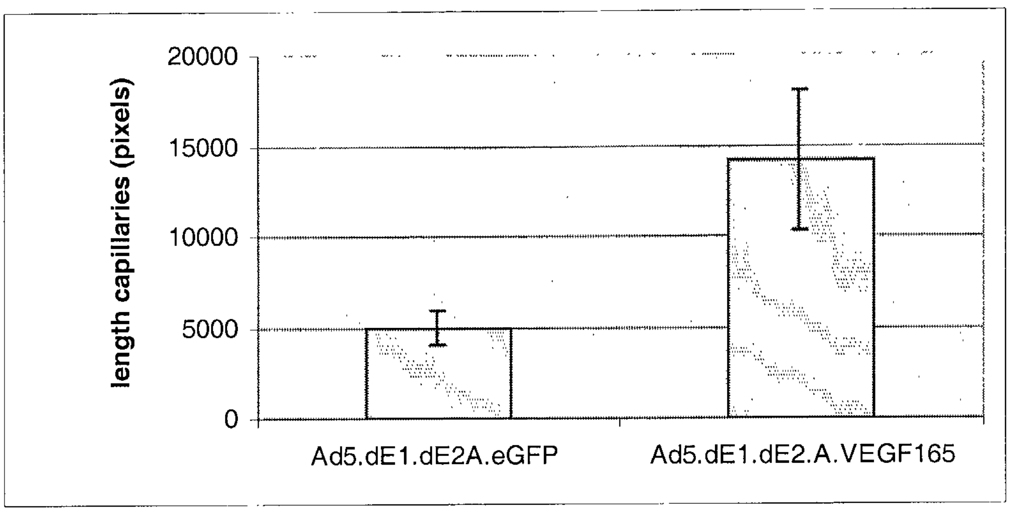

Figure 12:

Control plate. A: set up of the control plates containing 50µl of the indicated viruses and MOI's. B: Evaluation of a control plate in the *in vitro* capillary assay.

A:

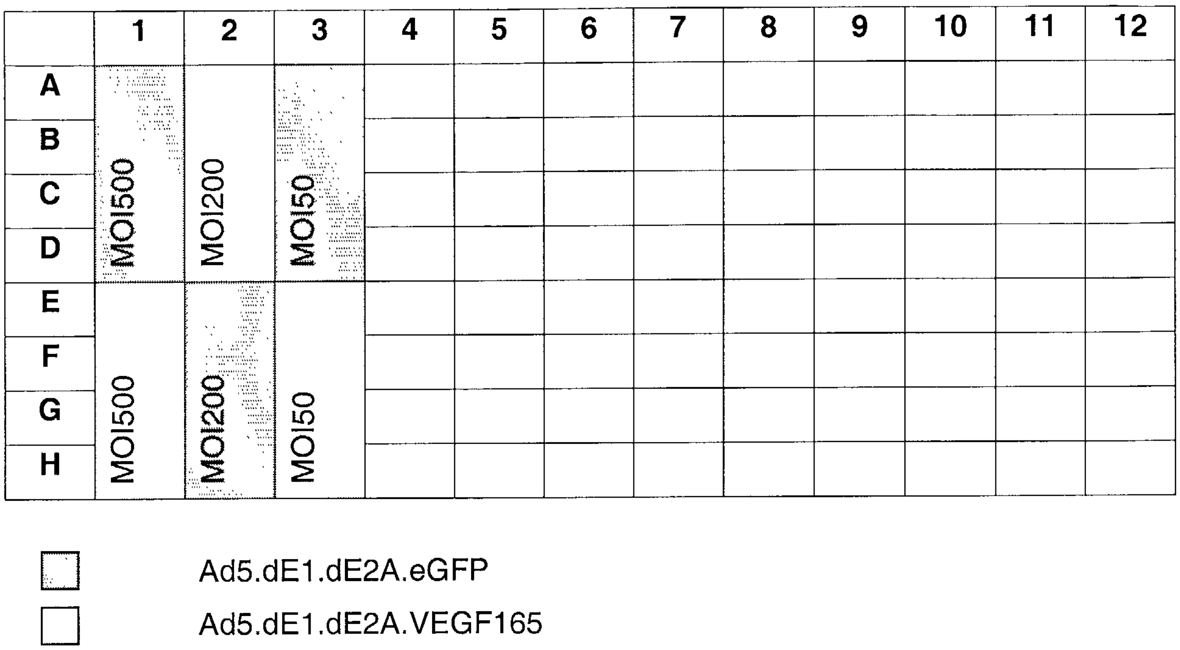


B:

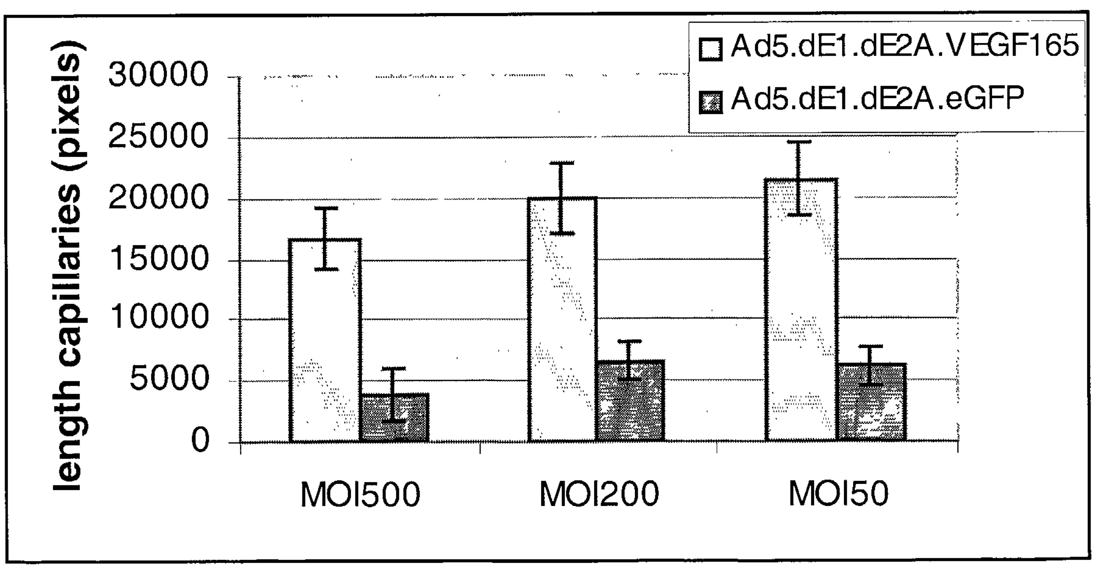

Figure 13:

Examples of results from plates obtained during the screening of 11000 viruses.

A: Scatterplots from 1 batch of library plates screened in the in vitro capillary assay.

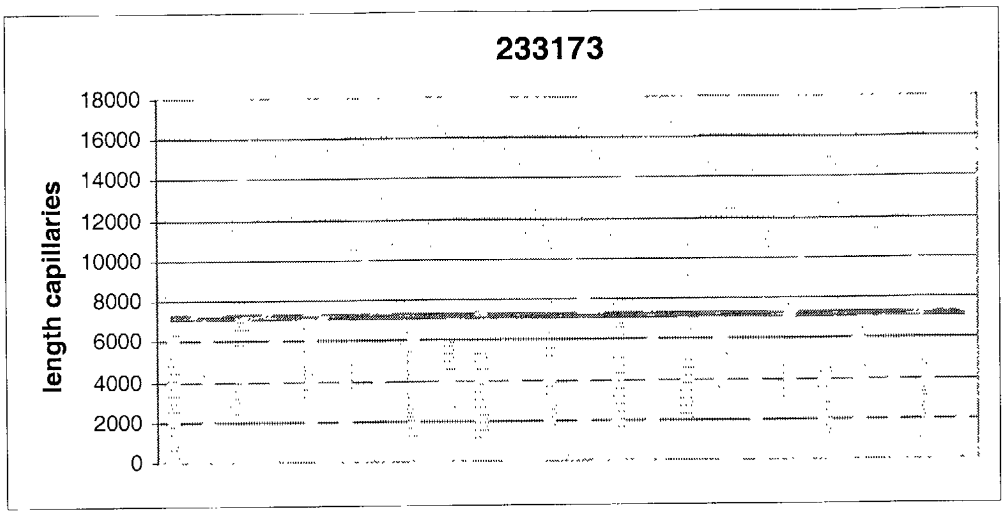


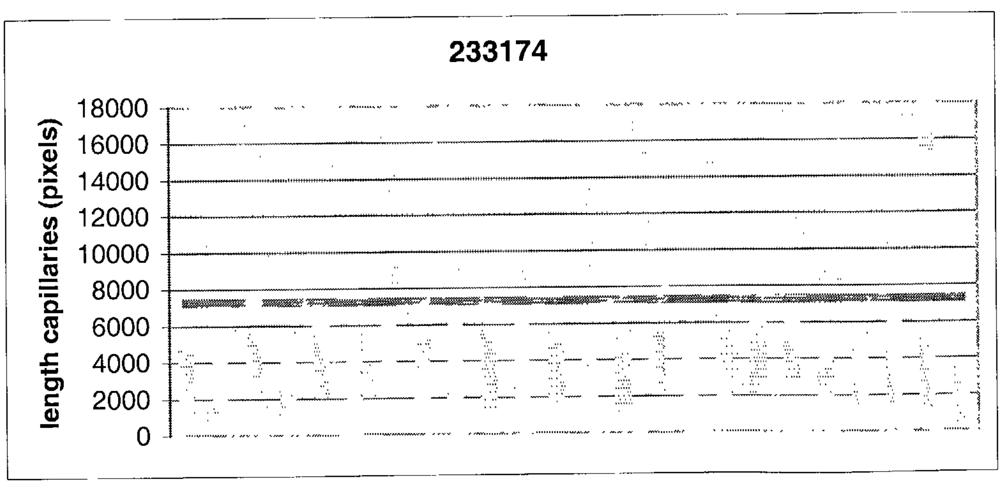

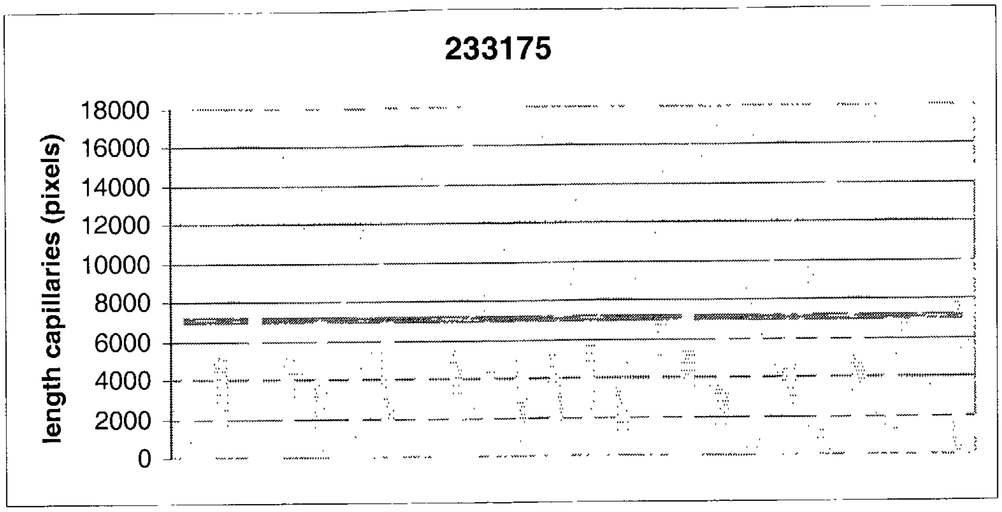
233175
18000
16000
14000
12000
10000
8000
6000
4000
2000
0
length capillaries (pixels)

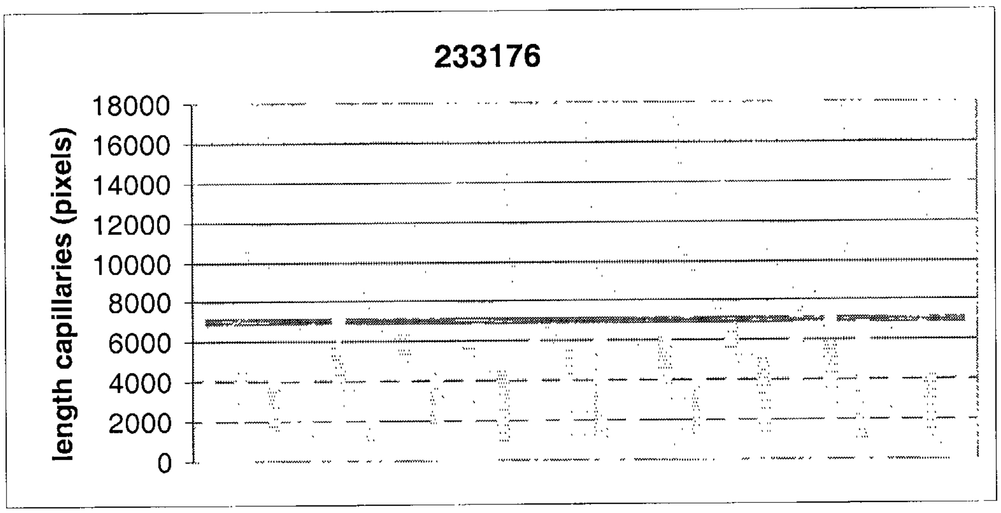
233176
18000
16000
14000
12000
10000
8000
6000
4000
2000
0
length capillaries (pixels)

B: Table showing the different thresholds calculated for the different batches of the screening.

| | Average threshold | Stdev |
|---|---|---|
| Run1 | | |
| Batch1 | 7946 | 660 |
| Batch2 | 7135 | 127 |
| Batch3 | 7498 | 410 |
| Batch4 | 10574 | 536 |
| Batch5 | 14735 | 1010 |
| Run2 | | |
| Batch1 | 8505 | 414 |
| Batch2 | 6812 | 458 |
| Batch3 | 5749 | 1012 |
| Batch4 | 7789 | 2238 |
| Batch5 | 7931 | 910 |

C: Distribution of samples of plate 233174.

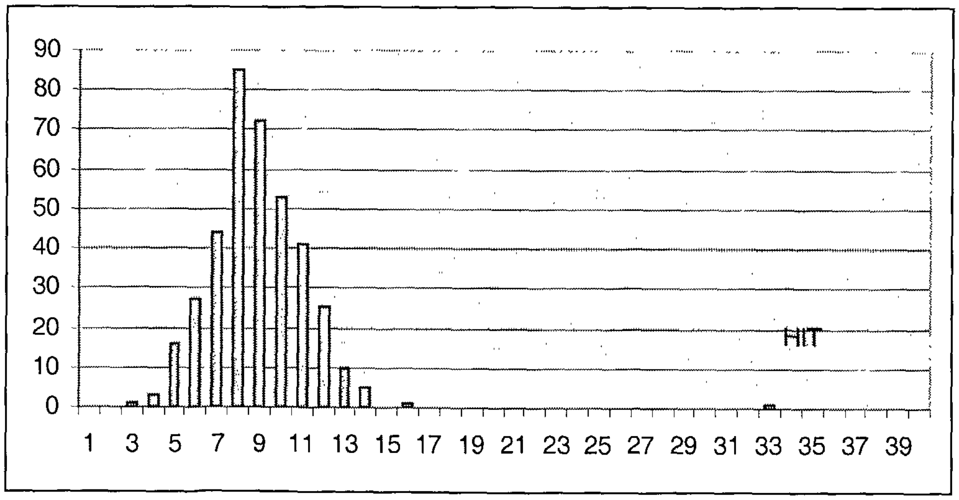

D: Results obtained for control plate during screening.

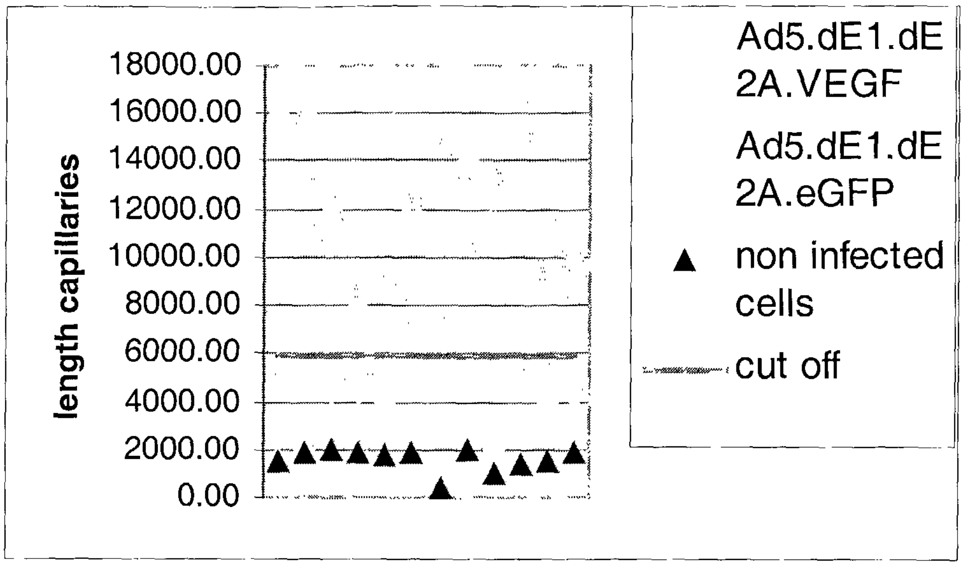


E: Overview of hits obtained during screening of 11000 viruses. Results are shown as fold induction of capillary formation.

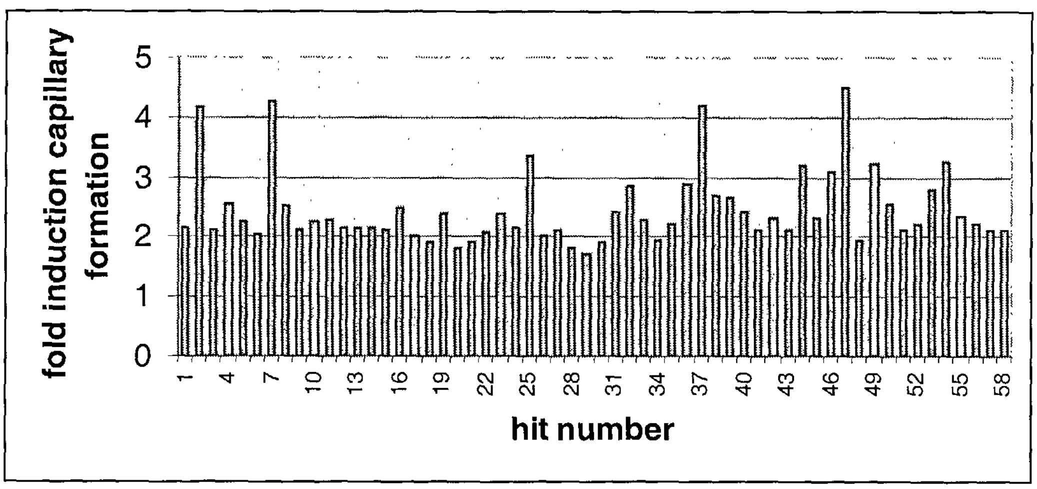

Figure 14: Rescreening hits obtained during screening 11000 viruses (n =7)

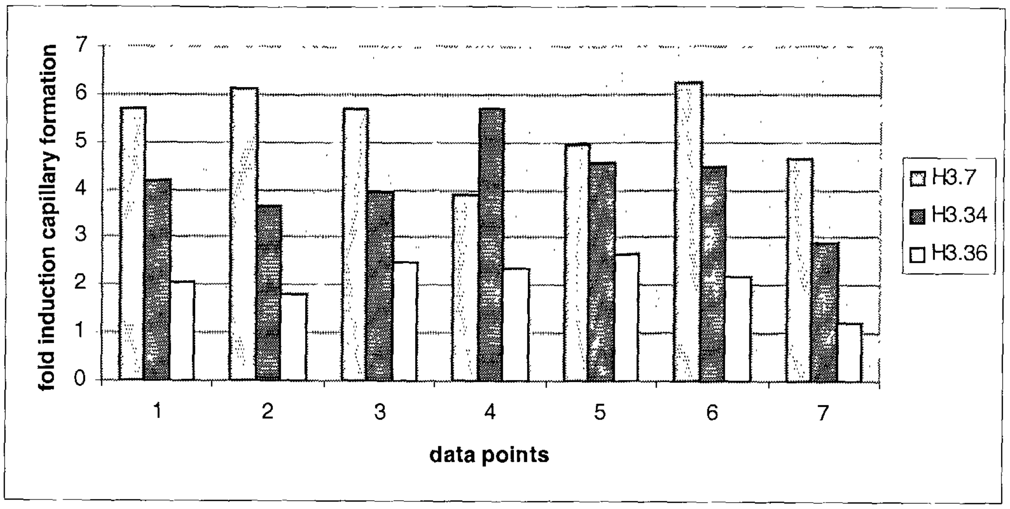

Figure 15:

Evaluation hits obtained from screening 11000 viruses in the direct *in vitro* capillary assay. (n=4)

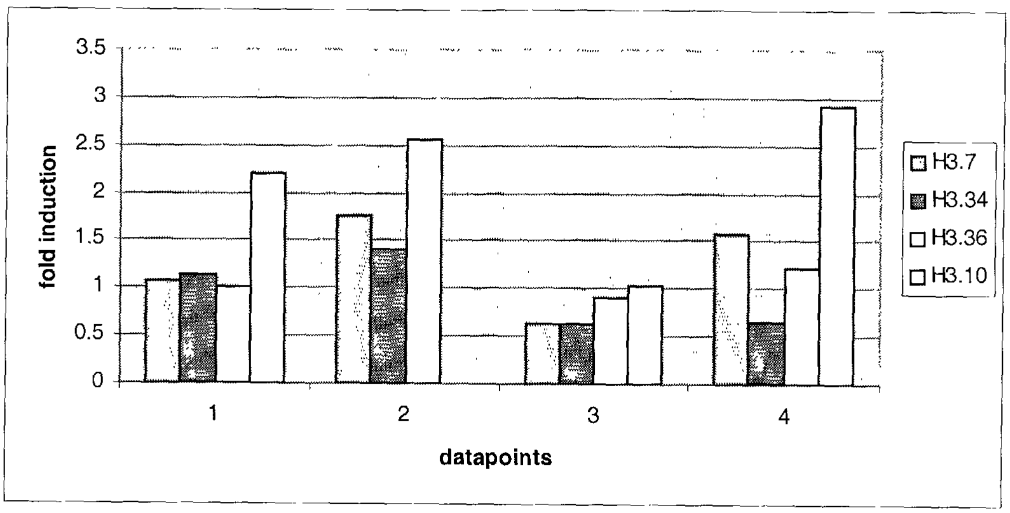

Figure 16:

Evaluation hits obtained from screening 11000 viruses in an EC proliferation assay. (n=2)

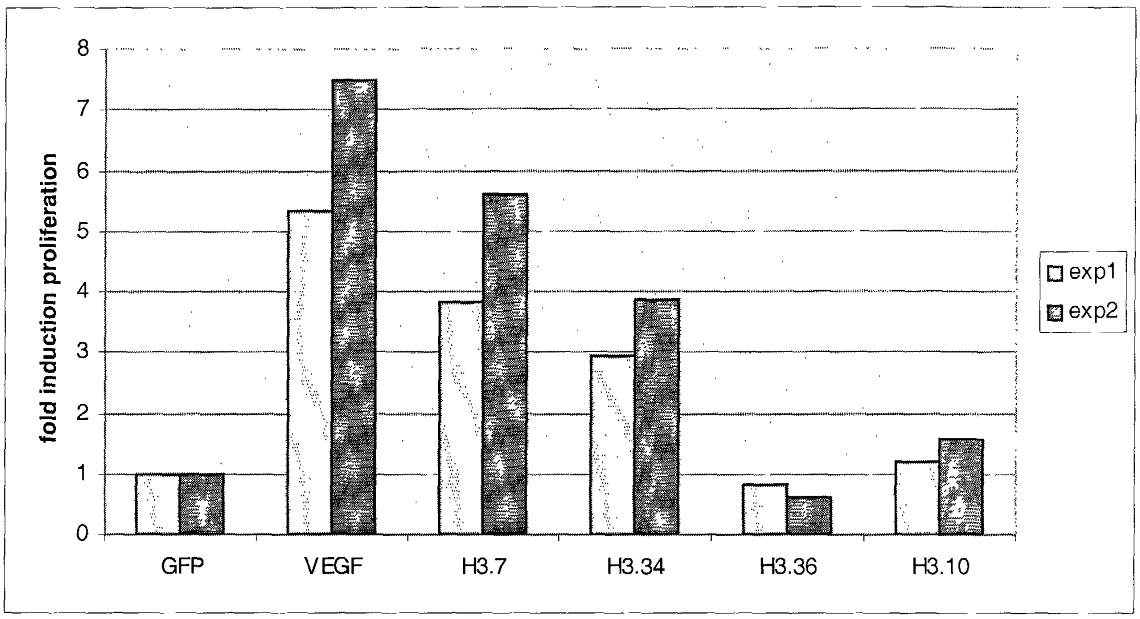

Figure 17: effect of anti-angiogenic compounds on capillary formation

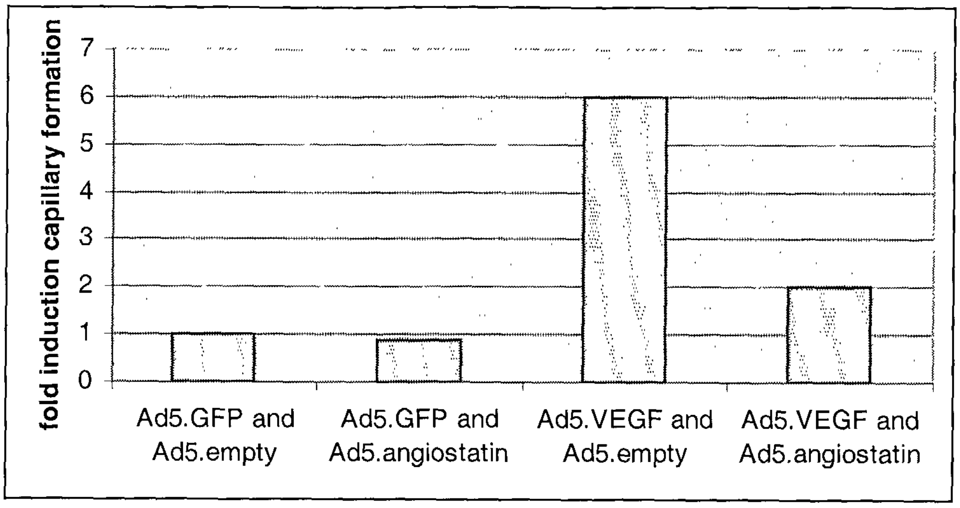

Figure 18

18.1 Hit H3-34 (SEQ ID NO 1)

```
1                    CGC TCCTGCAGCA GAGCCAACAT GCCCATCACT CGGATGCGCA
  TGAGACCCTG GCTAGAGATG CAGATTAATT CCAACCAAAT CCCGGGGCTC ATCTGGATTA
  ATAAAGAGGA GATGATCTTC CAGATCCCAT GGAAGCATGC TGCCAAGCAT GGCTGGGACA
  TCAACAAGGA TGCCTGTTTG TTCCGGAGCT GGGCCATTCA CACAGGCCGA TACAAAGCAG
  GGGAAAAGGA GCCAGATCCC AAGACGTGGA AGGCCAACTT TCGCTGTGCC ATGAACTCCC
  TGCCAGATAT CGAGGAGGTG AAAGACCAGA GCAGGAACAA GGGCAGCTCA GCTGTGCGAG
  TGTACCGGAT GCTTCCACCT CTCACCAAGA ACCAGAGAAA AGAAAGAAAG TCGAAGTCCA
  GCCGAGATGC TAAGAGCAAG GCCAAGAGGA AGTCATGTGG GGATTCCAGC CCTGATACCT
  TCTCTGATGG ACTCAGCAGC TCCACTCTGC CTGATGACCA CAGCAGCTAC ACAGTTCCAG
  GCTACATGCA GGACTTGGAG GTGGAGCAGG CCCTGACTCC AGCACTGTCG CCATGTGCTG
  TCAGCAGCAC TCTCCCCGAC TGGCACATCC CAGTGGAAGT TGTGCCGGAC AGCACCAGTG
  ATCTGTACAA CTTCCAGGTG TCACCCATGC CCTCCACCTC TGAAGCTACA ACAGATGAGG
  ATGAGGAAGG GAAATTACCT GAGGACATCA TGAAGCTCTT GGAGCAGTCG GAGTGGCAGC
  CAACAAACGT GGATGGGAAG GGGTACCTAC TCAATGAACC TGGAGTCCAG CCCACCTCTG
  TCTATGGAGA CTTTAGCTGT AAGGAGGAGC CAGAAATTGA CAGCCCAGGG GGGGATATTG
  GGCTGAGTCT ACAGCGTGTC TTCACAGATC TGAAGAACAT GGATGCCACC TGGCTGGACA
  GCCTGCTGAC CCCAGTCCGG TTGCCCTCCA TCCAGGCCAT TCCCTGTGCA CCGTAGCAGG
  GCCCCTGGGC CCCTCTTATT CCTCTAGGCA AGCAGGACCT GGCATCATGG TGGATATGGT
  GCAGAGAAGC TGGACTTCTG TGGGCCCCTC AACAGCCAAG TGTGACCCCA CTGCCAAGTG
  GGGATGGGGC CTCCCTCCTT GGGTCATTGA CCTCTCAGGG CCTGGCAGGC CAGTGTCTGG
  GTTTTTCTTG TGGTGTAAAG CTGGCCCTGC CTCCTGGGAA GATGAGGTTC TGAGACCAGT
  GTATCAGGTC AGGGACTTGG ACAGGAGTCA GTGTCTGGCT TTTTCCTCTG AGCCCAGCTG
  CCTGGAGAGG GTCTCGCTGT CACTGGCTGG CTCCTAGGGG AACAGACCAG TGACCCCAGA
  AAAGCATAAC ACCAATCCCA GGGCTGGCTC TGCACTAAGA GAAAATTGCA CTAAATGAAT
  CTCGTTCCCA AAGAACTACC CCCTTTTCAG CTGAGCCCTG GGGACTGTTC CAAAGCCAGT
  GAAATGTGAA GGAAAGTGGG GTCCTTCGGG GCGATGCTCC CTCAGCCTCA GAGGAGCTCT
  ACCCTGCTCC CTGCTTTGGC TGAGGGGCTT GGGAAAAAAA CTTGGCACTT TTTCGTGTGG
  ATCTTGCCAC ATTTCTGATC AGAGGTGTAC ACTAACATTT CCCCCGAGCT CTTGGCCTTT
  GCATTTATTT ATACAGTGCC TTGCTCGGCG CCCACCACCC CCTCAAGCCC CAGCAGCCCT
  CAACAGGCCC AGGGAGGGAA GTGTGAGCGC CTTGGTATGA CTTAAAATTG GAAATGTCAT
  CTAACCATTA AGTCATGTGT GAACACATAG GACGTGTGTA AATATGTACA TTTGTCTTTT
  TATAAAAAGT AAATTGTTTA TAAGGGGTGT GGCCTTTTTA GAGAGAAATT TAACTTGTAG
  ATGATTTTAC TTTTTATGGA AACACTGATG GACTTATTAT TGGCATCCCG CCTGAACTTG
  ACTTTGGGGT GAACAGGGAC ATGCATCTAT TATAAAATCC TTTCGGCCAG GCGCGGTGGC
  TCACACCTGT AATCCCAGCA CTTTGGGAGG CCGAGATGGG TGGATCACCT GAGGTCAGGA
  GTTCGAGACC AGCCTGGTGA AACTCCATTT CTACTAAAAA TGCAAAAATT AGCTGGGCGT
  GGTTGCGGGT GCTTGTAATC CCAGCTACTC AGGAGGCTGA GGCAAGAGAA TCGCTTGAAC
  CTGGGAGGTG GAGGTTGCAG TGAGCCGAGA ACATGCCATT GCACTCCAGC CCGGGCACCA
  AAAAAAAAAA AAAAAAA
```

18.1Hit H3-34 translational product (SEQ ID NO 2)

```
MPITRMRMRP WLEMQINSNQ IPGLIWINKE EMIFQIPWKH AAKHGWDINK DACLFRSWAI
HTGRYKAGEK EPDPKTWKAN FRCAMNSLPD IEEVKDQSRN KGSSAVRVYR MLPPLTKNQR
KERKSKSSRD AKSKAKRKSC GDSSPDTFSD GLSSSTLPDD HSSYTVPGYM QDLEVEQALT
PALSPCAVSS TLPDWHIPVE VVPDSTSDLY NFQVSPMPST SEATTDEDEE GKLPEDIMKL
LEQSEWQPTN VDGKGYLLNE PGVQPTSVYG DFSCKEEPEI DSPGGDIGLS LQRVFTDLKN
MDATWLDSLL TPVRLPSIQA IPCAP*
```

18.2 Hit H3-36 (SEQ ID NO 3)

```
1                    AGG AAACCACCCT ACCGGGGTGG AAGGGAGGGT CAGGGAAGAA
  ACCCACTCTT GCTCTACGAG GAGCAAGTGC CTGCCCCCTC CCAGCAGCCA GCCCTGCCAA
  AGTTGCATTA TCTTTGGCCA AGGCTGGGCC TGACGGTTAT GATTTCAGCC CTGGGCCTGC
  AGGAGAGGCT GAGACCAGCC CACCCAGCCA GTGGTCGAGC ACTGCCCCGC CGCCAAAGTC
  TGCAGAATGT GAGATGAGGT TCTCAAGGTC ACAGGCCCCA GTCCCAGCCT GGGGGCTGGC
  AGAGGCCCCC ATATACTCTG CTACAGCTCC TATCATGAAA AATAAAATGT TTGTCTTTGC
  AAAAAAAAAA AAAAAAAAAA AAAAA
```

18.2 Hit H3-36 translational product (SEQ ID NO 4)

```
MISALGLQER LRPAHPASGR ALPRRQSLQN VR*
```

European Patent Office

# EUROPEAN SEARCH REPORT

Application Number
EP 01 87 0154

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | MARUYAMA ET AL.: "Sequence of a cDNA coding for human IRF-1"<br>NUCLEIC ACIDS RESEARCH,<br>vol. 17, no. 8, 1989, page 3292<br>XP002196380<br>* the whole document *<br>& DATABASE GENBANK 'Online!<br>NCBI; 31 October 2000 (2000-10-31)<br>"Homo sapiens interferon regulatory factor 1 (IRF1), mRNA"<br>Database accession no. NM002198<br>* the whole document *<br>--- | 8 | C12Q1/68<br>C12N15/12<br>C07K14/47<br>C07K16/18<br>A61K38/17<br>A61P35/00<br>A61K39/395<br>C07K16/00 |
| X | CHA ET AL.: "Human interferon regulatory factor 1: intron-exon organization"<br>DNA AND CELL BIOLOGY,<br>vol. 11, no. 8, 1992, page 605-611<br>XP008002916<br>* the whole document *<br>--- | 8 | |
| X | DATABASE GENBANK 'Online!<br>NCBI; 15 May 2001 (2001-05-15)<br>ISOGAI T ET AL.: "NEDO human cDNA sequencing project"<br>Database accession no. AK027856<br>XP002196382<br>* the whole document *<br>& YUDATE ET AL.: "HUNT: launch of a full-length cDNA database from the Helix Research Institute""<br>NUCLEIC ACIDS RESEARCH,<br>vol. 29, no. 1, January 2001 (2001-01), pages 185-188,<br>* the whole document *<br>---<br>-/-- | 8 | TECHNICAL FIELDS SEARCHED (Int.Cl.7)<br>C12Q<br>C12N |

The present search report has been drawn up for all claims

| Place of search | Date of completion of the search | Examiner |
|---|---|---|
| THE HAGUE | 3 May 2002 | Pinta, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 01 87 0154

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | SCHICHMAN ET AL.: "ALL-1 tandem duplication in acute myeloid leukemia with a normal karyotype involves homologous recombination between Alu elements" CANCER RESEARCH, vol. 54, no. 16, 1994, pages 4277-4280, XP001073460 * see Fig. 3a * | 8 | |
| X | WO 95 14772 A (MATSUBARA KENICHI ;OKUBO KOUSAKU (JP)) 1 June 1995 (1995-06-01) * see sequence HUMGS05730, p. 1437 * | 8 | |
| X | DATABASE GENBANK 'Online! NCBI; CHOI ET AL.: "Cloning of ovine IRF-1" Database accession no. AF331970 XP002196383 * abstract * | 8 | |
| Y | YONEKURA ET AL.: "Antisense display - a method for functional gene screening: evaluation in a cell-free system and isolation of angiogenesis-related genes" NUCLEIC ACIDS RESEARCH, vol. 27, no. 13, July 1999 (1999-07), pages 2591-2600, XP002196381 * the whole document * | 1,2,4,7 | TECHNICAL FIELDS SEARCHED (Int.Cl.7) |
| Y | EP 1 022 335 A (INTROGENE BV) 26 July 2000 (2000-07-26) * the whole document * | 1,2,4,7 | |
| Y | WO 00 14103 A (BETH ISRAEL HOSPITAL) 16 March 2000 (2000-03-16) * the whole document * | 1,4,7 | |

-/--

The present search report has been drawn up for all claims

| Place of search | Date of completion of the search | Examiner |
|---|---|---|
| THE HAGUE | 3 May 2002 | Pinta, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 01 87 0154

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 00 62833 A (UNIV NEW YORK) 26 October 2000 (2000-10-26) * the whole document * --- | 1,2 | |
| Y | WO 00 52158 A (MITOTIX INC ;CELL GENESYS INC (US)) 8 September 2000 (2000-09-08) * the whole document * --- | 1,2 | |
| Y | VERNON AND SAGE: "A novel, quantitative model for study of endothelial cell migration and sprout formation within three-dimensional collagen matrices" MICROVASCULAR RESEARCH, vol. 57, pages 118-133, XP002196817 * the whole document * ----- | 1,2 | |

TECHNICAL FIELDS SEARCHED (Int.Cl.7)

The present search report has been drawn up for all claims

| Place of search | Date of completion of the search | Examiner |
|---|---|---|
| THE HAGUE | 3 May 2002 | Pinta, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 87 0154

This annex lists the patent family members relating to the patent documents cited in the above-mentioned European search report.
The members are as contained in the European Patent Office EDP file on
The European Patent Office is in no way liable for these particulars which are merely given for the purpose of information.

03-05-2002

| Patent document cited in search report | | Publication date | Patent family member(s) | | Publication date |
|---|---|---|---|---|---|
| WO 9514772 | A | 01-06-1995 | AU | 8116494 A | 13-06-1995 |
| | | | CA | 2153480 A1 | 01-06-1995 |
| | | | EP | 0679716 A1 | 02-11-1995 |
| | | | WO | 9514772 A1 | 01-06-1995 |
| EP 1022335 | A | 26-07-2000 | AU | 4294799 A | 30-12-1999 |
| | | | CA | 2301403 A1 | 16-12-1999 |
| | | | EP | 1022335 A1 | 26-07-2000 |
| | | | WO | 9964582 A2 | 16-12-1999 |
| | | | US | 6340595 B1 | 22-01-2002 |
| WO 0014103 | A | 16-03-2000 | AU | 5679499 A | 27-03-2000 |
| | | | EP | 1112283 A1 | 04-07-2001 |
| | | | WO | 0014103 A1 | 16-03-2000 |
| WO 0062833 | A | 26-10-2000 | AU | 4351000 A | 02-11-2000 |
| | | | WO | 0062833 A1 | 26-10-2000 |
| WO 0052158 | A | 08-09-2000 | AU | 3389700 A | 21-09-2000 |
| | | | AU | 3607500 A | 21-09-2000 |
| | | | AU | 3607600 A | 21-09-2000 |
| | | | EP | 1157122 A1 | 28-11-2001 |
| | | | EP | 1181362 A1 | 27-02-2002 |
| | | | EP | 1157108 A1 | 28-11-2001 |
| | | | WO | 0052158 A1 | 08-09-2000 |
| | | | WO | 0052159 A1 | 08-09-2000 |
| | | | WO | 0052184 A1 | 08-09-2000 |

EPO FORM P0459

For more details about this annex : see Official Journal of the European Patent Office, No. 12/82

SEQUENCE LISTING

<110> Galapagos Genomics N.V.

<120> High throughput identification of modulators of angiogenesis

<130> GAL-008-EP

<160> 4

<170> PatentIn version 3.1

<210> 1
<211> 2280
<212> DNA
<213> Homo sapiens

<400> 1
cgctcctgca gcagagccaa catgcccatc actcggatgc gcatgagacc ctggctagag 60

atgcagatta attccaacca aatcccgggg ctcatctgga ttaataaaga ggagatgatc 120

ttccagatcc catggaagca tgctgccaag catggctggg acatcaacaa ggatgcctgt 180

ttgttccgga gctgggccat tcacacaggc cgatacaaag caggggaaaa ggagccagat 240

cccaagacgt ggaaggccaa ctttcgctgt gccatgaact ccctgccaga tatcgaggag 300

gtgaaagacc agagcaggaa caagggcagc tcagctgtgc gagtgtaccg gatgcttcca 360

cctctcacca agaaccagag aaaagaaaga aagtcgaagt ccagccgaga tgctaagagc 420

aaggccaaga ggaagtcatg tggggattcc agccctgata ccttctctga tggactcagc 480

agctccactc tgcctgatga ccacagcagc tacacagttc caggctacat gcaggacttg 540

gaggtggagc aggccctgac tccagcactg tcgccatgtg ctgtcagcag cactctcccc 600

gactggcaca tcccagtgga agttgtgccg gacagcacca gtgatctgta caacttccag 660

gtgtcaccca tgccctccac ctctgaagct acaacagatg aggatgagga agggaaatta 720

cctgaggaca tcatgaagct cttggagcag tcggagtggc agccaacaaa cgtggatggg 780

aaggggtacc tactcaatga acctggagtc cagcccacct ctgtctatgg agactttagc 840

tgtaaggagg agccagaaat tgacagccca gggggggata ttgggctgag tctacagcgt 900

gtcttcacag atctgaagaa catggatgcc acctggctgg acagcctgct gaccccagtc 960

cggttgccct ccatccaggc cattccctgt gcaccgtagc agggcccctg ggcccctctt 1020

attcctctag gcaagcagga cctggcatca tggtggatat ggtgcagaga agctggactt 1080

ctgtgggccc ctcaacagcc aagtgtgacc ccactgccaa gtggggatgg ggcctccctc 1140

cttgggtcat tgacctctca gggcctggca ggccagtgtc tgggtttttc ttgtggtgta 1200

aagctggccc tgcctcctgg gaagatgagg ttctgagacc agtgtatcag gtcagggact 1260

tggacaggag tcagtgtctg gctttttcct ctgagcccag ctgcctggag agggtctcgc 1320

tgtcactggc tggctcctag gggaacagac cagtgacccc agaaaagcat aacaccaatc 1380

```
ccagggctgg ctctgcacta agagaaaatt gcactaaatg aatctcgttc ccaaagaact    1440
accccctttt cagctgagcc ctggggactg ttccaaagcc agtgaaatgt gaaggaaagt    1500
ggggtccttc ggggcgatgc tccctcagcc tcagaggagc tctaccctgc tccctgcttt    1560
ggctgagggg cttgggaaaa aaacttggca ctttttcgtg tggatcttgc cacatttctg    1620
atcagaggtg tacactaaca tttcccccga gctcttggcc tttgcattta tttatacagt    1680
gccttgctcg gcgcccacca ccccctcaag ccccagcagc cctcaacagg cccagggagg    1740
gaagtgtgag cgccttggta tgacttaaaa ttggaaatgt catctaacca ttaagtcatg    1800
tgtgaacaca taggacgtgt gtaaatatgt acatttgtct ttttataaaa agtaaattgt    1860
ttataagggg tgtggccttt ttagagagaa atttaacttg tagatgattt tactttttat    1920
ggaaacactg atggacttat tattggcatc ccgcctgaac ttgactttgg ggtgaacagg    1980
gacatgcatc tattataaaa tcctttcggc caggcgcggt ggctcacacc tgtaatccca    2040
gcactttggg aggccgagat gggtggatca cctgaggtca ggagttcgag accagcctgg    2100
tgaaactcca tttctactaa aaatgcaaaa attagctggg cgtggttgcg ggtgcttgta    2160
atcccagcta ctcaggaggc tgaggcaaga gaatcgcttg aacctgggag gtggaggttg    2220
cagtgagccg agaacatgcc attgcactcc agcccgggca ccaaaaaaaa aaaaaaaaaa    2280

<210>  2
<211>  325
<212>  PRT
<213>  Homo sapiens

<400>  2

Met Pro Ile Thr Arg Met Arg Met Arg Pro Trp Leu Glu Met Gln Ile
1               5                   10                  15

Asn Ser Asn Gln Ile Pro Gly Leu Ile Trp Ile Asn Lys Glu Glu Met
            20                  25                  30

Ile Phe Gln Ile Pro Trp Lys His Ala Ala Lys His Gly Trp Asp Ile
        35                  40                  45

Asn Lys Asp Ala Cys Leu Phe Arg Ser Trp Ala Ile His Thr Gly Arg
    50                  55                  60

Tyr Lys Ala Gly Glu Lys Glu Pro Asp Pro Lys Thr Trp Lys Ala Asn
65                  70                  75                  80

Phe Arg Cys Ala Met Asn Ser Leu Pro Asp Ile Glu Glu Val Lys Asp
                85                  90                  95
```

```
Gln Ser Arg Asn Lys Gly Ser Ser Ala Val Arg Val Tyr Arg Met Leu
            100                 105                 110

Pro Pro Leu Thr Lys Asn Gln Arg Lys Glu Arg Lys Ser Lys Ser Ser
        115                 120                 125

Arg Asp Ala Lys Ser Lys Ala Lys Arg Lys Ser Cys Gly Asp Ser Ser
    130                 135                 140

Pro Asp Thr Phe Ser Asp Gly Leu Ser Ser Ser Thr Leu Pro Asp Asp
145                 150                 155                 160

His Ser Ser Tyr Thr Val Pro Gly Tyr Met Gln Asp Leu Glu Val Glu
                165                 170                 175

Gln Ala Leu Thr Pro Ala Leu Ser Pro Cys Ala Val Ser Ser Thr Leu
            180                 185                 190

Pro Asp Trp His Ile Pro Val Glu Val Val Pro Asp Ser Thr Ser Asp
        195                 200                 205

Leu Tyr Asn Phe Gln Val Ser Pro Met Pro Ser Thr Ser Glu Ala Thr
    210                 215                 220

Thr Asp Glu Asp Glu Glu Gly Lys Leu Pro Glu Asp Ile Met Lys Leu
225                 230                 235                 240

Leu Glu Gln Ser Glu Trp Gln Pro Thr Asn Val Asp Gly Lys Gly Tyr
                245                 250                 255

Leu Leu Asn Glu Pro Gly Val Gln Pro Thr Ser Val Tyr Gly Asp Phe
            260                 265                 270

Ser Cys Lys Glu Glu Pro Glu Ile Asp Ser Pro Gly Gly Asp Ile Gly
        275                 280                 285

Leu Ser Leu Gln Arg Val Phe Thr Asp Leu Lys Asn Met Asp Ala Thr
    290                 295                 300

Trp Leu Asp Ser Leu Leu Thr Pro Val Arg Leu Pro Ser Ile Gln Ala
305                 310                 315                 320

Ile Pro Cys Ala Pro
                325

<210>  3
<211>  368
<212>  DNA
```

```
<213>  Homo sapiens

<400>  3
aggaaaccac cctaccgggg tggaagggag ggtcagggaa gaaacccact cttgctctac     60

gaggagcaag tgcctgcccc ctcccagcag ccagccctgc caaagttgca ttatctttgg    120

ccaaggctgg gcctgacggt tatgatttca gccctgggcc tgcaggagag gctgagacca    180

gcccacccag ccagtggtcg agcactgccc cgccgccaaa gtctgcagaa tgtgagatga    240

ggttctcaag gtcacaggcc ccagtcccag cctgggggct ggcagaggcc cccatatact    300

ctgctacagc tcctatcatg aaaaataaaa tgtttgtctt tgcaaaaaaa aaaaaaaaaa    360

aaaaaaaa                                                             368


<210>  4
<211>  32
<212>  PRT
<213>  Homo sapiens

<400>  4

Met Ile Ser Ala Leu Gly Leu Gln Glu Arg Leu Arg Pro Ala His Pro
1               5                   10                  15


Ala Ser Gly Arg Ala Leu Pro Arg Arg Gln Ser Leu Gln Asn Val Arg
            20                  25                  30
```